# NEW EUROPEAN PATENT SPECIFICATION

(11) **EP 1 490 397 B2**
(45) Date of publication and mention of the opposition decision: **09.04.2014**
(45) Mention of the grant of the patent: 26.05.2010
(21) Application number: 03744850.3
(22) Date of filing: 20.03.2003
(51) Int. Cl.: C07K 14/325

(54) **NOVEL BACILLUS THURINGIENSIS INSECTICIDAL PROTEINS**
NEUE INSEKTIZIDE PROTEINE VON BAZILLUS THURINGIENSIS
NOUVELLES PROTEINES INSECTICIDES ISSUES DU BACILLUS THURINGIENSIS

(30) Priority: 22.03.2002 US 366276 P; 06.11.2002 US 423999 P
(43) Date of publication of application: 29.12.2004
(60) Divisional application: 10075098.3
(73) Proprietor: Bayer CropScience NV, 1831 Diegem (BE)
(72) Inventor: ARNAUT, Greta, B-9910 Knesselare (BE); BOETS, Annemie, B-9620 Velzeke (BE); DE RUDDER, Karel, NL-4564 CM Sint Jansteen (NL); VANNESTE, Stijn, B-8500 Kortrijk (BE); VAN RIE, Jeroen, B-9900 Eeklo (BE)
(86) International application number: PCT/EP2003/003068
(87) International publication number: WO 2003/080656

(56) References cited:
- WO-A-97/46105
- WO-A-99/33991
- US-A- 5 849 870
- US-B1- 6 204 435
- SELVAPANDIYAN ET AL.: "Toxicity analysis of N- and C- terminus-deleted vegetative insecticidal protein from Bacillus thuringiensis" APPLIED AND ENVIRONMENTAL MICROBIOLOGY, vol. 67, no. 12, December 2001 (2001-12), page 5855-5858 XP002251845

## Description

### For the following Contracting State(s) : AT, BE, BG, CH, CY, CZ, DE, DK, EE, ES, FI, FR, GB, GR, HU, IE, IT, LI, LU, MC, NL, PT, SE, SI, SK, TR

### Field of the invention

The present invention relates to the field of plant pest control, particularly insect control. Provided are new nucleic acid sequences from *Bacillus thuringiensis* (Bt) strains, encoding insecticidal proteins expressed during vegetative growth stages. Particularly, DNA sequences encoding a protein designated as ISP3-327D are provided, which are useful to protect plants from insect damage. Further provided are plants and microorganisms comprising at least one of the new nucleic acid molecules, as well as methods and means for using these nucleic acid sequences for reducing insect damage of plants.

### Background art

Insect pests cause huge economic losses worldwide in crop production, and farmers face every year the threat of yield losses due to insect infestation. Genetic engineering of insect resistance in agricultural crops has been an attractive approach to reduce costs associated with crop-management and chemical control practices. The first generation of insect resistant crops have been introduced into the market since 1996, based on the expression in plants of proteins isolated from the gram-positive soil bacterium *Bacillus thuringiensis* (Bt). The insecticidal Bt Cry proteins are produced during the sporulation-stage of Bt strains and the proteins accumulate in large cytoplasmic crystals within the bacterium. When taken up by insects, a typical Lepidopteran-toxic Bt Cry protein is solubilized and processed in the insect midgut into an active form of about 60 to 65kDa. The active protein exerts its toxic effect by binding to the midgut epithelial cells, causing pore formation in the cell membrane, which leads to osmotic lysis of the cells (Gill et al., 1992).

A Bt strain may produce many different toxins. Since the isolation of the first insecticidal crystal protein encoding gene from Bt in 1981 (Schnepf and Whiteley, 1981) more than 100 Bt Cry toxin encoding genes have been cloned and insect pests have been effectively controlled by expressing Bt derived proteins in agricultural important crop species. However, the use of individual Bt proteins is often limited, as most Bt proteins are mostly only active against a relatively small number of the numerous existing insect pests. Specificity of Bt Cry proteins is thought to be determined by factors such as the activation of the toxin in the insect gut (Haider et al. 1986) and its ability to bind specific receptors (Hofmann et al., 1988).

It is widely recognized that there is a risk that susceptible insect species may develop resistance against Bt Cry toxins. Consequently, active efforts have been made to identify novel insecticidal proteins. One strategy, which has been used, was to screen *Bacillus* strains for the production of insecticidal proteins during vegetative growth stages, rather than during sporulation stages. Using this approach a number of "vegetative insecticidal proteins" or "VIPs" have been identified.

Estruch et al. (1996), WO94/21795, WO96/10083, WO98/44137, US 5,877,012, US 6,107,279, US 6,137,033 and US 6,291,156 describe the isolation of *vip3A(a), vip3A(b)* and *vip3A(c)* from supernatant fluids of Bt strains AB88, AB424 and AB51. According to the authors these genes encode proteins with insecticidal activity towards a broad range of *Lepidopteran* insect pests.

WO98/18932 and WO99/57282 describe a number of nucleotide sequences isolated from Bt strains. These sequences are referred to as *mis* (*mis-1* to *mis-8), war* and *sup.* According to the authors the encoded proteins have activity against *Lepidopteran* or *Coleopteran* pests.

WO00/09697 describes heat-labile, soluble MIS-type and WAR-type toxins, as well as smaller (1 to 10kDa) toxins, obtainable from the supernatant of cultures of *Bacillus laterosporus* strains, which, according to the authors, have activity against Western Corn Rootworm larvae.

WO98/00546 and US 6,274,721 describe the isolation of Bt strains and Bt toxins, which, according to the authors, have activity against Lepidopteran pests.

WO99/33991 describes the isolation of Bt strains and Bt toxins, which, according to the authors, have activity against Lepidopteran pests.

Recently, Selvapandiyan et al. (2001) described the isolation of a gene encoding a protein designated as VIP-S. According to the authors the VIP-S protein showed toxicity against a number of Lepidopteran insect species.

Doss et al. (2002) describe the cloning of VIP3V from strain *Bt kurstaki.*

WO02/078437 describes VIP3 toxins from Bt, such as VIP3A, VIP3B and VIP3A-B hybrid toxins.

Despite the isolation and characterization of a relatively large number of different insecticidal proteins to date, there remains a need for identification, isolation and characterization of new insecticidal proteins. The reasons for this are manifold. Firstly, due to the specificity of insecticidal proteins towards particular groups of target pests (host insect spectra), there is a need to clone genes encoding proteins with different spectra of activity, so that for different crops and different geographic regions suitable proteins for combating insect pests are available. The specificity of Bt Cry proteins, for example, is mostly limited. Identification of toxins with specificity towards different target insects remains desirable. Secondly, after prolonged use in one geographic region, insects are known to have the capacity to develop resistance towards chemical insecticides, microbial sprays (for example based on Bt spore-crystal mixtures), and are believed to have the capacity to develop resistance towards plants expressing insecticidal proteins. The development of resistance within insect populations could potentially render existing insecticidal proteins ineffective, providing a need for novel genes and proteins. Thirdly, for health and environmental reasons it is desirable to identify proteins with high, specific insecticidal potency and acute bioactivity towards target insect species.

Hereinafter, including the different embodiments described in the claims, novel nucleic acid sequences and amino acid sequences isolated from *Bacillus thuringiensis* strains are described, which are useful to protect plants from insect damage, either by the expression of the nucleic acid sequences within plants under the control of suitable promoters, or by external application of the toxins to the plants. The toxins of the subject invention are distinct from previously described pesticidal toxins.

### Summary of the invention

In the following, the invention should be understood to be limited to the embodiments as described in the claims.

The invention provides insecticidal ISP3 proteins and nucleic acids encoding them. Provided is the insecticidal protein ISP3-327D (SEQ ID No. 4) and the nucleic acids encoding it, *isp3-327D* (SEQ ID No. 3). The protein of the invention has insecticidal activity against Lepidopteran insect pests, particularly against insects selected from the group consisting of *Helicoverpa zea, Helicoverpa armigera, Helicoverpa punctigera, Heliothis virescens, Ostrinia nubilalis, Spodoptera frugiperda, Agrotis ipsilon, Pectinophora gossypiella, Scirphophaga incertulas, Cnaphalocrocis medinalis, Sesamia inferens, Chilo partellus*, *Anticarsia gemmatalis, Plathypena scabra, Pseudoplusia includens, Spodoptera exigua, Spodoptera omithogalli, Epinotia aporema* and *Rachiplusia nu.*

In another embodiment of the invention insecticidal variants and fragments of the ISP3 protein, and of the nucleic acids encoding it, are provided.

In one embodiment, the claimed ISP3 protein of the invention is an isolated protein insecticidal to Ostrinia nubilalis, comprising the amino acid sequence of a variant of the protein of SEQ ID No. 4, wherein said variant has 5 to 10 amino acids added, replaced or deleted as compared to the protein of SEQ ID No. 4 without significantly changing the insecticidal activity of the protein; or said protein comprising the amino acid sequence of a variant of SEQ ID No. 4 wherein said variant has less than 5 amino acids added, replaced or deleted as compared to the protein of SEQ ID No. 4 without significantly changing the insecticidal activity of the protein.

In another embodiment, the claimed ISP3 protein is a protein that starts with a Met-Asp or Met-Ala dipeptide, by insertion of a codon encoding an Asp or Ala amino acid downstream of the start codon in the DNA encoding such protein, or is a protein comprising the amino acid sequence of SEQ ID No. 4.

A claimed ISP3 protein, as provided herein, is also an isolated protein insecticidal to Ostrinia nubilalis, comprising the amino acid sequence of the smallest fragment of the protein of SEQ ID No. 4 retaining insecticidal activity, which is obtained by enzymatic digestion of the protein of SEQ ID No. 4 with gut-juice fluid from Ostrinia nubilalis.

Further provided herein is a claimed ISP3 protein which is insecticidal against *Pectinophora gossypiella, Scirphophaga incertulas, Cnaphalocrocis medinalis, Sesamia inferens, Chilo partellus* and *Anticarsia gemmatalis.*

In a further embodiment of the invention, isolated nucleic acid sequences encoding the claimed ISP3 protein are provided, such as a nucleic acid sequence comprising nucleotides 1 to 2367 of SEQ ID No. 3.

In yet a further embodiment nucleic acid sequences encoding the claimed ISP3 proteins of the invention are provided, whereby the nucleic acid sequence is a synthetic sequence which has been optimized for expression in monocotyledonous or dicotyledonous plants or plant cells.

It is another objective of the invention to provide chimeric genes, comprising a promoter sequence operably linked to a nucleic acid sequence encoding an ISP3-327D protein, or insecticidally active variants or fragments thereof as claimed herein. Also provided are vectors comprising nucleotide sequences encoding ISP3 proteins, particularly ISP3-327D, or insecticidally active variants or fragments thereof as claimed herein.

The invention further provides host cells comprising chimeric genes, particularly microorganisms or transgenic plant cells, plant tissues, plant organs, plant seeds or whole plants comprising nucleotide sequences encoding the ISP3-327D protein, or insecticidally active fragments or variants thereof as claimed herein. In one embodiment the transformed plant is a maize or cotton plant. In another embodiment the transformed plant is a rice or soybean plant. In a further embodiment the transformed plant is any plant, particularly any plant selected from the group of sorghum, wheat, barley, rye, sunflower, sugarcane, tobacco, Brassica species (such as oilseed rape, mustard, cabbage, broccoli, etc.), vegetable species (tomato, cauliflower, radish, spinach, pepper, onion, bean, pea, carrot, etc.), sugar beet, tree species (apple, pear, plum, conifers, deciduous trees etc.), potato, alfalfa, mango, papaya, banana.

In another embodiment methods of protecting a plant against insect damage are provided, comprising contacting said plant with a claimed insecticidal ISP3 protein. The plant may be contacted with such ISP3 protein by transforming the plant with a nucleotide sequence encoding that ISP3 protein or by applying that ISP3 protein externally to the plant.

In one embodiment of the invention a Bt strain comprising the claimed *isp3* nucleic acid, particularly *isp3-327D,* is provided.

In a further embodiment, an insecticidal composition comprising an insecticidally effective amount of the claimed ISP3 protein is provided. When applied externally to a plant, the insecticidal composition increases resistance to insect damage compared to control plants, to which no such composition is applied.

Also provided herein is the use of the claimed ISP3 protein against a Lepidopteran cotton, maize, rice or soybean insect pest, particularly when said insect pest is *Helicoverpa zea, Heliothis virescens, Ostrinia nubilalis*, *Spodoptera frugiperda, Pectinophora gossypiella*, *Scirphophaga incertulas, Cnaphalocrocis medinalis, Sesamia inferens, Chilo partellus* and *Anticarsia gemmatalis.*

Further provided herein is a plant comprising the claimed chimeric gene, which simultaneously expresses with the protein of SEQ ID No. 4 or its fragment or variant: a Cry protein, a Cry1F protein, a hybrid derived from a Cry1F protein, a Cry1A-type protein or a toxic fragment thereof, a Cry1Ac protein or a hybrid derived therefrom, a Cry1Ab protein or an insecticidal fragment thereof, a Cry2Ae protein, a VIP3Aa protein or a toxic fragment thereof, or an insecticidal protein from *Xenorhabdus, Serratia* or *Photorhabdus* species strains; particularly such plant which is maize, rice, cotton or soybean, or a which is a hybrid plant.

Also provided herein is the use of the claimed ISP3 protein, co-expressed in maize, rice, cotton, or soybean plants, in combination with another insect control protein, wherein said other insect control protein is: a Cry1Ac protein, a Cry1Ab protein, a Cry2Ae protein, or a VIP3Aa protein or derivatives thereof.

Even further provided herein is the plant cell or the plant comprising the claimed chimeric gene, also comprising a PAT gene conferring resistance to glufosinate ammonium, or a 2mEPSPS gene conferring resistance to glyphosate.

### Detailed description of the embodiments

In the following, the invention should be understood to be limited to the embodiments as described in the claims.

The field of the invention is to provide methods and means for reducing damage caused to plants by pests, particularly insect pests, more particularly lepidopteran insect pests. Novel nucleic acid sequences and proteins have been identified and isolated, which are distinct from previously described nucleic acid sequences and proteins and which can be used for controlling insect pests by either integration and expression of at least one of these new nucleotide sequences in plants or plant cells or by external treatment of plants or plant parts with compositions comprising the toxins encoded by these nucleic acid molecules.

It is an embodiment of the invention to provide novel pesticidal toxins isolated from the *Bacillus thuringiensis* strains. Particularly, a pesticidal proteins designated as ISP3-327D protein is provided.

In accordance with this invention, a "nucleic acid sequence" refers to a DNA or RNA molecule in single or double stranded form, preferably a DNA or RNA, particularly a DNA, encoding any of the ISP3 proteins of this invention. An "isolated nucleic acid sequence", as used herein, refers to a nucleic acid sequence which is no longer in the natural environment where it was isolated from, e.g., the nucleic acid sequence in another bacterial host or in a plant nuclear genome.

In accordance with this invention, the terms "protein" or "polypeptide" are used interchangeably to refer to a molecule consisting of a chain of amino acids, without reference to any specific mode of action, size, three-dimensional structures or origin. Hence, a fragment or portion of an ISP3 protein of the invention is still referred to herein as a "protein". An "isolated protein", as used herein, refers to a protein which is no longer in its natural environment. The natural environment of the protein refers to the environment in which the protein could be found when the nucleotide sequence encoding it was expressed and translated in its natural environment, i.e. in the environment from which the nucleotide sequence was isolated. For example, an isolated protein can be present *in vitro,* or in another bacterial host or in a plant cell or it can be secreted from another bacterial host or from a plant cell.

In accordance with this invention, nucleic acid sequences, particularly DNA sequences, encoding new ISP3 proteins have been isolated and characterized. The new genes was designated *isp3-327D* and its encoded protein ISP3-327D.

In accordance with this invention "ISP3-327D protein" refers to any protein insecticidal to Ostrinia nubilalis comprising the amino acid sequence of the smallest fragment of the protein of SEQ ID No. 4 which retains insecticidal activity, which is obtained by enzymatic digestion of the protein of SEQ ID No. 4 with gut-juice fluid from Ostrinia nubilalis (hereinafter referred to as "smallest toxic fragment"). This includes hybrid- or chimeric proteins comprising the smallest toxic fragment. Also disclosed herein are claimed variants of the amino acid sequence in SEQ ID No. 4, such as essentially similar amino acid sequences, having a sequence identity of at least 91%, particularly at least 92%, 93%, 94%, 95%, 96%, 97%, 98% or 99% at the amino acid sequence level, as determined using pairwise alignments using the GAP program of the Wisconsin package of GCG (Madison, Wisconsin, USA, version 10.2). The GAP program is used with the following parameters for the amino acid sequence comparisons: the 'blosum62' scoring matrix, a 'gap creation penalty' (or 'gap weight') of 8 and a 'gap extension penalty' (or length weight') of 2. In one embodiment of the invention, an ISP3 protein variant insecticidal to Ostrinia nubilalis is provided that has 5-10, particularly less than 5, amino acids added, replaced or deleted without significantly changing, preferably without changing the insecticidal activity of the protein, or at least without changing the insecticidal activity of the protein in a negative way.

As used herein "comprising" is to be interpreted as specifying the presence of the stated features, integers, steps or components as referred to, but does not preclude the presence or addition of one or more features, integers, steps or components, or groups thereof. Thus, the term "DNA/protein comprising the sequence or region X", as used herein, refers to a DNA or protein including or containing at least the sequence or region X, so that other nucleotide or amino acid sequences can be included at the 5' (or N-terminal) and/or3' (or C-terminal) end, e.g. (the nucleotide sequence of) a selectable marker protein as disclosed in EP 0 193 259, (the nucleotide sequence of) a transit peptide, and/or a 5' or 3' leader sequence.

The "smallest toxic fragment" of an ISP3 protein of the invention, as used herein, is that smallest fragment or portion of an ISP3 protein retaining insecticidal activity that can be obtained by enzymatic digestion of the full length ISP3 protein (the same fragment or portion of an ISP3 protein retaining insecticidal activity can also be obtained by making nucleotide deletions in the DNA encoding an ISP3 protein). It is understood, that DNA encoding shorter toxic ISP3 fragments claimed herein may also be synthesized chemically and that the smallest toxic fragment obtainable from transcription and translation of synthetic DNA is included In the definition of smallest toxic fragment.

In one embodiment of the invention, the smallest toxic fragment of an ISP3 protein has a molecular weight of about 65kDa as determined by SDS-PAGE (sodium dodecyl sulfate-polyacrylamide gel electrophoresis) analysis. In another embodiment, the smallest toxic fragment of an ISP3 protein has a molecular weight of about 23kDa. In another embodiment, the smallest toxic fragment of an ISP3 protein has a molecular weight of about 33kDa. In a further embodiment, the smallest toxic fragment comprises the central amino acids of the ISP3 protein, in particular from amino acid 200 to amino acid 455 of SEQ ID No. 4.

Enzymatic digestions of ISP3 proteins can be performed by either using purified enzymes or using gut-juice fluids from insect larvae and incubating gut-juice extracts with solutions comprising one of the ISP3 proteins, as described in Yu et al. (1997). Proteolytic products can be separated and visualized on SDS-PAGE. Bioassays can be carried out with processed, chromatographically fractioned protein fragments in order to determine the relationship between each proteolytic fragment and its insecticidal activity. Preferred gut-juice used to determine the smallest toxic fragment of ISP3 proteins is gut-juice from Lepidopteran insects, preferably from Corn Earworm *(Helicoverpa zea),* Cotton Bollworm *(Helicoverpa armigera),* Native Budworm (*Helicoverpa punctigera*), Tobacco Budworm (*Heliothis virescens*), European Corn Borer (*Ostrinia nubilalis*), Fall Armyworm *(Spodoptera frugiperda),* Black Cutworm *(Agrotis ipsilon),* Pink Bollworm *(Pectinophora gossypiella),* Yellow Stem Borer (*Scirphophaga incertulas*), Leaffolder *(Cnaphalocrocis medinalis),* Pink Stem Borer *(Sesamia inferens),* Com Spotted Stem Borer *(Chilo partellus),* Velvet Caterpillar (*Anticarsia gemmatalis*), Soybean Looper *(Pseudoplusia includens),* Pod Borer *(Epinotia aporema), Rachiplusia nu.*

The N- and C-terminal amino acid sequence ends of the smallest toxic fragment are conveniently determined by amino acid sequence determination of the above fragments by techniques routinely available in the art.

As used herein, the term *"isp3-327D"* refers to any DNA sequence encoding the "ISP3-327D protein", as defined above. This includes naturally occurring, artificial or synthetic DNA sequences encoding the proteins of SEQ ID No. 4, or its insecticidal fragments or variants as claimed herein. Also included herein are DNA sequences, encoding the claimed insecticidal proteins, which are similar enough to the DNA sequences provided in the sequence listing so that they can (i.e., have the ability to) hybridize to these DNA sequences under stringent hybridization conditions.

"Stringent hybridization conditions", as used herein, refers particularly to the following conditions: immobilizing the relevant DNA on a filter, and prehybridizing the filters for either 1 to 2 hours in 50 % formamide, 5x SSPE, 2x Denhardt's reagent and 0.1 % SDS at 42 ° C or 1 to 2 hours in 6x SSC, 2xDenhardt's reagent and 0.1 % SDS at 68 °C. The denatured (Digoxigenin- or radio-) labeled probe is then added directly to the prehybridization fluid and incubation is carried out for 18 to 24 hours at the appropriate temperature mentioned above. After incubation, the filters are then washed for 30 minutes at room temperature in 2x SSC, 0.1 % SDS, followed by 2 washes of 30 minutes each at 68 °C in 0.5 x SSC and 0.1 % SDS. An autoradiograph is established by exposing the filters for 24 to 48 hours to X-ray film (Kodak XAR-2 or equivalent) at -70 °C with an intensifying screen. [20x SSC = 3M NaCl and 0.3M sodiumcitrate; 100x Denhart's reagent= 2%(w/v) bovine serum albumin, 2%(w/v) Ficoll^{™} and 2% (w/v) polyvinylpyrrolidone; SDS = sodium dodecyl sulfate; 20x SSPE= 3.6M NaCl, 02M Sodium phosphate and 0.02M EDTA pH7.7]. Of course, equivalent conditions and parameters can be used in this process while still retaining the desired stringent hybridization conditions.

It is clear that there are many approaches known in the art for the isolation of variants of the DNA sequences of the invention. Variants can, for example, be isolated from Bt strains by hybridization as described supra, and/or by PCR technology as known in the art. Specific or degenerate primers can be made to regions of the *isp3* DNA sequences, and used to amplify variants from known or novel Bt strains.

Preferred variants of the *isp3-327D* DNA of this invention are DNA sequences encoding the claimed ISP3-327D protein variants described above, or a DNA sequence, encoding such claimed ISP3 protein, with at least 94%, preferably at least 95%, particularly at least 96%, 97%, 98% or at least 99% sequence identity to SEQ ID No. 3. The sequence identities referred to above are calculated using the GAP program of the Wisconsin package of GCG (Madison, Wisconsin, USA) Version 10.2. The GAP program is used with the following parameters for nucleic acids: the "'nwsgapdna" scoring matrix, a 'gap creation penalty' (or 'gap weight') of 50 and a 'gap extension penalty' (or length weight') of 3. Stringent hybridization conditions are as defined above.

"Insecticidal activity" of a protein, as used herein, means the capacity of a protein to kill insects when such protein is fed to insects, preferably by expression in a recombinant host such as a plant. It is understood that a protein has insecticidal activity if it has the capacity to kill the insect during at least one of its developmental stages, preferably the larval stage.

"Insect-controlling amounts" of a protein, as used herein, refers to an amount of protein which is sufficient to limit damage on a plant, caused by insects (e.g. insect larvae) feeding on such plant, to commercially acceptable levels, e.g. by killing the insects or by inhibiting the insect development, fertility or growth in such a manner that they provide less damage to a plant and plant yield is not significantly adversely affected.

In accordance with this invention, insects susceptible to the new ISP3 proteins of the invention are contacted with this protein in Insect-controlling amounts, preferably insecticidal amounts. Preferred target insects for the proteins of this invention are economically damaging insect pests of corn, cotton, rice or soybean plants, particularly in Northern and Southern American countries, Asia and Australia. The term plant, as used herein, encompasses whole plants as well as parts of plants, such as leaves, stems, seeds, flowers or roots. Particularly preferred target insects for the ISP3 proteins of this invention are leptidopteran insect pests, such as Heliothis spp., Helicoverpa spp., Spodoptera spp., Ostrinia spp., Pectinophora spp, Agrotis spp., Scirphophaga spp., Cnaphalocrocis spp., Sesamia spp, Chilo spp., Anticarsia spp., Pseudoplusia spp., Epinotia spp., and Rachiplusia spp., preferably *Heliothis virescens, Helicoverpa zea, Helicoverpa armigera, Helicoverpa punctera, Ostrinia nubilalis, Spodoptera frugiperda, Agrotis ipsilon, Pectinophora gossypiella, Scirphophaga incertulas, Cnaphalocrocis medinalis, Sesamia inferens, Chilo partellus, Anticarsia gemmatalis, Pseudoplusia includens, Epinotia aporema* and *-Rachiplusia nu.* The-ISP3 proteins of-the-invention-preferably have insecticidal activity against at least one lepidopteran insect species, more preferably against several Lepidopteran insect species.

The terms "ISP3 protein", "ISP3 protein of this invention", "ISP protein", or "ISP protein of this invention", as used herein, refers to the new protein isolated in accordance with this invention and identified and defined herein as ISP3-327D protein.

An ISP3 protein, as used herein, can be a protein in the full length size or can be in a truncated form as long as the insecticidal activity is retained, or can be a combination of different proteins or protein domains in a hybrid or fusion protein. An "ISP3 toxin" refers to an insecticidal fragment or portion of an ISP3, protein, particularly the smallest toxic fragment thereof. An *"isp* gene", *"isp3* gene", *"isp* DNA" or "*isp3* DNA", as used herein, is a DNA sequence encoding an ISP3 protein in accordance with this invention, referring particularly to the *isp3-327D* DNA sequences defined above.

The nucleic acid sequence, particularly DNA sequence, encoding the ISP3 proteins of this invention can be made synthetically and can be inserted in expression vectors to produce high amounts of ISP3 proteins. The ISP3 proteins can be used to prepare specific monoclonal or polyclonal antibodies in a conventional manner (Höfte et al., 1988; Harlow and Lane, 1988).

Also disclosed herein are antibodies that specifically bind to the ISP3 protein. In particular, monoclonal or polyclonal antibodies that bind ISP3-327D, or to fragments or variants thereof are disclosed. Included are fragments of monoclonal or polyclonal antibodies, which retain the ability to bind to the ISP3 protein or fragment against which they were raised. An antibody to an ISP3 protein can be prepared by using the ISP3 protein as an antigen in an animal (such as rabbit or mouse), using methods known in the art, such as described in Harlow and Lane "Using Antibodies: A Laboratory Manual" (New York: Cold Spring Harbor Laboratory Press, 1998), in Liddell and Cryer "A Practical Guide to Monoclonal Antibodies" (Wiley and Sons, 1991). The antibodies can be used to isolate, identify, characterize or purify the ISP3 protein to which it binds. For example, the antibody can be used to detect the ISP3 protein in a sample, by allowing antibody and protein to form an immunocomplex, and detecting the presence of the immunocomplex, for example through ELISA or immunoblots.

In addition, immunological kits, useful for the detection of ISP3 proteins, protein fragments or epitopes in a sample are provided. Samples may be cells, cell supernatants, cell suspensions, and the like. Such kit comprises an antibody that binds to the ISP3 protein (or fragment thereof) and one or more immunodetection reagents.

The antibodies can also be used to isolate insecticidal proteins with similar activity by for example ELISA (enzyme linked immunoassay) or Western blotting. Monoclonal antibody lines with desired binding specificity can also be used to clone the DNA for the particular monoclonal antibody.

Also disclosed herein are PCR primers and/or probes and kits for detecting the isp3-237D DNA sequences are provided. PCR primer pairs to amplify isp3 DNA from samples can be synthesized based on SEQ ID No. 3, as known in the art (see Dieffenbach and Dveksler (1995) PCR Primer: A Laboratory Manual, Cold Spring Harbor Laboratory Press, and McPherson at al. (2000) PCR- Basics: From Background to Bench, First Edition, Springer Verlag, Germany). Likewise, DNA fragments of SEQ ID No. 3 can be used as hybridization probes. An isp3 detection kit may comprise either isp3 specific primers or isp3 specific probes, and an associated protocol to use the primers or probe to detect isp3 DNA in a sample. Such a detection kit may, for example, be used to determine, whether a plant has been transformed with an isp3 gene (or part thereof) of the invention.

Because of the degeneracy of the genetic code, some amino acid codons can be replaced by others without changing the amino acid sequence of the protein. Furthermore, some amino acids can be substituted by other equivalent amino acids without significantly changing, preferably without changing, the insecticidal activity of the protein, at least without changing the insecticidal activity of the protein in a negative way. For example, to obtain a claimed ISP3 protein, conservative amino acid substitutions within the categories basic (e.g. Arg, His, Lys), acidic (e.g. Asp, Glu), nonpolar (e.g. Ala, Val, Trp, Leu, Ile, Pro, Met, Phe, Trp) or polar (e.g. Gly, Ser, Thr, Tyr, Cys, Asn, Gln) fall within the scope of the invention as long as the insecticidal activity of the ISP3 protein is not significantly, preferably not, changed, at least not changed in a negative way. In addition, in the claimed ISP3 proteins, non-conservative amino acid substitutions fall within the scope of the invention as long as the insecticidal activity of the ISP3 protein is not changed significantly, preferably not, or at least is not changed in a negative way. Variants or equivalents of the DNA sequences of the invention include DNA sequences hybridizing to the *isp3* DNA sequence of SEQ ID No. 3 under stringent hybridization conditions and encoding a claimed ISP3 protein, or DNA sequences having a different codon usage compared to the native *isp3* genes of this invention but which encode a claimed ISP3 protein. The *isp3* DNA sequences can be codon-optimized by adapting the codon usage to that most preferred in plant genes, particularly to genes native to the plant genus or species of interest (Bennetzen & Hall, 1982; Itakura et al., 1977) using available codon usage tables (e.g. more adapted towards expression in cotton, soybean corn or rice). Codon usage tables for various plant species are published for example by Ikemura (1993) and Nakamura et al. (2000).

Also long stretches of AT or GC nucleotides may be removed and suitable restriction sites may be introduced.

Also, the N-terminus of an ISP3 protein can be modified to have an optimum translation initiation context, thereby adding or deleting one or more amino acids at the N-terminal end of the protein. In most cases, it is preferred that the proteins of the invention to be expressed in plants-cells start with a Met-Asp or Met-Ala dipeptide for optimal translation initiation, requiring the insertion in the *isp3* DNA of a codon encoding an Asp or Ala amino acid downstream of the start codon as a new second codon. Alternatively, the fourth nucleotide of SEQ ID No. 3 may be replaced by a 'G', so that the second amino acid (following Met) is Asp. Likewise, the second codon (AAC or AAT, coding for Asn) may be replaced by a codon for Asp (GAT or GAC) or Ala (GCT, GCC, GCA or GCG), or by any other codon starting with a 'G'.

The DNA sequences may also be modified to remove illegitimate splice sites. As bacterial genes may contain motifs, which are recognised in other hosts, especially in eukaryotic host such as plants, as 5' or 3' splice sites, transcription in those other hosts may be terminated prematurely, resulting in truncated mRNA. Illegitimate splice sites can be identified by computer based analysis of the DNA sequences and/or by PCR analysis as known in the art.

Of course, any DNA sequence differing in its codon usage but encoding the same protein or a similar protein with substantially the same insecticidal activity, can be constructed, depending on the particular purpose. It has been described in prokaryotic and eukaryotic expression systems that changing the codon usage to that of the host cell is desired for gene expression in foreign hosts (Bennetzen & Hall, 1982; Itakura et al., 1977). Codon usage tables are available in the literature (Wada et al., 1990; Murray et al., 1989) and in the major DNA sequence databases (e.g. EMBL at Heidelberg, Germany) and as described by Nakamura et al (2000). Accordingly, synthetic DNA sequences can be constructed so that the same or substantially the same proteins are produced. It is evident that several DNA sequences can be made once the amino acid sequence of the ISP3 proteins of this invention is known. Such other DNA sequences include synthetic or semi-synthetic DNA sequences that have been changed in order to inactivate certain sites in the gene, e.g. by selectively inactivating certain cryptic regulatory or processing elements present in the native sequence as described in PCT publications WO 91/16432 and WO 93/09218, or by adapting the overall codon usage to that of a more related host organism, preferably that of the host organism in which expression is desired. Several techniques for modifying the codon usage to that preferred by the host cells can be found in patent and scientific literature. The exact method of codon usage modification is not critical for this invention as long as most or all of the cryptic regulatory sequences or processing elements have been replaced by other sequences.

Small modifications to a DNA sequence such as described above can be routinely made, i.e., by PCR-mediated mutagenesis (Ho et al., 1989, White et al., 1989). More profound modifications to a DNA sequence can be routinely done by *de novo* DNA synthesis of a desired coding region using available techniques.

With the term "substantially the same", when referring to the amino acid sequence of an ISP3 protein, is meant to include an amino acid sequence that differs no more than 5 %, preferably no more than 2 %, from the amino acid sequence of the protein compared to; and when referring to toxicity of an ISP3 protein, is meant to include a protein whose mean LC₅₀ value (which is the concentration of protein causing 50% mortality of the test population), calculated from three independent bioassays, carried out using the same bioassay-conditions, differs by no more than a factor 2 from the mean LC₅₀ value obtained for the protein compared to (also calculated from three independent bioassays, carried out using the same bioassay-conditions as for the protein compared to) LC₅₀ values are calculated with Probit analyis, using the program POLO PC (from LeOra Software, 1987, Berkely, California). It is understood, that 95% (or 90%) confidence limits (an associated parameter calculated with Probit analysis) are calculated for the LC₅₀ values of each of the two proteins to be compared in order to determine whether a statistically significant difference in LC₅₀ values exists. In general the toxicity of the two proteins is seen to be substantially the same, if the confidence limits overlap and substantially different if the confidence limits do not overlap.

The term "domain" of a ISP3 toxin (or ISP3 protein) as used herein means any part(s) or domain(s) of the toxin (or ISP3 protein) with a specific structure or function that can be transferred to another protein for providing a new hybrid protein with at least one functional characteristic (e.g., the binding and/or toxicity characteristics) of the ISP3 toxin (or ISP3 protein) of the invention (Ge et al., 1991). Such parts can form an essential feature of the hybrid protein with the binding and/or toxicity characteristics of the ISP3 proteins of this invention. Such a hybrid protein can have an enlarged host range, an improved toxicity and/or can be used in a strategy to prevent insect resistance development (EP 408 403; Visser et al., 1993). DNA sequences encoding the domains are encompassed by this definition. A hybrid protein or fusion protein is used herein to mean a protein comprised of different protein domains, forming a functional, chimeric protein with the characteristics of the individual domains. Another domain which a hybrid or chimeric protein may, for example, comprise is a stabilising domain. Stabilising domains have for example been described to be present at the C-terminus of VIP3(a) proteins, and are thoughtto provide stability to the toxic protein in the gut-environmentofsusceptible insects.

In addition to creating hybrid proteins, the function of specific domains can also be analyzed by the introduction of deletions of all or part of the domain(s) or the introduction of mutations into the domain, and analysis of the resulting effect on toxicity towards insects, protein stability, sensitivity to enzyme proteolysis, temperature changes, binding to DNA/proteins/specific cells, etc..

Also disclosed herein is a method of "evolving" a nucleic acid sequence-encoding an ISP3 protein, particularly ISP3-327D, into a new nucleic acid sequence, which encodes a protein as claimed having insecticidal activity. The evolved nucleic acid sequence preferably has improved insecticidal activity compared to the non-evolved sequence. The term "evolving" as used herein refers to a method of enhanced sequence evolution by recombination of sequences, as described in US 5,811,238, WO97/20078 and US 6,180,406. Nucleic acid "shuffling" is used herein to indicate in vitro or in vivo recombination between nucleic acid sequences of a nucleic acid population or pool and can be carried out as known in the art and as described In US 5,811,238, WO97/20078, US 6,180,406, US 6,117,679.

The method of evolving a nucleic acid sequence encoding a claimed ISP3 protein comprises the following steps:
(a) providing a population of nucleic acid sequences encoding the amino acid sequences of SEQ ID No. 4, or variants or fragments of the amino acid sequences of SEQ ID No. 4, wherein said variants or fragments have a sequence identity of at least 91 % to SEQ ID No. 4.
(b) shuffling said population of variants or fragments to form recombinant nucleic acid molecules
(c) selecting or screening for recombinant nucleic acid molecules, which encode claimed proteins that have insecticidal activity;
repeating steps (a) to (c) with the recombinant nucleic acid molecules selected in step (c) until a recombinant nucleic acid molecule has been found in step (c), wherein the claimed protein encoded by said nucleic acid molecule has the desired insecticidal property.

A non-evolved nucleic acid is a nucleic acid provided as starting material in step (a), while a corresponding evolved nucleic acid as used herein refers to a recombinant nucleic acid obtained in step (d) when carrying out the method using the non-evolved nucleic acid in step (a). Preferred nucleic acids used in step (a) are nucleic acid sequences encoding amino acid sequences ISP3-327D (SEQ ID No. 4) or variants or fragments thereof as claimed herein. The population of nucleic acid molecules and/orvariants and/orfragments of nucleic acid molecules in step (a) may comprise the DNA encoding a single ISP3 protein and/or variants and/or fragments of the nucleic acid encoding a single ISP3 protein of the invention, or a mixture of nucleic acids encoding different ISP3 proteins of the invention, and/orfragments and/or variants thereof.

Nucleic acid sequences encoding claimed variants of amino acid sequence SEQ ID No. 4 are nucleic acid sequences encoding amino acid sequences of claimed ISP3 proteins, e.g. those claimed ISP3 proteins which have at least 91%, preferably at least 92 or 93%, most preferably at least 94%, 95%, 98%, 99% or 100% sequence identity at the amino acid level to SEQ ID No. 4.

The *isp3* DNA sequences of the invention, prepared from total DNA, can be ligated in suitable expression vectors and transformed in a bacterial strain, such as *E. coli* or a Bt strain, and the clones can then be screened by conventional colony immunoprobing methods (French et al., 1986) for expression of the toxin with monoclonal or polyclonal antibodies raised against the ISP3 proteins.

The Bt or *E*. *coli* clones can then be screened for production of ISP3 proteins (cell-free culture supernatant or cell lysate can be run on SDS-PAGE gels using standard methods and standard western-blotting procedures can be carried out), or the bacteria can be tested for their insecticidal activity compared to the control bacteria. The clones can also be analysed for the presence of mRNA encoding ISP3 protein using standard PCR procedures, such as RT-PCR.

The genes encoding the ISP3 proteins of this invention can be sequenced in a conventional manner (Maxam and Gilbert, 1980; Sanger, 1977) to obtain the DNA sequence. Sequence comparisons indicated that the genes are different from previously described genes encoding toxins with activity against Lepidoptera.

An insecticidally effective part of the DNA sequences, encoding an insecticidally effective portion of the newly identified ISP3 proteins, can be made in a conventional manner after sequence analysis of the gene. The amino acid sequence of the ISP3 proteins can be determined from the DNA sequence of the isolated DNA sequences. By "an insecticidally effective part (or portion or fragment)" of DNA sequences encoding the ISP3 protein, also referred to herein as "truncated gene" or "truncated DNA", is meant a DNA sequence encoding a polypeptide which has fewer amino acids than the ISP3 full length protein form but which is insecticidal.

In order to express all or an insecticidally effective part of the DNA sequence encoding an ISP3 protein of this invention In *E. coli,* in other *Bt* strains and In plants, suitable restriction sites can be introduced, flanking the DNA sequence. This can be done by site-directed mutagenesis, using well-known procedures (Stanssens et al., 1989; White et al., 1989). In order to obtain improved expression in plants, the codon usage of the *isp3* gene or insecticidally effective *isp3* gene part of this invention can be modified to form an equivalent, modified or artificial gene or gene part in accordance with PCT publications WO 91 /16432 and WO 93/09218 and publications EP 0 385 962, EP 0 359 472 and US 5,689,052, or the *isp3* genes or gene parts can be inserted in the plastid, mitochondrial or chloroplast genome and expressed there using a suitable promoter (e.g., Mc Bride et al., 1995; US 5,693,507).

For obtaining enhanced expression in monocot plants such as corn or rice, an intron, preferably a monocot intron, can also be added to the chimeric gene. For example the insertion of the intron of the maize Adh1 gene into the 5' regulatory region has been shown to enhance expression in maize (Callis et. al., 1987). Likewise, the HSP70 intron, as described in US 5,859,347, may be used to enhance expression. The DNA sequence of the *isp3*gene or its insecticidal part can be further changed in a translationally neutral manner, to modify possibly inhibiting DNA sequences present in the gene part by means of site-directed intron insertion and/or by introducing changes to the codon usage, e.g., adapting the codon usage to that most preferred by plants, preferably the specific relevant plant genus (Murray et al., 1989), without changing significantly, preferably without changing, the encoded amino acid sequence.

In accordance with one embodiment of this invention, it is preferred that the proteins are targeted to intracellular organelles such as plastids, preferably chloroplasts, mitochondria, or are secreted from the cell, potentially optimizing protein stability and/or expression. For this purpose, in one embodiment of this invention, the chimeric genes of the invention comprise a coding region encoding a signal or target peptide, linked to the ISP3 protein coding region of the invention. Particularly preferred peptides to be included in the proteins of this invention are the transit peptides for chloroplast or other plastid targeting, especially duplicated transit peptide regions from plant genes whose gene product is targeted to the plastids, the optimized transit peptide of Capellades et al. (US 5,635,618), the transit peptide of ferredoxin-NADP⁺oxidoreductase from spinach (Oelmuller et al., 1993), the transit peptide described in Wong et al.

(1992) and the targeting peptides in published PCT patent application WO 00/26371. Also preferred are peptides signalling secretion of a protein linked to such peptide outside the cell, such as the secretion signal of the potato proteinase inhibitor II (Keil et al., 1986), the secretion signal of the alpha-amylase 3 gene of rice (Sutliff et al., 1991) and the secretion signal of tobacco PR1 protein (Cornelissen et al., 1986).

Particularly useful signal peptides in accordance with the invention include the chloroplast transit peptide (e.g., Van Den Broeck et al., 1985), or the optimized chloroplast transit peptide of US 5,510,471 and US 5,635,618 causing transport of the protein to the chloroplasts, a secretory signal peptide or a peptide targeting the protein to other plastids, mitochondria, the ER, or another organelle. Signal sequences for targeting to intracellular organelles or for secretion outside the plant cell or to the cell wall are found in naturally targeted or secreted proteins, preferably those described by Klösgen et al. (1989), Klösgen and Weil (1991), Neuhaus & Rogers (1998), Bih et al. (1999), Morris et al. (1999), Hesse et al. (1989), Tavladoraki et al. (1998), Terashima et al. (1999), Park et al. (1997), Shcherban et al. (1995), particularly the signal peptide sequences from targeted or secreted proteins of corn, cotton, soybean or rice.

To allow secretion of the ISP3 proteins to the outside of the transformed host cell, an appropriate secretion signal peptide may be fused to the amino terminal end (N-terminal end) of the ISP3 protein. Also, any putative native *Bacillus* secretion signal peptide can be deleted or can be replaced by an appropriate signal peptide, such as a eukaryotic secretion signal peptide as described above. Particularly, amino acids 1 to 54 of the ISP3 proteins of the invention comprise a putative *Bacillus* signal peptide. Amino acids 1 to 10, preferably 1 to 50, more preferably 1 to 54 may be removed from the ISP3 proteins or may be replaced by an appropriate signal peptide, particularly a eukaryotic signal peptide as described above. Putative signal peptides can be detected using computer based analysis, using programs such as the program Signal Peptide search (SignalP V1.1 or 2.0), using a matrix for prokaryotic gram-positive bacteria and a threshold score of less than 0.5, especially a threshold score of 0.25 or less (Von Heijne, Gunnar, 1986 and Nielsen et al., 1996).

Furthermore, the binding properties of the ISP3 proteins of the invention can be evaluated, using methods known in the art (e.g., Van Rie et al., 1990), to determine if the ISP3 proteins of the invention bind to sites in the insect gut, such as the midgut, that are not recognized (or competed for) by other *Bt* proteins. Insecticidal *Bt* proteins with different binding sites for which there is no competition for binding in relevant susceptible insects are very valuable to replace known Bt proteins to which insects may have developed resistance, or to use in combination with insecticidal *Bt* proteins having a different mode of action to prevent or delay the development of insect resistance against *Bt* proteins, particularly when expressed in a plant. Because of the characteristics of the newly isolated *Bt* toxins, they are extremely useful for transforming plants, e.g. monocots such as corn and rice and dicots such as cotton and soybean, to protect these plants from insect damage. It is expected that the binding properties of the ISP3 proteins of the current invention will be different compared to those of Cry toxins. Such different binding properties can be measured by routine binding assays as described above or in US 6,291,156 and US 6,137,033.

Especially for insect resistance management purposes for a specific insect pest, it is preferred to combine an ISP3 protein of this invention with another insect control protein, particularly a Bt Cry protein or a VIP or VIP-like protein, preferably a protein which does not recognise at least one binding site recognised by such ISP3 protein. Preferred insect control proteins to combine with the ISP3 proteins of this invention, particularly for simultaneous expression in plants, preferably maize, cotton, rice or soybean plants, include the Cry proteins, such as the Cry1 F protein or hybrids derived from a Cry1 F protein (e.g., the hybrid Cry1A-Cry1F proteins described in US 6,326,169; US 6,281,016; US 6,218,188, or toxic fragments thereof), the Cry1A-type proteins ortoxic fragments thereof, preferably the Cry1Ac protein or hybrids derived from the Cry1Ac protein (e.g., the hybrid Cry1Ab-Cry1Ac protein described in US 5,880,275) or the Cry1Ab or Bt2 protein or insecticidal fragments thereof as described in EP451878, the Cry2Ae, Cry2Af or Cry2Ag proteins as described in WO02/057664, the Cry proteins as described in WO01/47952, the VIP3Aa protein or a toxic fragment thereof as described in Estruch et al. (1996) and US 6,291,156, insecticidal proteins from *Xenorhabdus* (as described in WO98/50427), *Serratia* (particularly from *S. entomophila)* or *Photorhabdus* species strains, such as Tc-proteins from Photorhabdus as described in WO98/08932 (e.g., Waterfield et al., 2001; Ffrench-Constant and Bowen, 2000). In one embodiment, such co-expression is easily obtained by transforming a plant already expressing an insect control protein with a ISP3 of this invention, or by crossing plants transformed-with the insect control protein and plants transformed with one or more ISP proteins of this invention. For maize, rice, cotton or soybean plants, preferably the ISP3-327D protein is used as first insect control protein and as second insect control protein the Cry1 Ab, Cry1 Ac, Cry2Ae or VIP3Aa proteins or derivatives thereof are used. Methods for obtaining expression of different Bt (or similarly, for other insect control proteins) insecticidal proteins in the same plant in an effort to minimize or prevent resistance development to transgenic insect-resistant plants are described in EP 0 408 403. It is understood that the different proteins can be expressed in the same plant, or each can be expressed in a single plant and then combined in the same plant by crossing the single plants with one another. For example, in hybrid seed production, each parent plant can express a single protein. Upon crossing the parent plants to produce hybrids, both proteins are combined in the hybrid plant.

It is well known that Bt Cry proteins are expressed as protoxins, which are converted into the toxic core by proteolysis in the insect gut. When combining the ISP3 proteins of the invention with Bt Cry proteins, it is understood that Bt Cry genes either encoding the full protoxin or the toxic core or any intermediate form may be used.

Preferably, for selection purposes but also for increasing the weed control options, the transgenic plants of the invention are also transformed with a DNA encoding a protein conferring resistance to a broad-spectrum herbicide, e.g., herbicides based on glufosinate or glyphosate.

The insecticidally effective *isp3* gene part or its equivalent, preferably the *isp3* chimeric gene, encoding an insecticidally effective portion of the ISP3 protein, can be stably inserted in a conventional manner into the nuclear genome of a single plant cell, and the so-transformed plant cell can be used in a conventional manner to produce a transformed plant that is insect-resistant. In this regard, a T-DNA vector, containing the insecticidally effective *isp3* gene part, in *Agrobacterium tumefaciens* can be used to transform the plant cell, and thereafter, a transformed plant can be regenerated from the transformed plant cell using the procedures described, for example, in EP 0 116 718, EP 0 270 822, PCT publication WO 84/02913 and published European Patent application EPO 242 246 and in Gould et al. (1991). The construction of a T-DNA vector for Agrobacterium mediated plant transformation is well known in the art. The T-DNA vector may be either a binary vector as described in EP 0120 561 and EP 0 120 515 or a co-integrate vector which can integrate into the Agrobacterium Ti-plasmid by homologous recombination, as described in EP 0116 718. Preferred T-DNA vectors each contain a promoter operably linked to the insecticidally effective *isp3* gene part between T-DNA border sequences, or at least located to the left of the right border sequence. Border sequences are described in Gielen et al. (1984). Of course, other types of vectors can be used to transform the plant cell, using procedures such as direct gene transfer (as described, for example in EP 0 223 247), pollen mediated transformation (as described, for example in EP 0 270 356 and WO 85/01856), protoplast transformation as, for example, described in US 4,684,611, plant RNA virus-mediated transformation (as described, for example in EP 0 067 553 and US 4,407,956), liposome-mediated transformation (as described, for example in US 4,536,475), and other methods such as the recently described methods for transforming certain lines of corn (e.g., US 6,140,553; Fromm et al., 1990; Gordon-Kamm et al., 1990) and rice (Shimamoto et al., 1989; Datta et al. 1990) and the method for transforming monocots generally (PCT publication WO 92/09696). For cotton transformation, especially preferred is the method described in PCT patent publication WO 00/71733. For rice transformation, reference is made to the methods described in WO92/09696, WO94/00977 and WO95/06722.

The terms "maize" and "corp" are used herein synonymously, referring to *Zea mays.* Cotton as used herein refers to *Gossypium* spp., particularly *G. hirsutum* and *G. barbadense.* The term "rice" refers to *Oryza* spp., particularly *O. sativa.* "Soybean" refers to *Glycine* spp, particularly *G. max*

Besides transformation of the nuclear genome, also transformation of the plastid genome, preferably chloroplast genome, is included In the invention. Kota et al. (1999) have described a method to over-express a Cry2Aa protein in tobacco chloroplasts.

The resulting transformed plant can be used in a conventional plant breeding scheme to produce more transformed plants with the same characteristics or to Introduce the insecticidally effective *isp3* gene part into other varieties of the same or related plant species. Seeds, which are obtained from the transformed plants, contain the insecticidally effective *isp3* gene part as a stable genomic insert. Cells of the transformed plant can be cultured in a conventional manner to produce the insecticidally effective portion of the ISP3 toxin or protein, which can be recovered for use in conventional insecticide compositions against Lepidoptera (US 5,254,799).

The insecticidally effective *isp3* gene part is inserted in a plant cell genome so that the inserted gene is downstream (i.e., 3') of, and under the control of, a promoter which can direct the expression of the gene part in the plant cell. This is preferably accomplished by inserting the *isp3* chimeric gene in the plant cell genome, particularly in the nuclear or plastid (e.g., chloroplast) genome.

Preferred promoters include: the strong constitutive 35S promoters (the °35S promoters') of the cauliflower mosaic virus (CaMV) of isolates CM 1841 (Gardner et al., 1981), CabbB-S (Franck et al., 1980) and CabbB-JI (Hull and Howell, 1987); the 35S promoter described by Odell et al. (1985), promoters from the ubiquitin family (e.g., the maize ubiquitin promoter of Christensen et al., 1992, EP 0 342 926; see also Cornejo et al., 1993), the gos2 promoter (de Pater et al., 1992), the emu promoter (Last et al., 1990), Arabidopsis actin promoters such as the promoter described by An et al. (1996), rice actin promoters such as the promoter described by Zhang et al. (1991) and the promoter described in US 5,641,876; promoters of the Cassava vein mosaic virus (WO 97/48819, Verdaguer et al. (1998)), the pPLEX series of promoters from Subterranean Clover Stunt Virus (WO 96/06932, particularly the S7 promoter), a alcohol dehydrogenase promoter, e.g., pAdh1S (GenBank accession numbers X04049, X00581), and the TR1' promoter and the TR2' promoter (the "TR1' promoter" and 'TR2' promoter", respectively) which drive the expression of the 1' and 2' genes, respectively, of the T-DNA (Velten et al., 1984). Alternatively, a promoter can be utilized which is not constitutive but rather is specific for one or more tissues or organs of the plant (e.g., leaves and/or roots) whereby the inserted *isp3* gene part is expressed only in cells of the specific tissue(s) or organ(s). For example, the insecticidally effective *isp3* gene part could be selectively expressed in the leaves of a plant (e.g., corn, cotton, rice, soybean) by placing the insecticidally effective gene part under the control of a light-inducible promoter such as the promoter of the ribulose-1,5-bisphosphate carboxylase small subunit gene of the plant itself or of another plant, such as pea, as disclosed in US 5,254,799. The promoter can, for example, be chosen so that the *isp3* gene of the invention is only expressed in those tissues or cells on which the target insect pest feeds so that feeding by the susceptible target insect will result in reduced insect damage to the host plant, compared to plants which do not express the *isp3* gene. A Lepidopteran insect pest mainly damaging the roots can thus effectively be controlled by expressing an *isp3* gene under a root specific promoter. A promoter preferentially active in roots is described in WO00/29566. A preferred promoter for rootpreferential expression is the ZRP promoter (and modifications thereof) as described in US 5,633,363. Another alternative is to use a promoter whose expression is inducible, for example a wound-inducible promoter such as e.g. the MPI promoter described by Cordera et al. (1994), which is induced by wounding (such as caused by insect feeding), or a promoter inducible by a chemical, such as dexamethasone as described by Aoyama and Chua (1997) or a promoter inducible by temperature, such as the heat shock promoter described in US 5,447,858, or a promoter inducible by other external stimuli.

The insecticidally effective *isp3* gene part is inserted into the plant genome so that the inserted gene part is upstream (i.e., 5') of suitable 3' end transcription regulation signals (i.e., transcript formation and polyadenylation signals). This is preferably accomplished by inserting the *isp3* chimeric gene in the plant cell genome. Preferred polyadenylation and transcript formation signals include those of the CaMV 35S gene, the nopaline synthase gene (Depicker et al., 1982), the octopine synthase gene (Gielen et al., 1984) and the T-DNA gene 7 (Velten and Schell, 1985), which act as 3'-untranslated DNA sequences in transformed plant cells.

Introduction of the T-DNA vector into Agrobacterium can be carried out using known methods, such as electroporation or triparental mating.

The insecticidally effective *isp3* gene part can optionally be inserted in the plant genome as a hybrid gene (US 5,254,799; Vaeck et al., 1987) under the control of the same promoter as a selectable or scorable marker gene, such as the *neo* gene (EP 0 242 236) encoding kanamycin resistance, so that the plant expresses a fusion protein which is easily detectable.

Transformation of plant cells can also be used to produce the proteins of the invention in large amounts in plant cell cultures, e.g., to produce a ISP3 protein that can then be applied onto crops after proper formulation. When reference to a transgenic plant cell is made herein, this refers to a plant cell (or also a plant protoplast) as such in isolation or in tissue culture, or to a plant cell (or protoplast) contained in a plant or in a differentiated organ or tissue, and both possibilities are specifically included herein. Hence, a reference to a plant cell in the description or claims is not meant to refer only to isolated cells in culture, but refers to any plant cell, wherever it may be located or in whatever type of plant tissue or organ it may be present.

All or part of the *isp3* gene, encoding an anti-Lepidopteran protein, can also be used to transform other microorganisms, such as bacteria, such as a *B. thuringiensis* which has insecticidal activity against Lepidoptera or Coleoptera.

Thereby, a transformed Btstrain can be produced which is useful for combating a wide spectrum of Lepidopteran and/or Coleopteran insect pests or for combating additional Lepidopteran insect pests. Transformation of bacteria, such as bacteria of the genus *Pseudomonas*, *Agrobacterium, Bacillus* or *Escherichia*, with all or part of the *isp3* gene of this invention, incorporated in a suitable cloning vehicle, can be carried out In a conventional manner, preferably using conventional electroporation techniques as described in Mahillon et al. (1989) and in PCT Patent publication WO 90/06999.

Transformed *Bacillus* species strains containing the *isp3* gene of this Invention can be fermented by conventional methods (Dulmage, 1981; Bernhard and Utz, 1993) to provide high yields of cells. Under appropriate growth conditions which are well understood, these strains secrete ISP3 proteins in high yields.

Alternative suitable host microorganisms in which the *isp3* genes can be expressed are fungi, algae, or viruses, particularly species which are plant colonising (e.g., (endo)symbiontic) species or insect pathogens.

An insecticidal, particularly anti-Lepidopteran, composition of this invention can be formulated in a conventional manner using the microorganisms transformed with the *isp3* gene, or preferably their respective ISP3 proteins or the ISP3 toxin, or an insecticidally effective toxin portion as an active ingredient, together with suitable carriers, diluents, emulsifiers and/or dispersants (e.g., as described by Bemhard and Utz, 1993). This insecticide composition can be formulated as a wettable powder, pellets, granules or dust or as a liquid formulation with aqueous or non-aqueous solvents as a foam, gel, suspension, concentrate, etc.. Examples of compositions comprising insecticidal Bt spores are described in WO96/10083.

A method for controlling insects, particularly Lepidoptera, in accordance with this invention can comprise applying (e.g., spraying), to a locus (area) to be protected, an insecticidal amount of the ISP3 proteins or compositions comprising the ISP3 proteins or comprising host cells transformed with the *isp3* genes of this invention. The locus to be protected can include, for example, the habitat of the insect pests or growing vegetation (e.g. application to the foliage) or an area where vegetation is to be grown (e.g. application to soil or water). A preferred composition comprises an insecticidal amount of at least one of the ISP3 proteins of the invention, preferentially produced by a bacterial host, and the preferred application of the composition are leaf application, soil application or seed coating.

The term "contacting" is used herein to mean "to bring into physical contact with". Contacting a plant with an insecticidal protein means that the insecticidal protein is brought into contact with cells of the plant, either internally (for example by expression in the plant) or externally (for example by applying compositions comprising the insecticidal protein externally to the plant). It is understood that the term does not indicate the length of time of contact, but comprises any time period of contact (e.g. brief contact, long contact). When referring to a method of protecting a plant against insect damage comprising contacting said plant (or cells or tissues thereof) with an insecticidal protein of the invention, the contact is preferentially long enough and extensive enough (with a high enough amount of protein contacting a large enough number of cells) to prevent or reduce insect damage.

This invention further relates to a method for controlling Lepidopteran cotton insect pests, particularly bollworms, budworms, earworms, preferably a Lepidopteran cotton insect pest selected from the group of *Helicoverpa zea* (Corn Earworm), *Helicoverpa armigera* (Cotton Bollworm), *Helicoverpa punctigera* (Native Bollworm), *Heliothis virescens* (Tobacco Budworm), *Spodoptera frugiperda* (Fall Armyworm) and *Pectinophora gossypiella* (Pink Bollworm), which method comprises applying to an area or plant to be protected, a claimed ISP3 protein as defined herein, preferably a ISP3-327D protein as defined herein, (i.e. by contacting a cotton plant with an ISP3 protein of this invention, for example by planting a cotton plant transformed with an *isp3* gene of this invention, or spraying a composition containing a ISP3 protein of this invention). The invention also relates to the use of the ISP3 proteins of this invention, particularly the ISP3-327D protein, against Lepidopteran cotton insect pests to minimize damage to cotton plants.

This invention further relates to a method for controlling Lepidopteran maize insect pests, particularly earworms, armyworms, corn borers, preferably a maize insect pest selected from the group of *Helicoverpa zea* (Com Earworm), *Agrotis ipsilon* (Black Cutworm), *Ostrinia nubilalis* (European Com Borer) and *Spodoptera frugiperda* (Fall Armyworm), which method comprises applying to an area orplantto be protected, a claimed ISP3 protein as defined herein, preferably a ISP3-327D protein as defined herein, (i.e. by contacting a maize plant with an ISP3 protein of this invention, for example by planting a maize plant transformed with an *isp3* gene of this invention, or spraying a composition containing a ISP3 protein of this invention). The invention also relates to the use of the claimed ISP3 proteins of this invention, particularly the ISP3-327D protein, against Lepidopteran maize insect pests to minimize damage to maize plants.

This invention further relates to a method for controlling Lepidopteran rice insect pests, particularly rice stemborers, rice skippers, rice cutworms, rice armyworms, rice caseworms, rice leaffolders, preferably a rice insect pest selected from the group of Yellow Stem Borer (*Scirphophaga incertulas*), Leaffolder (*Cnaphalocrocis medinalls*), Pink Stem Borer (*Sesamia inferens*) and Corn Spotted Stem Borer (*Chilo partellus*), which method comprises applying to an area or plant to be protected, a claimed ISP3 protein as defined herein, preferably a ISP3-327D protein as defined herein, (i.e. by contacting a rice plant with an ISP3 protein of this invention, for example by planting a rice plant transformed with an *isp3* gene of this invention, or spraying a composition containing a ISP3 protein of this invention). The invention also relates to the use of the claimed ISP3 proteins of this invention, particularly the ISP3-327D protein, against Lepidopteran rice insect pests to minimize damage to rice plants.

This invention further relates to a method for controlling Lepidopteran soybean insect pests, preferably a soybean insect pest selected from the group of Velvet Bean Caterpillar (*Anticarsia gemmatalis*), Soybean Looper (*Pseudoplusia includens*), Beet Armyworm (*Spodoptera exigua*), Yellowstriped Armyworm (*Spodoptera omithogalli*), Com Earworm (*Helicoverpa zea*), Pod Borer (*Epinotia aporema*) and *Rachiplusia nu,* which method comprises applying to an area or plant to be protected, a claimed ISP3 protein as defined herein, preferably an ISP3-327D protein as defined herein, (i.e. by contacting a soybean plant with an ISP3 protein of this invention, for example by planting a soybean plant transformed with an *isp3* gene of this invention, or spraying a composition containing a ISP3 protein of this invention). The invention also relates to the use of the claimed ISP3 proteins of this invention, particularly the ISP3-327D protein, against Lepidopteran soybean insect pests to minimize damage to soybean plants.

To obtain the ISP3 toxin or protein, cells of the recombinant hosts expressing the ISP3 protein can be grown in a conventional manner on a suitable culture medium. The secreted toxin can be separated and purified from the growth medium. Alternatively, if the proteins are not secreted, the cells can be lysed using conventional means such as enzyme degradation or detergents or the like. The toxin can then be separated and purified by standard techniques such as chromatography, extraction, electrophoresis, or the like.

The term "gene" means any DNA or RNA fragment comprising a region (the "transcribed region") which is transcribed into an RNA, molecule (e.g., an mRNA) in a cell, operably linked to suitable regulatory regions, e.g., a plant-expressible promoter. A gene may thus comprise several operably linked fragments such as a promoter, a 5' leader sequence, a coding region, and a 3' nontranslated sequence, comprising a polyadenylation site. A gene endogenous to a particular organism (such as a plant species or a bacterial strain) is a gene, which is naturally found in that organism in nature. A chimeric gene, when referring to an *isp3* DNA of this invention, refers to an *isp3* DNA sequence having 5' and/or 3' regulatory sequences different from the naturally-occurring bacterial 5' and/or 3' regulatory sequences, which drive the expression of the *isp3* gene in its native host cell.

The term "expression of a gene" refers to the process wherein a DNA or RNA region which is operably linked to appropriate regulatory regions, particularly to a promoter, is transcribed into an RNA which is biologically active i.e., which is either capable of interaction with another nucleic acid or which is capable of being translated into a biologically active polypeptide or protein. A gene is said to encode an RNA when the end product of the expression of the gene is biologically active RNA, such as e.g. an antisense RNA, a ribozyme or a replicative intermediate. A gene is said to encode a protein when the end product of the expression of the gene is a biologically active protein or polypeptide.

For the purpose of this invention the "sequence identity" of two related nucleotide or amino acid sequences, expressed as a percentage, refers to the number of positions in the two optimally aligned sequences which have identical residues (x100) divided by the number of positions compared. A gap, i.e. a position in an alignment where a residue is present in one sequence but not in the other is regarded as a position with non-identical residues. To calculate sequence identity between two sequences for the purpose of this invention, the GAP program, which uses the Needleman and Wunsch algorithm (1970) and which is provided by the Wisconsin Package, Version 10.2, Genetics Computer Group (GCG), 575 Science Drive, Madison, Wisconsin 53711, USA, is used. The GAP parameters used are a gap creation penalty = 50 (nucleotides) / 8 (amino acids), a gap extension penalty = 3 (nucleotides) / 2 (amino acids), and a scoring matrix "nwsgapdna" (nucleotides) or "blosum62" (amino acids).

GAP uses the Needleman and Wunsch global alignment algorithm to align two sequences over their entire length, maximizing the number of matches and minimizes the numberof gaps. The default parameters are a gap creation penalty = 50 (nucleotides) /8 (proteins) and gap extension penalty = 3 (nucleotides) / 2 (proteins). For nucleotides the default scoring matrix used is "nwsgapdna" and for proteins the default scoring matrix is "blosum62" (Henikoff & Henikoff, 1992).

These and/or other embodiments of this invention are reflected in the wordings of the claims, which form part of the description of the invention.

The following Examples illustrate the invention, and are not provided to limit the invention or the protection sought. The sequence listing referred to in the Examples, the Claims and the Description is as follows:
SEQ ID No. 1: DNA sequence of *isp3-1099E* reference DNA
SEQ ID No. 2: amino acid sequence of ISP3-1099E reference protein
SEQ ID No. 3: DNA sequence *isp3-327D*
SEQ ID No. 4: amino acid sequence ISP3-327D
SEQ ID No. 5: DNA sequence of *isp3-2245J* reference DNA
SEQ ID No. 6: amino acid sequence of ISP3-2245J reference protein

Unless stated otherwise in the Examples, all recombinant DNAtechniques are carried out accordingto standard protocols as described in Sambrook and Russell (2001) Molecular Cloning: A Laboratory Manual, Third Edition, Cold Spring Harbor Laboratory Press, NY, in Volumes 1 and 2 of Ausubel et al. (1994) Current Protocols in Molecular Biology, Current Protocols, USA and in Volumes I and II of Brown (1998) Molecular Biology LabFax, Second Edition, Academic Press (UK). Standard materials and methods for plant molecular work are described in Plant Molecular Biology Labfax (1993) by R.D.D. Croy, jointly published by BIOS Scientific Publications Ltd (UK) and Blackwell Scientific Publications, UK. Standard materials and methods for polymerase chain reactions can be found in Dieffenbach and Dveksler (1995) PCR Primer: A Laboratory Manual, Cold Spring Harbor Laboratory Press, and in McPherson at al. (2000) PCR - Basics: From Background to Bench, First Edition, Springer Verlag, Germany.

### Examples

### Reference Example 1: Characterization of bacterial strains

. A bacterial strain, named herein BTS01099E, was isolated from grain dust from Belgium. A further bacterial strain, named herein BTS00327D, was isolated from horse dung from Spain. A further bacterial strain, herein named BTS02245J, was isolated from grain dust from the Philippines.

Each strain was grown overnight on LB agar plates (LB medium with 1.5 % agar added; LB medium: 10 g/l trypton, 10 g/l NaCl, 5 g/l yeast extract, pH 7.3) at 28°C. For small scale cultures, 20 ml TB medium (Terrific Broth: 12 g/l tryptone, 24 g/l yeast extract, 3.8 g/l KH₂PO₄, 12.5 g/l K₂HPO₄, 5 ml/l glycerol, pH 7.1) was inoculated and grown for 65 hours at 28°C on a rotating platform having about 70 rotations per minute. After 65 hours, a protease inhibitor mixture was added to the culture. This cocktail has the following ingredients (volumes given are those required to add to one 20 ml culture): 200µl PMSF (100mM), 200µl of a mixture of benzamidine HCl (100mM) and epsilon-amino-n-caproic acid (500 mM), 400µl EGTA (0.5M), 40µl antipain (0.5mg/ml) / leupeptin (0.5mg/ml) and 20µl beta-mercapto ethanol (14M).

The culture medium to which the protease inhibitor mixture had been added, was then centrifuged for 20 minutes at 3000 rpm. In some cases, the supernatant was concentrated about 4 times using Centriprep YM-1 0 Centrifugal Filter Devices (Millipore Cat. No. 4305).

For long term storage, a loop of sporulated cells was added to 0.5ml of 25% glycerol and after vortexing, stored at -70°C. Sporulated cells were obtained by growth of the strain on LB agar plates until sporulation (as visible under the light microscope).

After cultivating on LB agar plates of single cell colonies, microscopical analysis of the strain cultures of BTS01099E, BTS00327D and BTS02245J showed the presence of rod-shaped, motile, single, vegetative cells and sporangia containing an ovale spore. Parasporal crystals were detected in cultures of BTS00327D, BTS01099E and BTS02245J.

Based on the rod-like shape, the aerobic growth, and the presence of parasporal crystals, these 3 strains are believed to be *B. thuringlensis* species strains.

Each strain can be cultivated on conventional standard media, preferably T₃ medium (tryptone 3 g/l, tryptose 2 g/l, yeast extract 1.5 g/l, 5 mg MnCl₂, 0.05 M Na₂HPO₄.2H₂O, 0.05 M NaH₂PO₄.H₂O, pH 6.8 and 1.5% agar), preferably at 28°C. For long term storage, it is preferred to mix an equal volume of a spore-crystal suspension with an equal volume of 50% glycerol and store this at -70°C or lyophilize a spore-crystal suspension. For sporulation, growth on T₃ medium is preferred for 72 hours at 28 °C.

### Reference Example 2: Insect bioassays of Bacillus strains (using culture supernatant containing insecticidal protein)

Bioassays were performed on neonate larvae of *Helicoverpa zea, Heliothis virescens, Ostrinia nubilalis, Spodoptera frugiperda* and *Agrotis ipsilon.*

Cell-free supernatant of bacterial cultures of different Bacillus strains was used in insect bioassays ("surface contamination assay") against various lepidopteran insects.

Strain BTS01099E, BTS00327D or BTS02245J was grown at 28°C in TB medium. Cell-free culture supernatant was harvested 65 hours after culture initiation, dilutions were made and applied onto the surface of solidified artificial diet (agar 20g, water 1000ml, corn flour 96g, yeast 30g, wheat germs 64g, Wesson salt 7.5g, casein 15g, sorbic acid 2g, Aureomycin 0.3g, Nipagin 1 g, wheat germ oil 4ml, sucrose 15g, cholesterol 1 g, ascorbic acid 3.5g,

Vanderzand mod. vit. mix 12g) (based on Vanderzand 1962), dispensed in wells of Costar 48-well plates and allowed to solidify. 25µl supernatant solution was applied onto the surface of each well (1cm²). One neonate (L1; first instar) insect larvae was placed on each well and 18-20 larvae were used per sample. Dishes were kept at 25± 1°C and mortality rates (percentage dead versus living larvae) were recorded after 7 days. As a negative control standard diet and TB was used.

Results (shown in Table 1 below) showed that the cell-free supernatants of strains BTS01099E, BTS00327D and BTS02245J showed toxicity towards *Heliothis virescens, Helicoverpa zea* and *Ostrinia nubilalis* larvae. In addition, the supernatant of strain BTS02245J also showed toxicity towards Spodoptera frugiperda larvae and the supernatant of BTS00327D showed toxicity towards *Agrotis ipsilon* larvae.

**Table 1:**

| Strain | Hv mort (%) | Hz mort (%) | On mort (%) | Sf mort (%) | Ai mort (%) |
|---|---|---|---|---|---|
| BTS02245J | 11 (ir)/33 (gi) | 94 (gi) / 50 | 50 (gi) | 78 (gi) | nt |
| BTS00327D | 70 | 35-78 (gi) | 100 | nt | 100 |
| IBTS01099E | 25 | 10 | 46 | nt | nt |
| Hv: *Heliothis virescens*, Hz: *Helicoverpa zea;* On: *Ostrinia nubilalis,* Sf: *Spodoptera frugiperda;* Ai: *Agrotis ipsilon;* nt: not tested | | | | | |
| Negative controls (standard diet t): | | | | | |
| BTS02245J - Hv 9%, Hz 0%, On 0%. Sf 0% | | | | | |
| BTS00327D - Hv 10%, Hz 6%, On 4%, Ai 0% | | | | | |
| BTS01099E - Hv 10%, Hz 0%, On 0% | | | | | |

### Additional observations:

gi: growth inhibition of larvae (live larvae still in L1/L2 instar stage after 7 days) ir: irregular growth of larvae (a proportion of live larvae in L1/L2 instar stage, a proportion of live larvae in L3/L4 instar stage after 7 days)

Cell-free supernatant of strain BTS02245J caused 11 % and 33% mortality of *H. virescens* larvae, 94% and 50% mortality of *H. zea* larvae, 50% mortality of *O. nubilalis* larvae and 78% mortality of *S. frugiperda* larvae, showing that supernatant of this strain has insecticidal activity, particularly against *H. zea* and *S. frugiperda.* Toxicity is likely caused by a protein secreted by this strain into the culture medium.

The cell-free supernatant of strain BTS00327D caused 70% mortality of *H, virescens* larvae, 35% to 78% mortality of *H. zea* larvae, 100% mortality of O. *nubilalis* larvae and 100% mortality of *Agrotis ipsilon* larvae. Thus, the supernatant of this strain showed strong toxicity to at least four different species of Lepidopteran insects. Toxicity is likely to be caused by an insecticidally active protein secreted by this strain into the culture medium.

The cell-free supernatant of strain BTS01099E caused 25% mortality of *H. virescens* larvae, 10% mortality of *H. zea* larvae and 46% mortality of *O. nubilalis* larvae, indicating that the supernatant of this strain has toxic activity against different species of Lepidopteran insects. Toxicity is likely to be caused by an insecticidally active protein secreted by this strain into the culture medium.

### Example 3: Cloning of isp3 genes

### Cloning of the reference nucleotide sequence encoding ISP3-1099E from strain BTS01099E

Total DNA of strain BTS01099E was prepared and partially digested with *Sau3A.* The digested DNA was size fractioned on a sucrose gradient. Fragments ranging from 7kb to 10kb were ligated to cloning vector pUC191 (a derivative of pUC19; Yannisch-Perron et al. 1985), after *Bam*H1 digestion and treatment of the cloning vector with TsAP (thermosensitive alkaline phosphatase). The ligation mixture was then electroporated into *E. coli* XL1-Blue cells. Transformants were plated on LB-triacillin plates containing X-gal and IPTG and white colonies were selected to be used in filter hybridization experiments. Recombinant *E. coli* clones containing the vector were then screened with a DIG labeled probe which was prepared as follows. First, a PCR was performed using as template cells from strain BTS01099E. The resulting amplification product was gel-purifled and cloned into pGEM-T. The resulting plasmid was used as template in a PCR reaction using DIG- labeled dNTPs and the same primers as in the first PCR reaction. An appropriate amount of this amplification product was used in hybridization reactions.

Following the identification of a positive colony containing a plasmid harboring the full length *isp3* gene, the sequence of this gene was determined using the dye terminator labeling method and a Perkin Elmer ABI Prism-377 DNA sequencer. Both the coding and non-coding strand were sequenced.

The sequence of the open reading frame found in the cloned reference DNA fragment of a positive colony is shown in SEQ ID No. 1 (*isp3-f099E*). This DNA sequence was found to encode the novel reference protein shown in SEQ ID No. 2 (ISP3-1099E).

To show that this DNA sequence is the cause of the Insecticidal activity observed, the sequence was expressed in a bacterial strain and the supernatant or cell lysate of the recombinant strain was tested for insecticidal activity In insect bioassays.

### Cloning of the nucleotide sequence encoding ISP3-327D from strain BTS00327D

Total DNA of strain BTS00327D.was prepared and partially digested with Sau3A. The digested DNA was size fractioned on a sucrose gradient. Fragments ranging from 7kb to 10kb were ligated to cloning vector pUC19I (a derivative of pUC19; Yannisch-Perron et al. 1985), after *Bam*H1 digestion and treatment of the cloning vector with TsAP (thermosensitive alkaline phosphatase). The ligation mixture was then electroporated Into *E. coli* XL1-Blue cells. Transformants were plated on LB-triacillin plates containing X-gal and IPTG and white colonies were selected to be used in filter hybridization experiments. Recombinant *E. coli* clones containing the vector were then screened with a DIG labeled probe which was prepared as follows. First, a PCR was performed using as template cells from strain BTS00327D. The resulting amplification product was gel-purified and cloned into pGEM-T. The resulting plasmid was used as template in a PCR reaction using DIG- labeled dNTPs and the same primers as in the first PCR reaction. An appropriate amount of this amplification product was used in hybridization reactions.

Following the identification of a positive colony containing a plasmid harboring the full length *isp3* gene, the sequence of this gene was determined using the dye terminator labeling method and a Perkin Elmer ABI Prism-377 DNA sequencer. Both the coding and non-coding strand were sequenced.

The sequence of the open reading frame found in the cloned DNA fragment of a positive colony is shown in SEQ ID No. 3 *(isp3-327D).* This DNA sequence was found to encode the novel protein shown in SEQ ID No.4 (ISP3-327D).

To show that this DNA sequence is the cause of the insecticidal activity observed, the sequence was expressed in a bacterial strain and the supernatant or cell lysate of the recombinant strain was tested for insecticidal activity in insect bioassays.

### Cloning of the reference nucleotide sequence encoding ISP3-2245J from strain BTS02245J

Total DNA of strain BTS02245J was prepared and partially digested with *Sau3A.* The digested DNA was size fractioned on a sucrose gradient. Fragments ranging from 7kb to 10kb were ligated to cloning vector pUC19I (a derivative of pUC19; Yannisch-Perron et al. 1985), after *Bam*H1 digestion and treatment of the cloning vector with TsAP (thermosensitive alkaline phosphatase). The ligation mixture was then electroporated into *E. coli* XL1 -Blue cells. Transformants were plated on LB-triacillin plates containing X-gal and IPTG and white colonies were selected to be used in filter hybridization experiments. Recombinant *E. coli* clones containing the vector were then screened with a DIG labeled probe which was prepared as follows. First, a PCR was performed using as template cells from strain BTS02245J. The resulting amplification product was gel-purified and cloned into pGEM-T. The resulting plasmid was used as template in a PCR reaction using DIG- labeled dNTPs and the same primers as in the first PCR reaction. An appropriate amount of this amplification product was fused in hybridization reactions.

Following the identification of a positive colony containing a plasmid harboring the full length *isp3* gene, the sequence of this gene was determined using the dye terminator labeling method and a Perkin Elmer ABI Prism-377 DNA sequencer. Both the coding and non-coding strand were sequenced.

The sequence of the open reading frame found in the cloned reference DNA fragment of a positive colony is shown in SEQ ID No. 5 (*isp3-2245J*). This DNA sequence was found to encode the novel reference protein shown in SEQ ID No. 6 (ISP3-2245J).

To show that this DNA sequence is the cause of the Insecticidal activity observed, the sequence was expressed in a bacterial strain and the supernatant or cell lysate of the recombinant strain was tested for insecticidal activity in insect bioassays.

### Example 4: Recombinant expression of ISP3 proteins in E. coli

Reference DNA of SEQ ID No. 1 (*isp3-9099E*), SEQ ID No. 3 *(isp3-327D)* and reference DNA of SEQ ID No. 5 (*isp3-2245J*) were subcloned Into an expression vector, under control of the cry1Ab promoter, and expressed in *E. coli* strain WK6. During subcloning, an *Nco*I restriction site was introduced at the ATG start codon, thereby changing the second amino acid of SEQ ID No 2, SEa ID No 4 and SEQ ID No. 6 from Asparagine (Asn) into Aspartate (Asp).

SDS-PAGE analysis of transformed *E. coli* cell lysates showed that proteins of the expected molecular weight (±88kDa) were produced for each of the three genes. As negative controls cell lysate of non-transformed *E. coli* WK6 were used.

Cell lysate of recombinant *E. coli* cultures, expressing isp3-327D and isp3-1099E reference protein, was used in insect bioassays (surface contamination assays) as described in Example 5. The results are summarized in Table 2 below. Plus symbols indicate-significant insect mortality over the negative control.

**Table 2:**

| Gene In *E. coli* | Hz | Hv | Sf | Ag | Dvv |
|---|---|---|---|---|---|
| *isp3-327D* | + | + | + | + | - |
| *isp3-1099E* | + | + | + | + | - |
| negative control WK6 | - | - | - | - | - |
| Hz: *Helicoverpa zea, Hz*: *Heliothis virescens,* Sf: *Spodoptera frugiperda,* Dvv: *Diabrotica virgifera virgifera,* Ag: *Anticarsia gemmatalis* | | | | | |
| Bioassays: | | | | | |
| Hz: Surface contamination on heliothis food in 48 multiwell Costar plates, 25µl/well(1cm²), 18x1 L1 per concentration | | | | | |
| Hv: Surface contamination on heliothis food in 24 multiwell Costar plates, 50µl/well(2cm²), 20x1 L1 per concentration | | | | | |
| Sf: surface contamination on littoralis food in 48 multiwell Costar plates; 25µl/well (1cm²); 18x1 L1 per concentration | | | | | |
| Ag: surface contamination on littoralis food in 24 multiwell Costar plates, 50µl/well(2cm²), 12x2L1 per concentration. Incubation at T:25±1 °C; Score after 7 days | | | | | |

For ISP3-327D protein and ISP3-1099F reference protein, significant mortality was found in surface contamination assays with *Hellcoverpa zea, Heliothis virescens, Spodoptera frugiperda* and *Anticarsia gemmatalis.* In addition, undiluted cell lysate of recombinant *E. coli* expressing ISP3-327D or ISP3-1099E reference protein showed significant mortality against *Ostrinia nubilalis* (50%(gi) mortality for ISP3-327D, 26% (gi) mortality for ISP3-1099E compared to 0% mortality for the control WK6).

### Example 5: Recombinant expression of ISP3 proteins in Bt

Reference DNA of SEQ ID No. 1 (isp3-1099E) and SEQ ID No. 5 were subcloned into a shuttle vector and expressed in a crystal minus strain Bt strain (IPS 78/11 or Berliner 1715cry). In bioassays, supernatant from the non-transformed Bt strain is used as negative control.

The cell-free culture supernatant from the recombinant Bt strain is tested for toxicity against Lepidopteran insect larvae, particularly against *H. virescens, H. zea, H. armigera, O. nubilalis, S. frugiperda, Agrotis ipsilon, Pectinophora gossypiella* and *A. gemmatalls,* using a surface contamination assay as described above (Example 2) and below (to determine LC₅₀ values).

Supernatant from the recombinant Bt strain is obtained as follows.

The Bt strain is grown overnight on LB agar plates containing erythromycin (20 µg/ml) at 28°C. For small scale cultures, 20 ml TB medium containing erythromycin (20 µg/ml) is inoculated and grown for 65 hours at 28°C on a rotating platform having about 70 rotations per minute. After 65 hours, a protease inhibitor mixture is added to the culture. This cocktail has the following ingredients (volumes given are those required to add to one 20 ml culture): 200µl PMSF (100mM), 200µl of a mixture of benzamidine.HCl (100mM) / epsilon-amino-n-caproic acid (500 mM), 400µl EGTA (0.5M), 40µl antipain (0.5 mg/ml) / leupeptin (0.5 mg/ml) and 20µl beta-mercaptoethanol (14M).

The culture medium to which the protease inhibitor mixture had been added, is then centrifuged for 20 minutes at 3000 rpm. In some cases, the supernatant is concentrated about 4 times using centriprep YM-10 Centrifugal Filter Devices (Millipore, Cat. No. 4305).

For *Helicoverpa zea* and *Heliothis virescens* the following artificial diet is used in the surface contamination assay: water 1 liter, agar 20g, soyflour 81 g, wheat germ 36g, Wesson salt mix 10g, sucrose 14.7g, Nipagin 1g, sorbic acid 1.1 g, Vanderzant vit.mix. 9.5g, corn oil 5ml, Nystatin 0.06g, Aureomycin 0.17g.

In the surface contamination assays for other lepidopteran insects, the following artificial diet is used: water 1 liter, agar, 20g, corn flour 112g, wheatgerm 28g, yeast 30g, sorbic acid 1.2g, Nipagin 1 g, Aureomycin 0.06g, Nystatin 0.03g, ascorbic acid 4.8g.

The artifical diet is dispensed in wells of Costar 24-multiwell plates and allowed to solidify. 50µl of diluted supernatant is applied onto the surface of each well (2cm²). One or two neonate (L1; first instar) larvae are placed on each well (depending on the species, e.g. for *O. nubilalis* 2 larvae/well) and around 20 to 24 larvae are used per supernatant dilution. Six to eight supernatant dilutions (the dilution factor is around 3), ranging from about 4050 to 0.21 ng/cm² are tested. Dishes are kept at 25±1 °C and mortality rates (percentage dead versus living larvae) are recorded after 7 days. As a negative control standard diet and TB is used. LC₅₀ values and/or LC₉₀ values are calculated with probit analyis using the program POLO PC (from LeOra Software, 1987, Berkely, Califomia). The LC₅₀ value is the total supernatant protein concentration when 50% of the tested insect larvae are killed.

The bioassays show that the proteins encoded by the cloned reference sequences *isp3-1099E* and *isp3-2245J* each cause significant Insecticidal activity against selected Lepidopteran insects.

SEQ ID No. 3 (*isp3-327D*) was subcloned into a shuttle vector and expressed in the crystal minus Bt strain iPS78/11. In bioassays, supernatant from the non-transformed Bt strain was used as negative control.

SDS-page analysis showed that the culture supernatant contained a protein with a molecular weight close to the calculated molecular weight of the ISP3-327D protein (±88kDa).

Using the surface contamination assay as described in Example 2, the cell-free culture supernatant of Bt strain IPS78/11 expressing SEQ ID No. 3 (*isp3-327D*) showed significant insecticidal activity against *Ostrinia nubilalis* (On), *Pectinophora gossypiella* (Pg) and *Helicoverpa zea (Hz),* as shown in Table 3.

**Table 3:**

| | Hz mort (%) | On mort (%) | Pg mort (%) | Dvvmort(%) |
|---|---|---|---|---|
| *isp3-327D* in IPS 78/11 | 94 (gi) / 58 (gi) | 19 (gi) | 29 (gi) | 0 |
| Control IPS 78/11 | 0 | 6 | 0 | 5 |
| gi: growth inhibition of larvae / Dvv: *Diabrotica virgifera virgifera* | | | | |

### Bioassays:

Surface contamination assays, as described above, using concentrated supernatant after 26hrs culture in TB and following addition of protease inhibitor coctail. Heliothis artificial diet (as above) was used for *H. zea* and littoralis artificial diet for *O. nubilalis* and *P. gossypiella.* For *H. zea* and *O. nubilalis* 48 well Costar plates, 25µl/well (1cm²), 18 wells with one L1 larva per well, were used. For *P. gossypiella* 24 well Costar plates, 50µl/well (2cm²) and 12 wells with two L1 larvae per well were used. Dw artificial diet (as above) was used for Dw in 24 well plates, 50µl/well (2cm²), 6 wells with 4 L1/well.

The bioassay showed that the protein encoded by the cloned sequence *isp3-327D* has significant insecticidal activity against selected Lepidopteran insects.

### Example 6: further characterisation of ISP3-327D

Supernatant from the crystal minus Bt strain IPS78/11 expressing SEQ ID No. 3 (*isp3-327D*) was used to test trypsin digestability of ISP3-327D protein and toxicity of the resulting fragments. Trypsin treatment of supernatant of the transformed IPS78/11 culture resulted in two major bands of about 65kDa and about 23kDa, as determined by SDS-PAGE analysis.

Both trypsin treated supernatant (4 hours treatment; reaction was stopped with PMSF of a final concentration of 0.1 mM) and non-trypsin treated supernatant were used in surface contamination assays against *Helicoverpa zea.* The surface contamination assay was performed on heliothis food in 48 multiwell plates (25µl/well; 1cm²). Six supernatant dilutions were tested. Per dilution 18 wells and one L1 larva per well were used. Mortality was scored after incubation at 25°±I. °C after 7 days.

LC50 values for the untreated and trypsin treated ISP3-327D protein showed overlapping 90% confidence intervals, showing that the trypsin treated protein retains toxic activity against *H. zea.*

### Example 7: Rice insect bioassays of recombinant ISP3 proteins

The sequences for reference DNA *isp3-1099E* (SEQ ID No.1), *isp3-327D* (SEQ ID NO.3) and reference DNA *isp3-2245J* (SEQ ID No. 5) were expressed in *E. coli* as described above.

Cell lysates of recombinant *E. coli* were tested in insect bioassays for activity against four Lepidopteran rice pests. Five to six doses per protein were tested.

### (a) Yellow Stem Borer (Scirphophaga incertulas) bioassay:

Yellow Stem Borer adults were collected from rice fields. Eggs laid by the adults were collected and kept in Petri dishes for hatching at 30°C. The newly hatched larvae were used in the bioassays.

Rice stalk sheath of 6 cm were used. Six centimeter long segments of the leaf sheaths were cut from freshly excised rice stalks of the susceptible variety TN1. The inner most part of the segment, along with one sheath cover, were separately dipped into the different protein doses for 30 seconds. The treated stalk sheath was immobilized vertically on 2 cm agar gel in specimen tubes (7cm long; 2.5 cm diameter). 10-15 neonate larvae of Yellow Stem Borer were added to each treated stalk and tubes were sealed and incubated for five days. After 5 days, the numbers of surviving and of dead larvae were counted.

### (b) Leaffolder (Cnaphalocrocis medinalis) bioassay:

Insects were collected from rice fields in northern India. Insect larvae were reared on rice plants in the green house. Emerging adults were confined in oviposition chamber and oviposited plants were kept in water trays for larval hatching. One day old larvae were used in bioassays.

Bioassays were conducted in cylindrical chambers, using freshly excised leaves from rice plants at tillering stage. An 8 cm long leaf lamina was excised from the central whorl of the rice tiller. The leaf lamina was placed in the chamber and the different protein dosages applied. After 30 minutes, five to ten larvae were added per leaf. At least th ree leaves were used per protein dose. Five days after incubation, the number of dead and surviving larvae was counted.

### (c) Pink Stem Borer (Sesamia inferens) bioassay:

Insects were collected from rice fields in northern India and reared on rice plants.

Bioassays were conducted in glass vials (7.5cm x 2.5cm diameter) using an artificial diet made of dry bean powder, brewers yeast, sorbic acid, ascorbic acid, methyl paraben, agar and water. The diet was dispensed into the vials up to 2cm depth. At least three vials were prepared per protein dose. 40µl of test dose was spread uniformly onto the surface of the diet in each vial and left to dry for one hour. Ten to fifteen first instar larvae of Pink Stem Borer were added per vial. After seven days the number of dead and surviving larvae was counted.

### (d) Corn Spotted Stem Boter (Chilo partellus) bioassays:

Insects were maintained on artificial diet made up of red bean powder, brewers yeast, sorbic acid, sorghum leaf powder, ascorbic acid, methyl para-hydroxy benzoic acid, vitamins, wheat germ oil, Wesson salt mixture, agar, formaldehyde and water. Neonate larvae from these cultures were used in the bioassays. The bioassays were performed as described for Pink Stem Borer *(Sesamia inferens).*

### (e) Results of rice insect bioassays

The results of the insect bioassays, summarized in table 4 below, showed that ISP3-327D and reference protein ISP3-1099E were highly toxic to four Leptidopteran rice pests, namely Yellow Stem Borer *(Scirphophaga incertulas),* Leaffolder (*Cnaphalocrocis medinalis*), Pink Stem Borer (*Sesamia inferens*) and Corn

Spotted Stem Borer (*Chilo partellus*)*.* Further, ISP3-2245J reference protein showed significanttoxicity towards three lepidopteran rice pests, namely Yellow Stem Borer (*Scirphophaga incertulas*), Leaffolder (*Cnaphalocrocis medinalis*) and Pink Stem Borer (*Sesamia inferens*), while no toxicity of ISP3-2245J reference protein against Corn Spotted Stem Borer (*Chilo partellus*) was detected.

**Table 4:**

| Gene in *E. coli* | YSB | LF | PSB | SSB |
|---|---|---|---|---|
| *isp3-327D* | + | + | + | + |
| *isp3-1099E* | + | + | + | + |
| *Isp3-2245J* | + | + | + | - |
| negative control | - | - | - | - |
| YSB: Yellow Stem Borer, LF: Leaffolder, PSB: Pink Stem Borer, SSB: Spotted Stem Borer; plus symbols (+) indicate significant mortality over the negative control. | | | | |

### Example 8: Production of ISP3 proteins in transformed plants

Plant expression vectors are constructed comprising a plant-expressible promoter, operably linked to a DNA sequence encoding either reference protein ISP3-1099E, ISP3-327D or reference protein ISP3-2245J (oratoxicfragment or variant thereof), and a 3' polyadenylation signal. A leader sequence, such as that from the chlorophyl a/b binding protein gene from Petunia (Harpster et al. 1988), is inserted 5' of the *isp3* DNA. Preferably, codon-usage of reference DNA *isp3-1099E, isp3-327D* and reference DNA *isp3-2245J* is adapted to that of the host plant (e.g. com, cotton or rice), for example as described in WO94/24264.

The promoters used to drive the *isp3 genes* are selected from the constitutive promoters CaMV 35S (Hull and Howell, 1987), maize ubiquitin promoter, rice actin promoter and Cassava Vein Mosaic Virus promoter. As 3' transcript termination and polyadenylation signal the 3'35S, 3'nos (Depicker et al. 1982), 3'ocs or 3'gene7 are used. For *Agrobacterium* mediated transformation, the expression cassette is inserted into a T-DNA vector, between the right and left border sequence.

Transformation of corn (*Zea mays*), cotton (*Gossypium hirsutum* or *Gossypium barbadense*) and rice (*Oryza sativa*) plants.

Corn cells are stably transformed by *Agrobacterium* mediated transformation as described in US 6,140,553. Cotton cells are stably transformed by *Agrobacterium* mediated transformation as described in WO 00/71733. Rice cells are stably transformed as described in WO92/09696.

Transformed cells and plantlets are regenerated as described in the above references. For constructs additionally comprising a selectable marker gene, such as a herbicide resistance gene, for example 2mEPSPS (EPO 508 909 and EP 0 507 698) conferring resistance to glyphosate or the bar or PAT gene conferring resistance to glufosinate ammonium (EP 0 242 236 and EP 0 242 246) transformed cells are grown on selection media containing the selective agent, so that most of the selected regenerants are transformed.

From the regenerants transformants expressing the *isp3* gene are selected by ELISA, Southern blot, Northern blot and/or PCR analysis. Transformation events, particularly single copy events, are then selected for insecticidal activity towards Lepidopteran insect pests (using bioassays). Chimeric *isp3-1099E reference gene isp3-327D* or *isp3-2245J* reference gene-containing progeny plants show improved resistance to lepidopteran insects compared to non-transformed control plants. Particularly plants with high levels of insect tolerance express a high level of ISP3 protein and *isp3* mRNA.

Transformants are further selected for agronomic characteristics (normal phenotype, yield, plant architecture, etc.). Following seed increases, field trials of transformed com, cotton or rice plants are carried out in different regions where susceptible insect pests are present, demonstrating that plants expressing ISP3 proteins have an increased insect tolerance under field conditions in different environments. Particularly, following infestation (artificial or natural) of the field by insect pests, yield losses of the transformed plants are reduced compared to non-transformed control plants.

To generate plants with broad insecticidal activity, the ISP3 expressing events are crossed to each other and to other transgenic plants, expressing insecticidal proteins with a different, preferably complementary, insecticidal spectrum. Progeny of the crosses are assayed for insecticidal activity using bioassays for a range of different insect pests. Plants with insecticidal activity against the desired insect range are generated in this way.

This invention is not limited to the above corn, cotton, soybean or rice plants, transformation methods, vector constructs (promoters, 3'ends, etc.) or the particular ISP3 proteins or DNA sequences used. The invention includes variants or equivalents of the ISP3 proteins retaining insecticidal activity.

### REFERENCES

An et al. (1996) Plant J. 10, 107
Aoyama and Chua (1997) Plant Journal 11:605-612
Ausubel et al. (1994) Current Protocols in Molecular Biology, Current Protocols, Vol. 1 and 2, USA.
Bennetzen & Hall (1982) J. Biol. Chem. 257, 3026-3031.
Bemhard, K. and Utz, R. (1993) "Production of Bacillus thuringiensis insecticides for experimental and commercial uses", In Bacillus thuringiensis, An Environmental Biopesticide: Theory and Practice, pp.255-267, eds. Entwistle, P.F., Cory, J.S., Bailey, M.J. and Higgs, S., John Wiley and Sons, New York.
Bih et al. (1999), J. Biol. Chem. 274, 22884-22894.
Brown (1998) Molecular Biology LabFax, Volumes I and II, Second Edition,Academic Press (UK)
Callis et. al. (1987) Genes Developm. 1:1183-1200
Christensen et al. (1992) Plant Mol. Biol. 18, 675-689.
Cordera et al. (1994) The Plant Journal 6, 141
Comejo et al. (1993) Plant Mol. Biol. 23, 567-581.
Cornelissen et al. (1986) EMBO J. 5, 37-40.
Datta et al. (1990) Bio/Technology 8, 736-740
De Pater et al. (1992) Plant J. 2, 834-844.
Depicker et al. (1982) J. Molec. Appl. Genetics 1, 561-573.
Dieffenbach and Dveksler (1995) PCR Primer: A Laboratory Manual, Cold Spring Harbor Laboratory Press
Doss et al (2002) Protein Expression and Purification 26, 82-88
Dulmage, H.T. (1981) "Production of Bacteria for Biological Control of Insects" in Biological Control in Crop Production, Ed. Paparizas, D.C., Osmun Publishers, Totowa, N.J., USA, pp. 129-141.
Estruch et al. (1996) Proc Natl Acad Sci USA 93, 5389-94.
Franck et al. (1980) Cell 21, 285-294
French et al. (1986) Anal.Biochem. 156, 417-423
French-Constant and Bowen (2000) Cell Mol Life Sci 57, 828-33.
Fromm et al. (1990) Bio/Technology 8, 833-839
Gardner et al. (1981) Nucleic Acids Research 9, 2871-2887
Ge et al. (1991) J. Biol. Chem. 266, 17954-17958
Gielen et al. (1984) EMBO J 3, 835-845
Gill et al. (1992) Ann. Rev. Entomol. 37: 615-636
Gordon-Kamm et al. (1990) The Plant Cell 2, 603-618
Gould et al. (1991) Plant Physiol. 95, 426-434
Haider et al. (1986) Europ J Biochem 156:531-540
Harlow and Lane (1988) Antibodies: A Manual Laboratory, Cold Spring Harbor Lab Press NY
Harpster et al.(1988), Molecular and General Genetics 212, 182-190
Henikoff and Henikoff (1992) Proc. Natl. Academy Science 89 (10):915-919
Hesse et al. (1989), EMBO J. 8 2453-2461.
Ho et al.(1989). Gene 77, 51-59.
Hofmann et al. (1988) PNAS 85:7844-7848
Höfte et al. (1988) Appl. and Environm. Microbiol. 54, 2010-2017
Hull and Howell (1987) Virology 86, 482-493
Ikemura, 1993, In "Plant Molecular Biology Labfax", Croy, ed., Bios Scientific Publishers Ltd. Itakura et al.(1977). Science 198, 1056-1063.
Keil et al. (1986), Nucl. Acids Res. 14, 5641-5650.
Klösgen et al. (1989), Mol. Gen. Genet. 217, 155-161.
Klösgen and Weil (1991), Mol. Gen. Genet. 225, 297-304.
Kota et al. (1999) Proc. Natl. Acad. Sci. USA 96, 1840-1845.
Last et al. (1990) Theor. Appl. Genet. 81, 581-588.
Mahillon et al. (1989), FEMS Microbiol. Letters 60, 205-210.
Maxam and Gilbert (1980) Methods in Enzymol. 65, 499-560.
McBride et al.(1995) Bio/Technology 13, 362
McPherson et al. (2000) PCR - Basics: From Background to Bench, First Edition, Springer Verlag, Germany
Morris et al. (1999), Biochem. Biophys. Res. Commun. 255, 328-333.
Murray et al. (1989) Nucleic Acids Research 17(2), 477-498.
Nakamura et al. (2000) Nucl. Acids Res. 28, 292.
Needleman and Wunsch algorithm (1970) J. Mol. Biol. 48: 443-453
Neuhaus & Rogers (1998) Plant Mol. Biol. 38, 127-144.
Nielsen, H. J. Engelbrecht, S. Brunak, and G. von Heijne (1996) A neural network method for identification of prokaryotic and eukaryotic signal peptides and prediction of their cleavage sites
Odell et al. (1985) Nature 313, 810-812.
Oelmuller et al.(1993) Mol. Gen. Genet. 237, 261-272.
Park et al. (1997) J. Biol. Chem. 272, 6876-6881.
R.D.D. Croy (1993) Plant Molecular Biology Labfax jointly published by BIOS Scientific Publications Ltd (UK) and Blackwell Scientific Publications, UK.
Sambrook et al. (1989) Molecular Cloning: A Laboratory Manual, Second Edition, Cold Spring Harbor Laboratory Press, NY
Sambrook and Russell (2001) Molecular Cloning: A Laboratory Manual, Third Edition, Cold Spring Harbor Laboratory Press, NY
Sanger et al.(1977) Proc. Natl. Acad. Sci. U S A. 74(12), 5463-5467.
Schnepf and Whiteley (1981) Proc. Natl. Acad. Sci. USA 87: 2893-2897
Selvapandiyan et al. (2001) Appl. and Environm. Microbial 67 (12), 5855-5858
Shcherban et al. (1995), Proc. Natl. Acad. Sci USA 92, 9245-9249.
Shimamoto et al (1989) Nature 338, 274-276
Smith and Waterman (1981)-Advances in Applied Mathematics 2: 482-489
Stanssens et al.(1989) Nucleic Acids Research 12, 4441-4454.
Sutliff et al. (1991) Plant Molec. Biol. 16, 579-591.
Tavladoraki et al. (1998), FEBS Lett. 426, 62-66.
Terashima et al. (1999), Appl. Microbiol. Biotechnol. 52, 516-523.
Vaeck et al. (1987) Nature 328, 33-37.
Van Den Broeck et al. (1985) Nature 313, 358.
Van Rie et al. (1990) Science 247, 72.
Vanderzand (1962) J. Econ. Entomol. 55, 140
Velten et al. (1984) EMBO J 3, 2723-2730
Velten and Schell (1985), Nucleic Acids Research 13, 6981-6998
Verdaguer et al. (1998), Plant Mol. Biol. 37, 1055-1067
Visser et al. (1993) "Domain-Structure Studies of Bacillus thuringiensis Crystal Proteins: A Genetic Approach", in Bacillus thuringiensis, An Environmental Biopesticide: Theory and Practice, pp.71-88, eds. Entwistle, P.F., Cory, J.S., Bailey, M.J. and Higgs, S., John Wiley and Sons, New York.
Von Heijne, Gunnar (1986) Secretory signal peptide identification algorithm described by Gunnarvon Heijne Nucleic Acids Research. 14:11, 4683-4690
Wada et al. (1990). Nucl. Acids Res. 18, 2367-1411.
Warren (1997) "Advances in Insect Control: The role of transgenic plants", 1997, editors Carozzi and Koziel, p109-121, Taylor and Francis London, UK
Waterfield et al.(2001) Trends Microbiol 9, 185-91.
White et al.(1989). Trends in Genet. 5, 185-189
Wilbur and Lipmann (1983) Proc. Nat. Acad. Sci. USA 80: 726
Wong et al.(1992), Plant Molec. Biol.20, 81-93.
Yannisch-Perron et al. (1985) Gene 33, 103-119
Yu et al. (1997) Applied and Environmental Microbioloby 532-536
Zhang et al. (1991) The Plant Cell 3, 1155-1165.

### SEQUENCE LISTING

<110> Bayer BioScience N.V.
<120> Novel Bacillus thuringiensis insecticidal proteins
<130> isp3
<150> US 60/366276
   <151 > 2002-03-22
<150> US 60/423999
   <151> 2002-11-06
<160> 6
<170> Patent In version 3.0
<210> 1
   <211> 2364
   <212> DNA
   <213> artificial
<220>
   <223> nucleotide sequence of isp3-1099E of Bacillus thuringlensis
<400> 1
   atgaacatga ataatactaa attaaacgca agggccttac caagttttat tgattatttt      60
   aatggcattt atggatttgc cactggtatc aaagacatta tgaatatgat ttttaaaacg     120
   gatacaggtg gtaatctaac cctagacgaa atcttaaaga atcagcagtt actaaatgag     180
   atttctggta aattggatgg ggtaaatggg agcttaaatg atcttatcgc acagggaaac    240
   ttaaatacag aattatctaa ggaaatctta aaaattgcaa atgaacagaa tcaagtctta    300
   aatgatgtta ataacaaact cgatgcgata aatacgatgc ttcatatata tctacctaaa    360
   attacatcta tgttaagtga tgtaatgaag caaaattatg cgctaagcct gcaaatagaa    420
   tacttaagta aacaattgca agaaatttcc gataagttag atattattaa cgtaaatgtt    480
   cttattaact ctacacttac tgaaattaca cctgcatatc aacggattaa atatgtgaat    540
   gaaaaatttg aagaattaac ttttgctaca gaaaccactc taaaagtaaa agaggatgga    600
   tcccctgcag atattcttga tgagttaact gagttaactg aattagcgaa aagtgtaaca    660
   aaaaatgaag tggatggttt tgaattttac cttaatacat tccacgatgt aatggtagga    720
   aataatttat tcgggcgttc agctttaaaa actgcttcgg aattaattgc taaagaaaat    780
   gtgaaaacaa gtggcagtga ggtaggaaat gtttataatt tcttaattgt attaacagct    840
   ctacaagcaa aagcttttct tactttaaca acatgccgga aattattagg attagcagat    900
   attgattata cttctattat gaatgaacat ttaaataagg aaaaagagga atttagagta    960
   aacatccttc ctacactttc taatactttt tctaatccta attatgtaaa agctaaagga   1020
   agcgatagag atgcaaagat gattgtggaa gctaaaccag gatatgcttt ggttggattt   1080
   gaaatgagta atgattcaat gacagtatta aaagcatatc aggccaagct aaagcaagat   1140
   tatcaagttg ataaggattc attatcagaa attgtctatg gtgatatgaa taagttatta   1200
   tgtccggatc aatctgaaca aatatattat acaaataata tagcatttcc ccgtgaatat   1260
   gttattacta aacttacttt tacaaaaaaa atgaacagtt taaggtatga ggcgacagct   1320
   aatttttatg attcttctac aggagatatg gatctaaata agacaaaagt agaatcaagt   1380
   gaagcggagt atagtaggct aagtgctagt aatgatggag tctatatgcc gttaggtctt   1440
   atcagtgaaa catttttgac tccaattaat ggttttggac tcgtagtcga tgaaaattca   1500
   agattagtaa ctttaacatg taaatcatat ttaagagaga tattattagc aacagactta   1560
   agtaataaag aaactaaatt gattgtccca cctaatggtt ttattagcaa tattgtagaa   1620
   aatgggaact tagagggaga aaacttagag ccgtggaaag caaataacaa aaatgcgtat   1680
   atagatcata caggcggcgt aaaaggaact aaagtattat atgttcataa ggatggagag   1740
   ttctcacaat ttattgggta taaattgaaa tcgaaaacag aatatgtaat tcaatatatt   1800
   gtaaaaggaa aagctgttat ttatttaaaa gatgaaaaaa atggggatta catttatgaa   1860
   gaaataaata atgaattaga agattttcaa acggttacta aacgttttat tacaggaaca   1920
   gattcttcag gagttcattt aatttttacc agtcaaaatg gcgaggaagc attcgggggg   1980
   aactttatta tttcagaaat taggccatcc gaagagttat taagtccaga attgattaag   2040
   tcggatgctt gggttggaac tcagggagct tggaattcag ggaattctct cacaatttat   2100
   actaatacaa atggaacctt tcgacaaaac cttccgttag aaagttattc aacttatagt   2160
   atgaacttta atataactgg atttggcaag gtgacaataa ggaattctcg tgaagtatta   2220
   tttgaaaaga acttttccca gctttcgcct aaagattatt ctgaaaaatt tacaactgca   2280
   gccaataata ccggattcta tgtagagctt tctcgcggaa cgcagggtgg taatataact   2340
   ttccgagatt tttcaattaa gtaa                                          2364
<210> 2
   <211> 787
   <212> PRT
   <213> artificial
<220>
   <223> amino acid sequence of ISP3-1099E of Bacillus thuringiensis
<400> 2
   Met Asn Met Asn Asn Thr Lys Leu Asn Ala Arg Ala Leu Pro Ser Phe
   1               5                   10                  15
   Ile Asp Tyr Phe Asn Gly Ile Tyr Gly Phe Ala Thr Gly Ile Lys Asp
               20                  25                  30
   Ile Met Asn Met Ile Phe Lys Thr Asp Thr Gly Gly Asn Leu Thr Leu
           35                  40                  45
   Asp Glu Ile Leu Lys Asn Gln Gln Leu Leu Asn Glu Ile Ser Gly Lys
       50                  55                  60
   Leu Asp Gly Val Asn Gly Ser Leu Asn Asp Leu Ile Ala Gln Gly Asn
   65                  70                  75                  80
   Leu Asn Thr Glu Leu Ser Lys Glu Ile Leu Lys Ile Ala Asn Glu Gln
                   85                  90                  95
   Asn Gln Val Leu Asn Asp Val Asn Asn Lys Leu Asp Ala Ile Asn Thr
               100                 105                 110
   Met Leu His Ile Tyr Leu Pro Lys Ile Thr Ser Met Leu Ser Asp Val
           115                 120                 125
   Met Lys Gln Asn Tyr Ala Leu Ser Leu Gln Ile Glu Tyr Leu Ser Lys
   130 135 140
   Gln Leu Gln Glu Ile Ser Asp Lys Leu Asp Ile Ile Asn Val Asn Val
   145 150 155 160
   Leu Ile Asn Ser Thr Leu Thr Glu Ile Thr Pro Ala Tyr Gln Arg Ile
   165 170 175
   Lys Tyr Val Asn Glu Lys Phe Glu Glu Leu Thr Phe Ala Thr Glu Thr
   180 185 190
   Thr Leu Lys Val Lys Glu Asp Gly Ser Pro Ala Asp Ile Leu Asp Glu
   195 200 205
   Leu Thr Glu Leu Thr Glu Leu Ala Lys Ser Val Thr Lys Asn Glu Val
   210 215 220
   Asp Gly Phe Glu Phe Tyr Leu Asn Thr Phe His Asp Val Met Val Gly
   225 230 235 240
   Asn Asn Leu Phe Gly Arg Ser Ala Leu Lys Thr Ala Ser Glu Leu Ile
   245 250 255
   Ala Lys Glu Asn Val Lys Thr Ser Gly Ser Glu Val Gly Asn Val Tyr
   260 265 270
   Asn Phe Leu Ile Val Leu Thr Ala Leu Gln Ala Lys Ala Phe Leu Thr
   275 280 285
   Leu Thr Thr Cys Arg Lys Leu Leu Gly Leu Ala Asp Ile Asp Tyr Thr
   290 295 300
   Ser Ile Met Asn Glu His Leu Asn Lys Glu Lys Glu Glu Phe Arg Val
   305 310 315 320
   Asn Ile Leu Pro Thr Leu Ser Asn Thr Phe Ser Asn Pro Asn Tyr Val
   325 330 335
   Lys Ala Lys Gly Ser Asp Arg Asp Ala Lys Met Ile Val Glu Ala Lys
   340 345 350
   Pro Gly Tyr Ala Leu Val Gly Phe Glu Met Ser Asn Asp Ser Met Thr
   355 360 365
   Val Leu Lys Ala Tyr Gln Ala Lys Leu Lys Gln Asp Tyr Gln Val Asp
   370 375 380
   Lys Asp Ser Leu Ser Glu Ile Val Tyr Gly Asp Met Asn Lys Leu Leu
   385 390 395 400
   Cys Pro Asp Gln Ser Glu Gln Ile Tyr Tyr Thr Asn Asn Ile Ala Phe
   405 410 415
   Pro Arg Glu Tyr Val Ile Thr Lys Leu Thr Phe Thr Lys Lys Met Asn
   420 425 430
   Ser Leu Arg Tyr Glu Ala Thr Ala Asn Phe Tyr Asp Ser Ser Thr Gly
           435                 440                 445
   Asp Met Asp Leu Asn Lys Thr Lys Val Glu Ser Ser Glu Ala Glu Tyr
       450                 455                 460
   Ser Arg Leu Ser Ala Ser Asn Asp Gly Val Tyr Met Pro Leu Gly Leu
   465                 470                 475                 480
   Ile Ser Glu Thr Phe Leu Thr Pro Ile Asn Gly Phe Gly Leu Val Val
                   485                 490                 495
   Asp Glu Asn Ser Arg Leu Val Thr Leu Thr Cys Lys Ser Tyr Leu Arg
               500                 505                 510
   Glu Ile Leu Leu Ala Thr Asp Leu Ser Asn Lys Glu Thr Lys Leu Ile
           515                 520                 525
   Val Pro Pro Asn Gly Phe Ile Ser Asn Ile Val Glu Asn Gly Asn Leu
       530                 535                 540
   Glu Gly Glu Asn Leu Glu Pro Trp Lys Ala Asn Asn Lys Asn Ala Tyr
   545                 550                 555                 560
   Ile Asp His Thr Gly Gly Val Lys Gly Thr Lys Val Leu Tyr Val His
                   565                 570                 575
   Lys Asp Gly Glu Phe Ser Gln Phe Ile Gly Tyr Lys Leu Lys Ser Lys
               580                 585                 590
   Thr Glu Tyr Val Ile Gln Tyr Ile Val Lys Gly Lys Ala Val Ile Tyr
           595                 600                 605
   Leu Lys Asp Glu Lys Asn Gly Asp Tyr Ile Tyr Glu Glu Ile Asn Asn
       610                 615                 620
   Glu Leu Glu Asp Phe Gln Thr Val Thr Lys Arg Phe Ile Thr Gly Thr
   625                 630                 635                 640
   Asp Ser Ser Gly Val His Leu Ile Phe Thr Ser Gln Asn Gly Glu Glu
                   645                 650                 655
   Ala Phe Gly Gly Asn Phe Ile Ile Ser Glu Ile Arg Pro Ser Glu Glu
               660                 665                 670
   Leu Leu Ser Pro Glu Leu Ile Lys Ser Asp Ala Trp Val Gly Thr Gln
           675                 680                 685
   Gly Ala Trp Asn Ser Gly Asn Ser Leu Thr Ile Tyr Thr Asn Thr Asn
       690                 695                 700
   Gly Thr Phe Arg Gln Asn Leu Pro Leu Glu Ser Tyr Ser Thr Tyr Ser
   705                 710                 715                 720
   Met Asn Phe Asn Ile Thr Gly Phe Gly Lys Val Thr Ile Arg Asn Ser
                   725                 730                 735
   Arg Glu Val Leu Phe Glu Lys Asn Phe Ser Gln Leu Ser Pro Lys Asp
               740                 745                 750
   Tyr Ser Glu Lys Phe Thr Thr Ala Ala Asn Asn Thr Gly Phe Tyr Val
           755                 760                 765
   Glu Leu Ser Arg Gly Thr Gln Gly Gly Asn Ile Thr Phe Arg Asp Phe
       770                 775                 780
   Ser Ile Lys
   785
<210> 3
   <211> 2367
   <212> DNA
   <213> artificial
<220>
   <223> nucleotide sequence of isp3-327D of Bacillus thuringiensis
<400> 3
   atgaatatga ataatactaa attaaacgca agggccctac cgagttttat tgattatttt      60
   aatggcattt atggatttgc cactggtatc aaagacatta tgaatatgat tttaaaaacg     120
   gatacaggtg gtaatctaac cttagacgaa atcctaaaga atcagcagtt actaaatgag     180
   atttctggta aattggatgg ggtaaatggg agcttaaatg atcttatcgc acagggaaac     240
   ttaaatacag aattatctaa ggaaatctta aaaattgcaa atgaacagaa tcaagtctta     300
   aatgatgtta ataacaaact cgatgcgata aatacgatgc ttcatatata tctacctaaa     360
   attacatcta tgttaagtga tgtaatgaag caaaattatg cgctaagtct gcaaatagaa     420
   tacttaagta agcaattgca agaaatttct gataaattag atattattaa cgtaaatgtt     480
   cttattaact ctacacttac tgaaattaca cctgcatatc aacggattaa atatgtgaat     540
   gaaaaatttg aagaattaac ttttgctaca gaaaccactt taaaagtaaa aaaggatagc     600
   tcgcctgctg atattcttga tgagttaact gaattaactg aactagcgaa aagtgttaca     660
   aaaaatgacg ttgatggttt tgaattttac cttaatacat tccacgatgt aatggtagga     720
   aataatttat tcgggcgttc agctttaaaa actgcttcag aattaattgc taaagaaaat     780
   gtgaaaacaa gtggcagtga agtaggaaat gtttataatt tcttaattgt attaacagct     840
   ctacaagcaa aagcttttct tactttaaca acatgccgaa aattattagg cttagcagat     900
   attgattata cttctattat gaatgaacat ttaaataagg aaaaagagga atttagagta 960
   aacatccttc ctacactttc taatactttt tctaatccta attatgcaaa agttaaagga 1020
   agtgatgaag atgcaaagat gattgtggaa gctaaaccag gacatgcatt ggttgggttt 1080
   gaaatgagca atgattcaat cacagtatta aaagtatatg aggctaagct aaaacaaaat 1140
   tatcaagttg ataaggattc cttatcggag gttatttatg gtgatacgga taaattattt 1200
   tgtccagatc aatctgaaca aatatattat acaaataaca tagtattccc aaatgaatat 1260
   gtaattacta aaattgattt cactaaaaaa atgaaaactt taagatatga ggtaacagcg 1320
   aatttttatg attcttctac aggagaaatt gacttaaata agaaaaaagt agaatcaagt 1380
   gaagcggagt atagaacgtt aagtgctaat gatgatggag tgtatatgcc attaggtgtc 1440
   atcagtgaaa catttttgac tccgataaat gggtttggcc tccaagctga tgaaaattca 1500
   agattaatta ctttaacatg taaatcatat ttaagagaac tactgctagc aacagactta 1560
   agcaataaag aaactaaatt gatcgtccca ccaagtggtt ttattagcaa tattgtagag 1620
   aacgggtcca tagaagagga caatttagag ccgtggaaag caaataataa gaatgcgtat 1680
   gtagatcata caggcggagt gaatggaact aaagctttat atgttcataa ggacggagga 1740
   ttttcacaat ttattggaga taagttaaaa ccgaaaactg agtatgtaat ccaatatact 1800
   gttaaaggaa aaccttctat tcatttaaaa gatgaaaata ctggatatat tcattatgaa 1860
   gatacaaata ataatttaaa agattatcaa actattacta aacgttttac tacaggaact 1920
   gatttaaagg gagtgtattt aattttaaaa agtcaaaatg gagatgaagc ttggggagat 1980
   aaatttacaa ttttagaaat taagcctgcg gaggatttat taagcccaga attaattaat 2040
   ccgaattctt ggattacgac tccaggggct agcatttcag gaaataaact tttcattaac 2100
   ttggggacaa atgggacctt tagacaaagt ctttcattaa acagttattc aacttatagt 2160
   ataagcttta ctgcatcagg accatttaat gtgacggtaa gaaattctag ggaagtatta 2220
   tttgaacgaa gcaaccttat gtcttcaact agtcatattt ctgggacatt caaaactgaa 2280
   tccaataata ccggattata tgtagaactt tcccgtcgct ctggtggtgg tggtcatata 2340
   tcatttgaaa acgtttctat taaataa 2367
<210> 4
   <211> 788
   <212> PRT
   <213> artificial
<220>
   <223> amino acid sequence of ISP3-327D of Bacillus thuringlensls
<400> 4
   Met Asn Met Asn Asn Thr Lys Leu Asn Ala Arg Ala Leu Pro Ser Phe
   1 5 10 15
   Ile Asp Tyr Phe Asn Gly Ile Tyr Gly Phe Ala Thr Gly Ile Lys Asp
   20 25 30
   Ile Met Asn Met Ile Phe Lys Thr Asp Thr Gly Gly Asn Leu Thr Leu
   35 40 45
   Asp Glu Ile Leu Lys Asn Gln Gln Leu Leu Asn Glu Ile Ser Gly Lys
   50 55 60
   Leu Asp Gly Val Asn Gly Ser Leu Asn Asp Leu Ile Ala Gln Gly Asn
   65 70 75 80
   Leu Asn Thr Glu Leu Ser Lys Glu Ile Leu Lys Ile Ala Asn Glu Gln
   85 90 95
   Asn Gln Val Leu Asn Asp Val Asn Asn Lys Leu Asp Ala Ile Asn Thr
   100 105 110
   Met Leu His Ile Tyr Leu Pro Lys Ile Thr Ser Met Leu Ser Asp Val
   115 120 125
   Met Lys Gln Asn Tyr Ala Leu Ser Leu Gln Ile Glu Tyr Leu Ser Lys
   130 135 140
   Gln Leu Gln Glu Ile Ser Asp Lys Leu Asp Ile Ile Asn Val Asn Val
   145 150 155 160
   Leu Ile Asn Ser Thr Leu Thr Glu Ile Thr Pro Ala Tyr Gln Arg Ile
   165 170 175
   Lys Tyr Val Asn Glu Lys Phe Glu Glu Leu Thr Phe Ala Thr Glu Thr
   180 185 190
   Thr Leu Lys Val Lys Lys Asp Ser Ser Pro Ala Asp Ile Leu Asp Glu
   195 200 205
   Leu Thr Glu Leu Thr Glu Leu Ala Lys Ser Val Thr Lys Asn Asp Val
   210 215 220
   Asp Gly Phe Glu Phe Tyr Leu Asn Thr Phe His Asp Val Met Val Gly
   225 230 235 240
   Asn Asn Leu Phe Gly Arg Ser Ala Leu Lys Thr Ala Ser Glu Leu Ile
   245 250 255
   Ala Lys Glu Asn Val Lys Thr Ser Gly Ser Glu Val Gly Asn Val Tyr
   260 265 270
   Asn Phe Leu Ile Val Leu Thr Ala Leu Gln Ala Lys Ala Phe Leu Thr
   275 280 285
   Leu Thr Thr Cys Arg Lys Leu Leu Gly Leu Ala Asp Ile Asp Tyr Thr
   290 295 300
   Ser Ile Met Asn Glu His Leu Asn Lys Glu Lys Glu Glu Phe Arg Val
   305 310 315 320
   Asn Ile Leu Pro Thr Leu Ser Asn Thr Phe Ser Asn Pro Asn Tyr Ala
   325 330 335
   Lys Val Lys Gly Ser Asp Glu Asp Ala Lys Met Ile Val Glu Ala Lys
   340 345 350
   Pro Gly His Ala Leu Val Gly Phe Glu Met Ser Asn Asp Ser Ile Thr
   355 360 365
   Val Leu Lys Val Tyr Glu Ala Lys Leu Lys Gln Asn Tyr Gln Val Asp
   370 375 380
   Lys Asp Ser Leu Ser Glu Val Ile Tyr Gly Asp Thr Asp Lys Leu Phe
   385 390 395 400
   Cys Pro Asp Gln Ser Glu Gln Ile Tyr Tyr Thr Asn Asn Ile Val Phe
   405 410 415
   Pro Asn Glu Tyr Val Ile Thr Lys Ile Asp Phe Thr Lys Lys Met Lys
   420 425 430
   Thr Leu Arg Tyr Glu Val Thr Ala Asn Phe Tyr Asp Ser Ser Thr Gly
   435 440 445
   Glu Ile Asp Leu Asn Lys Lys Lys Val Glu Ser Ser Glu Ala Glu Tyr
   450 455 460
   Arg Thr Leu Ser Ala Asn Asp Asp Gly Val Tyr Met Pro Leu Gly Val
   465 470 475 480
   Ile Ser Glu Thr Phe Leu Thr Pro Ile Asn Gly Phe Gly Leu Gln Ala
   485 490 495
   Asp Glu Asn Ser Arg Leu Ile Thr Leu Thr Cys Lys Ser Tyr Leu Arg
   500 505 510
   Glu Leu Leu Leu Ala Thr Asp Leu Ser Asn Lys Glu Thr Lys Leu Ile
   515 520 525
   Val Pro Pro Ser Gly Phe Ile Ser Asn Ile Val Glu Asn Gly Ser Ile
   530 535 540
   Glu Glu Asp Asn Leu Glu Pro Trp Lys Ala Asn Asn Lys Asn Ala Tyr
   545 550 555 560
   Val Asp His Thr Gly Gly Val Asn Gly Thr Lys Ala Leu Tyr Val His
   565 570 575
   Lys Asp Gly Gly Phe Ser Gln Phe Ile Gly Asp Lys Leu Lys Pro Lys
   580 585 590
   Thr Glu Tyr Val Ile Gln Tyr Thr Val Lys Gly Lys Pro Ser Ile His
   595 600 605
   Leu Lys Asp Glu Asn Thr Gly Tyr Ile His Tyr Glu Asp Thr Asn Asn
   610 615 620
   Asn Leu Lys Asp Tyr Gln Thr Ile Thr Lys Arg Phe Thr Thr Gly Thr
   625 630 635 640
   Asp Leu Lys Gly Val Tyr Leu Ile Leu Lys Ser Gln Asn Gly Asp Glu
   645 650 655
   Ala Trp Gly Asp Lys Phe Thr Ile Leu Glu Ile Lys Pro Ala Glu Asp
   660 665 670
   Leu Leu Ser Pro Glu Leu Ile Asn Pro Asn Ser Trp Ile Thr Thr Pro
   675 680 685
   Gly Ala Ser Ile Ser Gly Asn Lys Leu Phe Ile Asn Leu Gly Thr Asn
   690 695 700
   Gly Thr Phe Arg Gln Ser Leu Ser Leu Asn Ser Tyr Ser Thr Tyr Ser
   705 710 715 720
   Ile Ser Phe Thr Ala Ser Gly Pro Phe Asn Val Thr Val Arg Asn Ser
   725 730 735
   Arg Glu Val Leu Phe Glu Arg Ser Asn Leu Met Ser Ser Thr Ser His
   740 745 750
   Ile Ser Gly Thr Phe Lys Thr Glu Ser Asn Asn Thr Gly Leu Tyr Val
   755 760 765
   Glu Leu Ser Arg Arg Ser Gly Gly Gly Gly His Ile Ser Phe Glu Asn
   770 775 780
   Val Ser Ile Lys
   785
<210> 5
   <211> 2361
   <212> DNA
   <213> artificial
<220>
   <223> nucleotide sequence isp3-2245J of Bacillus thuringiensls
<400> 5
   atgaacatga ataatgctaa attaaatgca agggccttac caagttttat tgattatttt     60
   aatggtattt atggatttgc cactggtatt aaagacatta tgaatatgat ttttaaaacg     120
   gatacaggtt ctaatctaac cctagacgaa attttaaaga atcagcagtt actaaatgaa    180
   atttctggta aattggatgg ggtaaatggg agcttaaatg atcttatcgc acagggaaac    240
   ttaaatacag aattagctaa gcaaatctta aaagttgcaa atgaacaaaa tcaagtttta    300
   aatgatgtta ataacaaact agatgcgata aattcgatgc ttaaaatata tctacctaaa    360
   attacatcta tgttaagtga tgtaatgaag caaaattatg tgctaagctt gcaaatagaa    420
   tacttaagta aacaattgca agaaatctcc gacaagctag atattattaa cgtaaatgtg    480
   cttattaact ctacgcttac tgaaattaca cctgcgtatc aacgaatgaa atatgtgaat    540
   gaaaaatttg aagaattaac ttttgctaca gaaaccactt taaaagtaaa aaaggatagc    600
   cctcctgctg atattcttga cgaattaact gaattaactg aactagcgaa aagtgttaca    660
   aaaaatgacg tggatggttt tgaattttac cttaatacat tccacgatgt aatggtagga    720
   aataatttat tcggtcgttc agctttaaaa actgcttcgg aattaattgc taaagaaaat    780
   gtgaaaacaa gtggcagtga agtaggaaac gtttataatt tcttaattgt attaacagct    840
   ctacaagcaa aagcttttct tactttaaca acatgccgaa aattattagg cttagcagat    900
   attgattata cttctatcat gaatgagcat ttaaataagg aaaaagaaga atttagagta    960
   aacatccttc ccacactttc taataccttt tctaatccta attatgcaaa agctaaggga   1020
   agtaatgaag atacaaagat gattgtggaa gctaaaccag gatatgtttt ggttggattt   1080
   gaaatgagca atgattcaat tacagtatta aaagcatatc aagctaagct aaaaaaagat   1140
   tatcaaattg ataaggattc gttatcagaa ataatatata gtgatacgga taaattatta   1200
   tgtccggatc aatctgaaca aatatattat acaaagaaca tagcatttcc aaatgaatat   1260
   gttattacta aaattgcttt tacaaaaaaa atgaacagtt taaggtatga ggcgacagcg   1320
   aatttttatg attcttctac aggggatatt gatctaaata agacaaaagt agaatcaagt   1380
   gaagcggagt atagtatgct aaaagctagt gatgatgaag tttacatgcc gctaggtctt   1440
   atcagtgaaa catttttgaa tccaattaat ggatttaggc ttgcagtcga tgaaaattcc   1500
   agactagtaa ctttaacatg tagatcatat ttaagagaga cattgttagc gacagattta   1560
   aataataaag aaactaaatt gattgtccca cctaatgttt ttattagcaa tattgtagag 1620
   aatggaaata tagaaatgga caccttagaa ccatggaagg caaataatga gaatgcgaat 1680
   gtagattatt caggcggagt gaatggaact agagccttat atgttcataa ggatggtgaa 1740
   ttctcacatt ttattggaga caagttgaaa tctaaaacag aatacttgat tcgatatatt 1800
   gtaaaaggaa aagcttctat ttttttaaaa gatgaaaaaa atgaaaatta catttatgag 1860
   gatacaaata ataatttaga agattatcaa actattacta aacgttttac tacaggaact 1920
   gattcgacag gagtttattt aatttttaat agtcaaaatg gagatgaagc ttggggagat 1980
   aactttatta ttttggaaat tagtccgtgt gaaaagttat taagtccaga attaattaaa 2040
   acagataaat ggaatagtac gggatcaact tatattagcg atgatagact cactctttat 2100
   cggggaggac gaggaatttt aaagcaaaac cttcaattag acggtttttc aacttataga 2160
   gtcaattttt ctgtggacgg agatgctaat gtaaggattc gtaattctag ggaagtgtta 2220
   cttgaaaaaa gatatttgaa ccgtaaaggt gtttctgaaa tgtttactac aaaatttgat 2280
   aaagataact tttatgtaga gctttctcaa ggggataatc ttggtactat tgtacatttt 2340
   tatgatttct ctattaaata a 2361
<210> 6
   <211> 786
   <212> PRT
   <213> artificial
<220>
   <223> amino acid sequence of ISP3-2245J of Bacillus thuringiensis
<400> 6

### For the following Contracting State(s) : RO

### Field of the invention

The present invention relates to the field of plant pest control, particularly insect control. Provided are new nucleic acid sequences from *Bacillus thuringiensis* (Bt) strains, encoding insecticidal proteins expressed during vegetative growth stages. Particularly, DNA sequences encoding a protein designated as ISP3-327D are provided, which are useful to protect plants from insect damage. Further provided are plants and microorganisms comprising at least one of the new nucleic acid molecules, as well as methods and means for using these nucleic acid sequences for reducing insect damage of plants.

### Background art

Insect pests cause huge economic losses worldwide in crop production, and farmers face every year the threat of yield losses due to insect infestation. Genetic engineering of insect resistance in agricultural crops has been an attractive approach to reduce costs associated with crop-management and chemical control practices. The first generation of insect resistant crops have been introduced into the market since 1996, based on the expression in plants of proteins isolated from the gram-positive soil bacterium *Bacillus thuringiensis* (Bt). The insecticidal Bt Cry proteins are produced during the sporulation-stage of Bt strains and the proteins accumulate in large cytoplasmic crystals within the bacterium. When taken up by insects, a typical Lepidopteran-toxic Bt Cry protein is solubilized and processed in the insect midgut into an active form of about 60 to 65kDa. The active protein exerts its toxic effect by binding to the midgut epithelial cells, causing pore formation in the cell membrane, which leads to osmotic lysis of the cells (Gill et al., 1992).

A Bt strain may produce many different toxins. Since the isolation of the first insecticidal crystal protein encoding gene from Bt in 1981 (Schnepf and Whiteley, 1981) more than 100 Bt Cry toxin encoding genes have been cloned and insect pests have been effectively controlled by expressing Bt derived proteins in agricultural important crop species. However, the use of individual Bt proteins is often limited, as most Bt proteins are mostly only active against a relatively small number of the numerous existing insect pests. Specificity of Bt Cry proteins is thought to be determined by factors such as the activation of the toxin in the insect gut (Haider et al. 1986) and its ability to bind specific receptors (Hofmann et al., 1988).

It is widely recognized that there is a risk that susceptible insect species may develop resistance against Bt Cry toxins. Consequently, active efforts have been made to identify novel insecticidal proteins. One strategy, which has been used, was to screen *Bacillus* strains for the production of insecticidal proteins during vegetative growth stages, rather than during sporulation stages. Using this approach a number of "vegetative insecticidal proteins" or "VIPs" have been identified.

Estruch et al. (1996), WO94/21795, WO96/10083, WO98/44137, US 5,877,012, US 6,107,279, US 6,137,033 and US 6,291,156 describe the isolation of *vip3A(a), vip3A(b)* and *vip3A(c)* from supernatant fluids of Bt strains AB88, AB424 and AB51. According to the authors these genes encode proteins with insecticidal activity towards a broad range of *Lepidopteran* insect pests.

WO98/18932 and WO99/57282 describe a number of nucleotide sequences isolated from Bt strains. These sequences are referred to as *mis* (*mis-1* to *mis-*8*), war* and *sup.* According to the authors the encoded proteins have activity against *Lepidopteran* or *Coleopteran* pests.

WO00/09697 describes heat-labile, soluble MIS-type and WAR-type toxins, as well as smaller (1 to 10kDa) toxins, obtainable from the supernatant of cultures of *Bacillus laterosporus* strains, which, according to the authors, have activity against Western Corn Rootworm larvae.

WO98/00546 and US 6,274,721 describe the isolation of Bt strains and Bt toxins, which, according to the authors, have activity against Lepidopteran pests.

WO99/33991 describes the isolation of Bt strains and Bt toxins, which, according to the authors, have activity against Lepidopteran pests.

Recently, Selvapandiyan et al. (2001) described the isolation of a gene encoding a protein designated as VIP-S. According to the authors the VIP-S protein showed toxicity against a number of Lepidopteran insect species.

Doss et al. (2002) describe the cloning of VIP3V from strain *Bt kurstaki.*

WO02/078437 describes VIP3 toxins from Bt, such as VIP3A, VIP3B and VIP3A-B hybrid toxins.

Despite the isolation and characterization of a relatively large number of different insecticidal proteins to date, there remains a need for identification, isolation and characterization of new insecticidal proteins. The reasons for this are manifold. Firstly, due to the specificity of insecticidal proteins towards particular groups of target pests (host insect spectra), there is a need to clone genes encoding proteins with different spectra of activity, so that for different crops and different geographic regions suitable proteins for combating insect pests are available. The specificity of Bt Cry proteins, for example, is mostly limited. Identification of toxins with specificity towards different target insects remains desirable. Secondly, after prolonged use in one geographic region, insects are known to have the capacity to develop resistance towards chemical insecticides, microbial sprays (for example based on Bt spore-crystal mixtures), and are believed to have the capacity to develop resistance towards plants expressing insecticidal proteins. The development of resistance within insect populations could potentially render existing insecticidal proteins ineffective, providing a need for novel genes and proteins. Thirdly, for health and environmental reasons it is desirable to identify proteins with high, specific insecticidal potency and acute bioactivity towards target insect species.

Hereinafter, including the different embodiments described in the claims, novel nucleic acid sequences and amino acid sequences isolated from *Bacillus thuringiensis* strains are described, which are useful to protect plants from insect damage, either by the expression of the nucleic acid sequences within plants under the control of suitable promoters, or by external application of the toxins to the plants. The toxins of the subject invention are distinct from previously described pesticidal toxins.

### Summary of the invention

The invention provides insecticidal ISP3 proteins and nucleic acids encoding them. Provided is the insecticidal protein ISP3-327D (SEQ ID No. 4) and the nucleic acids encoding it, *isp3-327D* (SEQ ID No. 3). The protein of the invention has insecticidal activity against Lepidopteran insect pests, particularly against insects selected from the group consisting of *Helicoverpa zea, Helicoverpa armigera, Helicoverpa punctigera, Heliothis virescens, Ostrinia nubilalis, Spodoptera frugiperda, Agrotis ipsilon, Pectinophora gossypiella, Scirphophaga incertulas, Cnaphalocrocis medinalis, Sesamia inferens, Chilo partellus, Anticarsia gemmatalis, Plathypena scabra, Pseudoplusia includens, Spodoptera exigua, Spodoptera ornithogalli, Epinotia aporema* and *Rachiplusia nu.*

In another embodiment of the invention insecticidal variants and fragments of the ISP3 protein, and of the nucleic acids encoding it, are provided.

In one embodiment, the claimed ISP3 protein of the invention is an isolated protein insecticidal to Ostrinia nubilalis, comprising the amino acid sequence of a variant of the protein of SEQ ID No. 4, wherein said variant has 5 to 10 amino acids added, replaced or deleted as compared to the protein of SEQ ID No. 4 without significantly changing the insecticidal activity of the protein; or said protein comprising the amino acid sequence of a variant of SEQ ID No. 4 wherein said variant has less than 5 amino acids added, replaced or deleted as compared to the protein of SEQ ID No. 4 without significantly changing the insecticidal activity of the protein.

In another embodiment, the claimed ISP3 protein is a protein that starts with a Met-Asp or Met-Ala dipeptide, by insertion of a codon encoding an Asp or Ala amino acid downstream of the start codon in the DNA encoding such protein, or is a protein comprising the amino acid sequence of SEQ ID No. 4.

A claimed ISP3 protein, as provided herein, is also an isolated protein insecticidal to Ostrinia nubilalis, comprising the amino acid sequence of the smallest fragment of the protein of SEQ ID No. 4 retaining insecticidal activity, which is obtained by enzymatic digestion of the protein of SEQ ID No. 4 with gut-juice fluid from Ostrinia nubilalis.

Further provided herein is a claimed ISP3 protein which is insecticidal against *Pectinophora gossypiella, Scirphophaga incertulas, Cnaphalocrocis medinalis, Sesamia inferens, Chilo partellus* and *Anticarsia gemmatalis.*

In a further embodiment of the invention, isolated nucleic acid sequences encoding the claimed ISP3 protein are provided, such as a nucleic acid sequence comprising nucleotides 1 to 2367 of SEQ ID No. 3.

In yet a further embodiment nucleic acid sequences encoding the claimed ISP3 proteins of the invention are provided, whereby the nucleic acid sequence is a synthetic sequence which has been optimized for expression in monocotyledonous or dicotyledonous plants or plant cells.

It is another objective of the invention to provide chimeric genes, comprising a promoter sequence operably linked to a nucleic acid sequence encoding an ISP3-327D protein, or insecticidally active variants or fragments thereof as claimed herein. Also provided are vectors comprising nucleotide sequences encoding ISP3 proteins, particularly ISP3-327D, or insecticidally active variants or fragments thereof as claimed herein.

The invention further provides host cells comprising chimeric genes, particularly microorganisms or transgenic plant cells, plant tissues, plant origans, plant seeds or whole plants comprising nucleotide sequences encoding the ISP3-327D protein, or insecticidally active fragments or variants thereof as claimed herein. In one embodiment the transformed plant is a maize or cotton plant. In another embodiment the transformed plant is a rice or soybean plant. In a further embodiment the transformed plant is any plant, particularly any plant selected from the group of sorghum, wheat, barley, rye, sunflower, sugarcane, tobacco, Brassica species (such as oilseed rape, mustard, cabbage, broccoli, etc.), vegetable species (tomato, cauliflower, radish, spinach, pepper, onion, bean, pea, carrot, etc.), sugar beet, tree species (apple, pear, plum, conifers, deciduous trees etc.), potato, alfalfa, mango, papaya, banana.

In another embodiment methods of protecting a plant against Insect damage are provided, comprising contacting said plant with a claimed insecticidal ISP3 protein. The plant may be contacted with such ISP3 protein by transforming the plant with a nucleotide sequence encoding that ISP3 protein or by applying that ISP3 protein externally to the plant.

In one embodiment of the invention a Bt strain comprising the claimed *isp3* nucleic acid, particularly *isp3-327D,* is provided.

In a further embodiment, an insecticidal composition comprising an insecticidally effective amount of the claimed ISP3 protein is provided. When applied externally to a plant, the insecticidal composition increases resistance to insect damage compared to control plants, to which no such composition is applied.

Also provided herein is the use of the claimed ISP3 protein against a Lepidopteran cotton, maize, rice or soybean insect pest, particularly when said insect pest is *Helicoverpa zea, Heliothis virescens, Ostrinia nubilalis, Spodoptera frugiperda, Pectinophora gossypiella, Scirphophaga incertulas, Cnaphalocrocis medinalis, Sesamia inferens, Chilo partellus* and *Anticarsia gemmatalis.*

Further provided herein is a plant comprising the claimed chimeric gene, which simultaneously expresses with the protein of SEQ ID No. 4 or its fragment or variant: a Cry protein, a Cry1F protein, a hybrid derived from a Cry1F protein, a Cry1A-type protein or a toxic fragment thereof, a Cry1Ac protein or a hybrid derived therefrom, a Cry1Ab protein or an insecticidal fragment thereof, a Cry2Ae protein, a VIP3Aa protein or a toxic fragment thereof, or an insecticidal protein from *Xenorhabdus, Serratia* or *Photorhabdus* species strains; particularly such plant which is maize, rice, cotton or soybean, or a which is a hybrid plant.

Also provided herein is the use of the claimed ISP3 protein, co-expressed in maize, rice, cotton, or soybean plants, in combination with another insect control protein, wherein said other insect control protein is: a Cry1 Ac protein, a Cry1Ab protein, a Cry2Ae protein, or a VIP3Aa protein or derivatives thereof.

Even further provided herein is the plant cell or the plant comprising the claimed chimeric gene, also comprising a PAT gene conferring resistance to glufosinate ammonium, or a 2mEPSPS gene conferring resistance to glyphosate.

### Detailed description of the embodiments

The field of the invention is to provide methods and means for reducing damage caused to plants by pests, particularly insect pests, more particularly lepidopteran insect pests. Novel nucleic acid sequences and proteins have been identified and isolated, which are distinct from previously described nucleic acid sequences and proteins and which can be used for controlling insect pests by either integration and expression of at least one of these new nucleotide sequences in plants or plant cells or by external treatment of plants or plant parts with compositions comprising the toxins encoded by these nucleic acid molecules.

It is an embodiment of the invention to provide novel pesticidal toxins isolated from the *Bacillus thuringiensis* strains. Particularly, a pesticidal proteins designated as ISP3-327D protein is provided.

In accordance with this invention, a "nucleic acid sequence" refers to a DNA or RNA molecule in single or double stranded form, preferably a DNA or RNA, particularly a DNA, encoding any of the ISP3 proteins of this invention. An "isolated nucleic acid sequence", as used herein, refers to a nucleic acid sequence which is no longer in the natural environment where it was isolated from, e.g., the nucleic acid sequence in another bacterial host or in a plant nuclear genome.

In accordance with this invention, the terms "protein" or "polypeptide" are used interchangeably to refer to a molecule consisting of a chain of amino acids, without reference to any specific mode of action, size, three-dimensional structures or origin. Hence, a fragment or portion of an ISP3 protein of the invention is still referred to herein as a "protein". An "isolated protein", as used herein, refers to a protein which is no longer in its natural environment. The natural environment of the protein refers to the environment in which the protein could be found when the nucleotide sequence encoding it was expressed and translated in its natural environment, i.e. in the environment from which the nucleotide sequence was isolated. For example, an isolated protein can be present *in vitro,* or in another bacterial host or in a plant cell or it can be secreted from another bacterial host or from a plant cell.

In accordance with this invention, nucleic acid sequences, particularly DNA sequences, encoding new ISP3 proteins have been isolated and characterized. The new genes was designated *isp3-327D* and its encoded protein ISP3-327D.

In accordance with this invention "ISP3-327D protein" refers to any protein insecticidal to Ostrinia nubilalis comprising the amino acid sequence of the smallest fragment of the protein of SEQ ID No. 4 which retains insecticidal activity, which is obtained by enzymatic digestion of the protein of SEQ ID No. 4 with gut-juice fluid from Ostrinia nubilalis (hereinafter referred to as "smallest toxic fragment"). This includes hybrid- or chimeric proteins comprising the smallest toxic fragment. Also disclosed herein are claimed variants of the amino acid sequence in SEQ ID No. 4, such as essentially similar amino acid sequences, having a sequence identity of at least 91%, particularly at least 92%, 93%, 94%, 95%, 96%, 97%, 98% or 99% at the amino acid sequence level, as determined using pairwise alignments using the GAP program of the Wisconsin package of GCG (Madison, Wisconsin, USA, version 10.2). The GAP program is used with the following parameters for the amino acid sequence comparisons: the 'blosum62' scoring matrix, a 'gap creation penalty' (or 'gap weight') of 8 and a 'gap extension penalty' (or 'length weight') of 2. In one embodiment of the invention, an ISP3 protein variant insecticidal to Ostrinia nubilalis is provided that has 5-10, particularly less than 5, amino acids added, replaced or deleted without significantly changing, preferably without changing the insecticidal activity of the protein, or at least without changing the insecticidal activity of the protein in a negative way.

As used herein "comprising" is to be interpreted as specifying the presence of the stated features, Integers, steps or components as referred to, but does not preclude the presence or addition of one or more features, integers, steps or components, or groups thereof. Thus, the term "DNA/protein comprising the sequence or region X", as used herein, refers to a DNA or protein including or containing at least the sequence or region X, so that other nucleotide or amino acid sequences can be included at the 5' (or N-terminal) and/or3' (or C-terminal) end, e.g. (the nucleotide sequence of) a selectable marker protein as disclosed in EP 0 193 259, (the nucleotide sequence of) a transit peptide, and/or a 5' or 3' leader sequence.

The "smallest toxic fragment" of an ISP3 protein of the invention, as used herein, is that smallest fragment or portion of an ISP3 protein retaining insecticidal activity that can be obtained by enzymatic digestion of the full length ISP3 protein (the same fragment or portion of an ISP3 protein retaining insecticidal activity can also be obtained by making nucleotide deletions in the DNA encoding an ISP3 protein). It is understood, that DNA encoding shorter toxic ISP3 fragments claimed herein may also be synthesized chemically and that the smallest toxic fragment obtainable from transcription and translation of synthetic DNA is included in the definition of smallest toxic fragment.

In one embodiment of the invention, the smallest toxic fragment of an ISP3 protein has a molecular weight of about 65kDa as determined by SDS-PAGE (sodium dodecyl sulfate-polyacrylamide gel electrophoresis) analysis. In another embodiment, the smallest toxic fragment of an ISP3 protein has a molecular weight of about 23kDa. In another embodiment, the smallest toxic fragment of an ISP3 protein has a molecularweight of about 33kDa. In a further embodiment, the smallest toxic fragment comprises the central amino acids of the ISP3 protein, in particular from amino acid 200 to amino acid 455 of SEQ ID No. 4.

Enzymatic digestions of ISP3 proteins can be performed by either using purified enzymes or using gut-juice fluids from insect larvae and incubating gut-juice extracts with solutions comprising one of the ISP3 proteins, as described in Yu et al. (1997). Proteolytic products can be separated and visualized on SDS-PAGE. Bioassays can be carried out with processed, chromatographically fractioned protein fragments in order to determine the relationship between each proteolytic fragment and its insecticidal activity. Preferred gut-juice used to determine the smallest toxic fragment of ISP3 proteins is gut-juice from Lepidopteran insects, preferably from Corn Earworm (*Helicoverpa zea*), Cotton Bollworm (*Helicoverpa armigera*), Native Budworm (*Helicoverpa punctigera*), Tobacco Budworm (*Heliothis virescens*), European Corn Borer (*Ostrinia nubilalis*), Fall Armyworm (*Spodoptera frugiperda*), Black Cutworm (*Agrotis ipsilon*), Pink Bollworm (*Pectinophora gossypiella*), Yellow Stem Borer (*Scirphophaga incertulas*), Leaffolder (*Cnaphalocrocis medinalis*), Pink Stem Borer (*Sesamia inferens*), Corn Spotted Stem Borer (*Chilo partellus*), Velvet Caterpillar (*Anticarsia gemmatalis*), Soybean Looper (*Pseudoplusia includens*), Pod Borer (*Epinotia aporema*), *Rachiplusia nu*.

The N- and C-terminal amino acid sequence ends of the smallest toxic fragment are conveniently determined by amino acid sequence determination of the above fragments by techniques routinely available in the art.

As used herein, the term "*isp3-327D*" refers to any DNA sequence encoding the "ISP3-327D protein", as defined above. This includes naturally occurring, artificial or synthetic DNA sequences encoding the proteins of SEQ ID No. 4, or its insecticidal fragments or variants as claimed herein. Also included herein are DNA sequences, encoding the claimed insecticidal proteins, which are similar enough to the DNA sequences provided in the sequence listing so that they can (i.e., have the ability to) hybridize to these DNA sequences under stringent hybridization conditions.

"Stringent hybridization conditions", as used herein, refers particularly to the following conditions: immobilizing the relevant DNA on a filter, and prehybridizing the filters for either 1 to 2 hours in 50 % formamide, 5x SSPE, 2x Denhardt's reagent and 0.1 % SDS at 42 ° C or 1 to 2 hours in 6x SSC, 2xDenhardt's reagent and 0.1 % SDS at 68 °C. The denatured (Digoxigenin- or radio-) labeled probe is then added directly to the prehybridization fluid and incubation is carried out for 16 to 24 hours at the appropriate temperature mentioned above. After incubation, the filters are then washed for 30 minutes at room temperature in 2x SSC, 0.1 % SDS, followed by 2 washes of 30 minutes each at 68 °C in 0.5 x SSC and 0.1 % SDS. An autoradiograph is established by exposing the filters for 24 to 48 hours to X-ray film (Kodak XAR-2 or equivalent) at -70 °C with an intensifying screen. [20x SSC = 3M NaCl and 0.3M sodiumcitrate; 100x Denhart's reagent= 2%(w/v) bovine serum albumin, 2%(w/v) Ficoli™ and 2% (w/v) polyvinylpyrrolidone; SDS = sodium dodecyl sulfate; 20x SSPE= 3.6M NaCl, 02M Sodium phosphate and 0.02M EDTA pH7.7]. Of course, equivalent conditions and parameters can be used in this process while still retaining the desired stringent hybridization conditions.

It is clear that there are many approaches known in the art for the isolation of variants of the DNA sequences of the invention. Variants can, for example, be isolated from Bt strains by hybridization as described supra, and/or by PCR technology as known in the art. Specific or degenerate primers can be made to regions of the *isp3* DNA sequences, and used to amplify variants from known or novel Bt strains.

Preferred variants of the *isp3-327D* DNA of this invention are DNA sequences encoding the claimed ISP3-327D protein variants described above, or a DNA sequence, encoding such claimed ISP3 protein, with at least 94%, preferably at least 95%, particularly at least 96%, 97%, 98% or at least 99% sequence identity to SEQ ID No. 3. The sequence identities referred to above are calculated using the GAP program of the Wisconsin package of GCG (Madison, Wisconsin, USA) Version 10.2. The GAP program is used with the following parameters for nucleic acids: the "nwsgapdna" scoring matrix, a 'gap creation penalty' (or 'gap weight') of 50 and a 'gap extension penalty' (or length weight') of 3. Stringent hybridization conditions are as defined above.

"Insecticidal activity" of a protein, as used herein, means the capacity of a protein to kill insects when such protein is fed to insects, preferably by expression in a recombinant host such as a plant. It is understood that a protein has insecticidal activity if it has the capacity to kill the insect during at least one of its developmental stages, preferably the larval stage.

"Insect-controlling amounts" of a protein, as used herein, refers to an amount of protein which is sufficient to limit damage on a plant, caused by insects (e.g. insect larvae) feeding on such plant, to commercially acceptable levels, e.g. by killing the insects or by inhibiting the insect development, fertility or growth in such a manner that they provide less damage to a plant and plant yield is not significantly adversely affected.

In accordance with this invention, insects susceptible to the new ISP3 proteins of the invention are contacted with this protein in insect-controlling amounts, preferably insecticidal amounts. Preferred target insects for the proteins of this invention are economically damaging insect pests of corn, cotton, rice or soybean plants, particularly in Northern and Southern American countries, Asia and Australia. The term plant, as used herein, encompasses whole plants as well as parts of plants, such as leaves, stems, seeds, flowers or roots. Particularly preferred target insects for the ISP3 proteins of this invention are leptidopteran insect pests, such as Heliothis spp., Helicoverpa spp., Spodoptera spp., Ostrinia spp., Pectinophora spp, Agrotis spp., Scirphophaga spp., Cnaphalocrocis spp., Sesamia spp, Chilo spp., Anticarsia spp., Pseudoplusia spp., Epinotia spp., and Rachiplusia spp., preferably *Heliothis virescens, Helicoverpa zea, Helicoverpa armigera, Helicoverpa punctera, Ostrinia nubilalis, Spodoptera frugiperda, Agrotis ipsilon, Pectinophora gossypiella, Scirphophaga incertulas, Cnaphalocrocis medinalis, Sesamia inferens, Chilo partellus, Anticarsia gemmatalis, Pseudoplusia includens, Epinotia aporema* and *-Rachiplusia nu.* The-ISP3 proteins of-the-invention-preferably have insecticidal activity against at least one lepidopteran insect species, more preferably against several Lepidopteran insect species.

The terms "ISP3 protein", "ISP3 protein of this invention", "ISP protein", or "ISP protein of this invention", as used herein, refers to the new protein isolated in accordance with this invention and identified and defined herein as ISP3-327D protein.

An ISP3 protein, as used herein, can be a protein in the full length size or can be in a truncated form as long as the insecticidal activity is retained, or can be a combination of different proteins or protein domains in a hybrid or fusion protein. An "ISP3 toxin" refers to an insecticidal fragment or portion of an ISP3, protein, particularly the smallest toxic fragment thereof. An "*isp* gene", "*isp3* gene", "*isp* DNA" or "*isp3* DNA", as used herein, is a DNA sequence encoding an ISP3 protein in accordance with this invention, referring particularly to the *isp3-327D* DNA sequences defined above.

The nucleic acid sequence, particularly DNA sequence, encoding the ISP3 proteins of this invention can be made synthetically and can be inserted in expression vectors to produce high amounts of ISP3 proteins. The ISP3 proteins can be used to prepare specific monoclonal or polyclonal antibodies in a conventional manner (Höfte et al., 1988; Harlow and Lane, 1988).

Also disclosed herein are antibodies that specifically bind to the ISP3 protein. In particular, monoclonal or polyclonal antibodies that bind ISP3-327D, or to fragments or variants thereof are disclosed. Included are fragments of monoclonal or polyclonal antibodies, which retain the ability to bind to the ISP3 protein or fragment against which they were raised. An antibody to an ISP3 protein can be prepared by using the ISP3 protein as an antigen in an animal (such as rabbit or mouse), using methods known in the art, such as described in Harlow and Lane "Using Antibodies: A Laboratory Manual" (New York: Cold Spring Harbor Laboratory Press, 1998), in Liddell and Cryer "A Practical Guide to Monoclonal Antibodies" (Wiley and Sons, 1991). The antibodies can be used to isolate, identify, characterize or purify the ISP3 protein to which it binds. For example, the antibody can be used to detect the ISP3 protein in a sample, by allowing antibody and protein to form an immunocomplex, and detecting the presence of the immunocomplex, for example through ELISA or immunoblots.

In addition, immunological kits, useful for the detection of ISP3 proteins, protein fragments or epitopes in a sample are provided. Samples may be cells, cell supernatants, cell suspensions, and the like. Such kit comprises an antibody that binds to the ISP3 protein (or fragment thereof) and one or more immunodetection reagents.

The antibodies can also be used to isolate insecticidal proteins with similar activity by for example ELISA (enzyme linked immunoassay) or Western blotting. Monoclonal antibody lines with desired binding specificity can also be used to clone the DNA for the particular monoclonal antibody.

Also disclosed herein are PCR primers and/or probes and kits for detecting the isp3-237D DNA sequences are provided. PCR primer pairs to amplify isp3 DNA from samples can be synthesized based on SEQ ID No. 3, as known in the art (see Dieffenbach and Dveksler (1995) PCR Primer: A Laboratory Manual, Cold Spring Harbor Laboratory Press, and McPherson at al. (2000) PCR - Basics: From Background to Bench, First Edition, Springer Verlag, Germany). Likewise, DNA fragments of SEQ ID No. 3 can be used as hybridization probes. An isp3 detection kit may comprise either isp3 specific primers or isp3 specific probes, and an associated protocol to use the primers or probe to detect isp3 DNA in a sample. Such a detection kit may, for example, be used to determine, whether a plant has been transformed with an isp3 gene (or part thereof) of the invention.

Because of the degeneracy of the genetic code, some amino acid codons can be replaced by others without changing the amino acid sequence of the protein. Furthermore, some amino acids can be substituted by other equivalent amino acids without significantly changing, preferably without changing, the insecticidal activity of the protein, at least without changing the insecticidal activity of the protein in a negative way. For example, to obtain a claimed ISP3 protein, conservative amino acid substitutions within the categories basic (e.g. Arg, His, Lys), acidic (e.g. Asp, Glu), nonpolar (e.g. Ala, Val, Trp, Leu, Ile, Pro, Met, Phe, Trp) or polar (e.g. Gly, Ser, Thr, Tyr, Cys, Asn, Gln) fall within the scope of the invention as long as the insecticidal activity of the ISP3 protein is not significantly, preferably not, changed, at least not changed in a negative way. In addition, in the claimed ISP3 proteins, non-conservative amino acid substitutions fall within the scope of the invention as long as the insecticidal activity of the ISP3 protein is not changed significantly, preferably not, or at least is notchanged in a negative way. Variants orequivalents of the DNA sequences of the invention include DNA sequences hybridizing to the *isp3* DNA sequence of SEQ ID No. 3 under stringent hybridization conditions and encoding a claimed ISP3 protein, or DNA sequences having a different codon usage compared to the native *isp3* genes of this invention but which encode a claimed ISP3 protein. The *isp3* DNA sequences can be codon-optimized by adapting the codon usage to that most preferred in plant genes, particularly to genes native to the plant genus or species of interest (Bennetzen & Hall, 1982; Itakura et al., 1977) using available codon usage tables (e.g. more adapted towards expression in cotton, soybean corn or rice). Codon usage tables for various plant species are published for example by Ikemura (1993) and Nakamura et al. (2000).

Also long stretches of AT or GC nucleotides may be removed and suitable restriction sites may be introduced.

Also, the N-terminus of an ISP3 protein can be modified to have an optimum translation initiation context, thereby adding or deleting one or more amino acids at the N-terminal end of the protein. In most cases, it is preferred that the proteins of the invention to be expressed in plants-cells start with a Met-Asp or Met-Ala dipeptide for optimal translation initiation, requiring the insertion in the *isp3* DNA of a codon encoding an Asp or Ala amino acid downstream of the start codon as a new second codon. Alternatively, the fourth nucleotide of SEQ ID No. 3 may be replaced by a 'G', so that the second amino acid (following Met) is Asp. Likewise, the second codon (AAC or AAT, coding for Asn) may be replaced by a codon for Asp (GAT or GAC) or Ala (GCT, GCC, GCA or GCG), or by any other codon starting with a 'G'.

The DNA sequences may also be modified to remove illegitimate splice sites. As bacterial genes may contain motifs, which are recognised in other hosts, especially in eukaryotic host such as plants, as 5' or 3' splice sites, transcription in those other hosts may be terminated prematurely, resulting in truncated mRNA. Illegitimate splice sites can be identified by computer based analysis of the DNA sequences and/or by PCR analysis as known in the art.

Of course, any DNA sequence differing in its codon usage but encoding the same protein or a similar protein with substantially the same insecticidal activity, can be constructed, depending on the particular purpose. It has been described in prokaryotic and eukaryotic expression systems that changing the codon usage to that of the host cell is desired for gene expression in foreign hosts (Bennetzen & Hall, 1982; Itakura et al., 1977). Codon usage tables are available in the literature (Wada et al., 1990; Murray et al., 1989) and in the major DNA sequence databases (e.g. EMBL at Heidelberg, Germany) and as described by Nakamura et al (2000). Accordingly, synthetic DNA sequences can be constructed so that the same or substantially the same proteins are produced. It is evident that several DNA sequences can be made once the amino acid sequence of the ISP3 proteins of this invention is known. Such other DNA sequences include synthetic or semi-synthetic DNA sequences that have been changed in order to inactivate certain sites in the gene, e.g. by selectively inactivating certain cryptic regulatory or processing elements present in the native sequence as described in PCT publications WO 91/16432 and WO 93/09218, or by adapting the overall codon usage to that of a more related host organism, preferably that of the host organism in which expression is desired. Several techniques for modifying the codon usage to that preferred by the host cells can be found in patent and scientific literature. The exact method of codon usage modification is not critical for this invention as long as most or all of the cryptic regulatory sequences or processing elements have been replaced by other sequences.

Small modifications to a DNA sequence such as described above can be routinely made, i.e., by PCR-mediated mutagenesis (Ho et al., 1989, White et al., 1989). More profound modifications to a DNA sequence can be routinely done by *de novo* DNA synthesis of a desired coding region using available techniques.

With the term "substantially the same", when referring to the amino acid sequence of an ISP3 protein, is meant to include an amino acid sequence that differs no more than 5 %, preferably no more than 2 %, from the amino acid sequence of the protein compared to; and when referring to toxicity of an ISP3 protein, is meant to include a protein whose mean LC₅₀ value (which is the concentration of protein causing 50% mortality of the test population), calculated from three independent bioassays, carried out using the same bioassay-conditions, differs by no more than a factor 2 from the mean LC₅₀ value obtained for the protein compared to (also calculated from three independent bioassays, carried out using the same bioassay-conditions as for the protein compared to) LC₅₀ values are calculated with Probit analyis, using the program POLO PC (from LeOra Software, 1987, Berkely, California). It is understood, that 95% (or 90%) confidence limits (an associated parameter calculated with Probit analysis) are calculated for the LC₅₀ values of each of the two proteins to be compared in order to determine whether a statistically significant difference in LC₅₀ values exists. In general the toxicity of the two proteins is seen to be substantially the same, if the confidence limits overlap and substantially different if the confidence limits do not overlap.

The term "domain" of a ISP3 toxin (or ISP3 protein) as used herein means any part(s) or domain(s) of the toxin (or ISP3 protein) with a specific structure or function that can be transferred to another protein for providing a new hybrid protein with at least one functional characteristic (e.g., the binding and/or toxicity characteristics) of the ISP3 toxin (or ISP3 protein) of the invention (Ge et al., 1991). Such parts can form an essential feature of the hybrid protein with the binding and/or toxicity characteristics of the ISP3 proteins of this invention. Such a hybrid protein can have an enlarged host range, an improved toxicity and/or can be used in a strategy to prevent insect resistance development (EP 408 403; Visser et al., 1993). DNA sequences encoding the domains are encompassed by this definition. A hybrid protein or fusion protein is used herein to mean a protein comprised of different protein domains, forming a functional, chimeric protein with the characteristics of the individual domains. Another domain which a hybrid or chimeric protein may, for example, comprise is a stabilising domain. Stabilising domains have for example been described to be present at the C-terminus of VIP3(a) proteins, and arethoughtto provide stability to the toxic protein in the gut-environment of susceptible insects.

In addition to creating hybrid proteins, the function of specific domains can also be analyzed by the introduction of deletions of all or part of the domain(s) or the introduction of mutations into the domain, and analysis of the resulting effect on toxicity towards insects, protein stability, sensitivity to enzyme proteolysis, temperature changes, binding to DNA/proteins/specific cells, etc..

Also disclosed herein is a method of "evolving" a nucleic acid sequence-encoding an ISP3 protein, particularly ISP3-327D, into a new nucleic acid sequence, which encodes a protein as claimed having insecticidal activity. The evolved nucleic acid sequence preferably has improved insecticidal activity compared to the non-evolved sequence. The term "evolving" as used herein refers to a method of enhanced sequence evolution by recombination of sequences, as described in US 5,811,238, WO97/20078 and US 6,180,406. Nucleic acid "shuffling" is used herein to indicate in vitro or in vivo recombination between nucleic acid sequences of a nucleic acid population or pool and can be carried out as known in the art and as described in US 5,811,238, WO97/20078, US 6,180,406, US 6,117,679.

The method of evolving a nucleic acid sequence encoding a claimed ISP3 protein comprises the following steps:
(a) providing a population of nucleic acid sequences encoding the amino acid sequences of SEQ ID No. 4, or variants or fragments of the amino acid sequences of SEQ ID No. 4, wherein said variants or fragments have a sequence identity of at least 91 % to SEQ ID No. 4.
(b) shuffling said population of variants or fragments to form recombinant nucleic acid molecules
(c) selecting or screening for recombinant nucleic acid molecules, which encode claimed proteins that have insecticidal activity;
repeating steps (a) to (c) with the recombinant nucleic acid molecules selected in step (c) until a recombinant nucleic acid molecule has been found in step (c), wherein the claimed protein encoded by said nucleic acid molecule has the desired insecticidal property.

A non-evolved nucleic acid is a nucleic acid provided as starting material in step (a), while a corresponding evolved nucleic acid as used herein refers to a recombinant nucleic acid obtained in step (d) when carrying out the method using the non-evolved nucleic acid in step (a). Preferred nucleic acids used in step (a) are nucleic acid sequences encoding amino acid sequences ISP3-327D (SEQ ID No. 4) or variants or fragments thereof as claimed herein. The population of nucleic acid molecules and/or variants and/orfragments of nucleic acid molecules in step (a) may comprise the DNA encoding a single ISP3 protein and/or variants and/or fragments of the nucleic acid encoding a single ISP3 protein of the invention, or a mixture of nucleic acids encoding different ISP3 proteins of the invention, and/or fragments and/or variants thereof.

Nucleic acid sequences encoding claimed variants of amino acid sequence SEQ ID No. 4 are nucleic acid sequences encoding amino acid sequences of claimed ISP3 proteins, e.g. those claimed ISP3 proteins which have at least 91%, preferably at least 92 or 93%, most preferably at least 94%, 95%, 98%, 99% or 100% sequence identity at the amino acid level to SEQ ID No. 4.

The *isp3* DNA sequences of the invention, prepared from total DNA, can be ligated in suitable expression vectors and transformed in a bacterial strain, such as *E. coli* or a Bt strain, and the clones can then be screened by conventional colony immunoprobing methods (French et al., 1986) for expression of the toxin with monoclonal or polyclonal antibodies raised against the ISP3 proteins.

The Bt or *E*. *coli* clones can then be screened for production of ISP3 proteins (cell-free culture supernatant or cell lysate can be run on SDS-PAGE gels using standard methods and standard western-blotting procedures can be carried out), or the bacteria can be tested for their insecticidal activity compared to the control bacteria. The clones can also be analysed for the presence of mRNA encoding ISP3 protein using standard PCR procedures, such as RT-PCR.

The genes encoding the ISP3 proteins of this invention can be sequenced in a conventional manner (Maxam and Gilbert, 1980; Sanger, 1977) to obtain the DNA sequence. Sequence comparisons indicated that the genes are different from previously described genes encoding toxins with activity against Lepidoptera.

An insecticidally effective part of the DNA sequences, encoding an insecticidally effective portion of the newly identified ISP3 proteins, can be made in a conventional manner after sequence analysis of the gene. The amino acid sequence of the ISP3 proteins can be determined from the DNA sequence of the isolated DNA sequences. By "an insecticidally effective part (or portion or fragment)" of DNA sequences encoding the ISP3 protein, also referred to herein as "truncated gene" or "truncated DNA", is meant a DNA sequence encoding a polypeptide which has fewer amino acids than the ISP3 full length protein form but which is insecticidal.

In order to express all or an insecticidally effective part of the DNA sequence encoding an ISP3 protein of this invention in *E*. *coli,* in other *Bt* strains and in plants, suitable restriction sites can be introduced, flanking the DNA sequence. This can be done by site-directed mutagenesis, using well-known procedures (Stanssens et al., 1989; White et al., 1989). In order to obtain improved expression in plants, the codon usage of the *isp3* gene or insecticidally effective *isp3* gene part of this invention can be modified to form an equivalent, modified or artificial gene or gene part in accordance with PCT publications WO 91/16432 and WO 93/09218 and publications EP 0 385 962, EP 0 359 472 and US 5,689,052, or the *isp3* genes or gene parts can be inserted in the plastid, mitochondrial or chloroplast genome and expressed there using a suitable promoter (e.g., Mc Bride et al., 1995; US 5,693,507).

For obtaining enhanced expression in monocot plants such as corn or rice, an intron, preferably a monocot intron, can also be added to the chimeric gene. For example the insertion of the intron of the maize Adh1 gene into the 5' regulatory region has been shown to enhance expression in maize (Callis et. al., 1987). Likewise, the HSP70 intron, as described in US 5,859,347, may be used to enhance expression. The DNA sequence of the *isp3* gene or its insecticidal part can be further changed in a translationally neutral manner, to modify possibly inhibiting DNA sequences present in the gene part by means of site-directed intron insertion and/or by introducing changes to the codon usage, e.g., adapting the codon usage to that most preferred by plants, preferably the specific relevant plant genus (Murray et al., 1989), without changing significantiy, preferably without changing, the encoded amino acid sequence.

In accordance with one embodiment of this invention, it is preferred that the proteins are targeted to intracellular organelles such as plastids, preferably chloroplasts, mitochondria, or are secreted from the cell, potentially optimizing protein stability and/or expression. For this purpose, in one embodiment of this invention, the chimeric genes of the invention comprise a coding region encoding a signal or target peptide, linked to the ISP3 protein coding region of the invention. Particularly preferred peptides to be included in the proteins of this invention are the transit peptides for chloroplast or other plastid targeting, especially duplicated transit peptide regions from plant genes whose gene product is targeted to the plastids, the optimized transit peptide of Capellades et al. (US 5,635,618), the transit peptide of ferredoxin-NADP⁺oxidoreductase from spinach (Oelmuller et al., 1993), the transit peptide described in Wong et al. (1992) and the targeting peptides in published PCTpatent application WO 00/26371. Also preferred are peptides signalling secretion of a protein linked to such peptide outside the cell, such as the secretion signal of the potato proteinase inhibitor II (Keil et al., 1986), the secretion signal of the alpha-amylase 3 gene of rice (Sutliff et al., 1991) and the secretion signal of tobacco PR1 protein (Cornelissen et al., 1986).

Particularly useful signal peptides in accordance with the invention include the chloroplast transit peptide (e.g., Van Den Broeck et al., 1985), or the optimized chloroplast transit peptide of US 5,510,471 and US 5,635,618 causing transport of the protein to the chloroplasts, a secretory signal peptide or a peptide targeting the protein to other plastids, mitochondria, the ER, or another organelle. Signal sequences for targeting to intracellular organelles or for secretion outside the plant cell or to the cell wall are found in naturally targeted or secreted proteins, preferably those described by Klösgen et al. (1989), Klösgen and Weil (1991), Neuhaus & Rogers (1998), Bih et al. (1999), Morris et al. (1999), Hesse et al. (1989), Tavladoraki et al. (1998), Terashima et al. (1999), Park et al. (1997), Shcherban et al. (1995), particularly the signal peptide sequences from targeted or secreted proteins of corn, cotton, soybean or rice.

To allow secretion of the ISP3 proteins to the outside of the transformed host cell, an appropriate secretion signal peptide may be fused to the amino terminal end (N-terminal end) of the ISP3 protein. Also, any putative native *Bacillus* secretion signal peptide can be deleted or can be replaced by an appropriate signal peptide, such as a eukaryotic secretion signal peptide as described above. Particularly, amino acids 1 to 54 of the ISP3 proteins of the invention comprise a putative *Bacillus* signal peptide. Amino acids 1 to 10, preferably 1 to 50, more preferably 1 to 54 may be removed from the ISP3 proteins or may be replaced by an appropriate signal peptide, particularly a eukaryotic signal peptide as described above. Putative signal peptides can be detected using computer based analysis, using programs such as the program Signal Peptide search (SignalP V1.1 or 2.0), using a matrix for prokaryotic gram-positive bacteria and a threshold score of less than 0.5, especially a threshold score of 0.25 or less (Von Heijne, Gunnar, 1986 and Nielsen et al., 1996).

Furthermore, the binding properties of the ISP3 proteins of the invention can be evaluated, using methods known in the art (e.g., Van Rie et al., 1990), to determine if the ISP3 proteins of the invention bind to sites in the insect gut, such as the midgut, that are not recognized (or competed for) by other *Bt* proteins. Insecticidal *Bt* proteins with different binding sites for which there is no competition for binding in relevant susceptible insects are very valuable to replace known *Bt* proteins to which insects may have developed resistance, or to use in combination with insecticidal *Bt* proteins having a different mode of action to prevent or delay the development of insect resistance against *Bt* proteins, particularly when expressed in a plant. Because of the characteristics of the newly isolated *Bt* toxins, they are extremely useful for transforming plants, e.g. monocots such as corn and rice and dicots such as cotton and soybean, to protect these plants from insect damage. It is expected that the binding properties of the ISP3 proteins of the current invention will be different compared to those of Cry toxins. Such different binding properties can be measured by routine binding assays as described above or in US 6,291,156 and US 6,137,033.

Especially for insect resistance management purposes for a specific insect pest, it is preferred to combine an ISP3 protein of this invention with another insect control protein, particularly a Bt Cry protein or a VIP or VIP-like protein, preferably a protein which does not recognise at least one binding site recognised by such ISP3 protein. Preferred insect control proteins to combine with the ISP3 proteins of this invention, particularly for simultaneous expression in plants, preferably maize, cotton, rice or soybean plants, include the Cry proteins, such as the Cry1 F protein or hybrids derived from a Cry1 F protein (e.g., the hybrid Cry1A-Cry1F proteins described in US 6,326,169; US 6,281,016; US 6,218,188, or toxic fragments thereof), the Cry1A-type proteins or toxic fragments thereof, preferably the Cry1 Ac protein or hybrids derived from the Cry1 Ac protein (e.g., the hybrid Cry1 Ab-Cry1 Ac protein described in US 5,880,275) or the Cry1 Ab or Bt2 protein or insecticidal fragments thereof as described in EP451878, the Cry2Ae, Cry2Af or Cry2Ag proteins as described in WO02/057664, the Cry proteins as described in WO01/47952, the VIP3Aa protein or a toxic fragment thereof as described in Estruch et al. (1996) and US 6,291,156, insecticidal proteins from *Xenorhabdus* (as described in WO98/50427) *Serratia* (particularly from *S*. *entomophila*) or *Photorhabdus* species strains, such as Tc-proteins from Photorhabdus as described in WO98/08932 (e.g., Waterfield et al., 2001; Ffrench-Constant and Bowen, 2000). In one embodiment, such co-expression is easily obtained by transforming a plant already expressing an insect control protein with a ISP3 of this invention, or by crossing plants transformed-with the insect control protein and plants transformed with one or more ISP proteins of this invention. For maize, rice, cotton or soybean plants, preferably the ISP3-327D protein is used as first insect control protein and as second insect control protein the Cry1 Ab, Cry1 Ac, Cry2Ae or VIP3Aa proteins or derivatives thereof are used. Methods for obtaining expression of different Bt (or similarly, for other insect control proteins) insecticidal proteins in the same plant in an effort to minimize or prevent resistance development to transgenic insect-resistant plants are described in EP 0 408 403. It is understood that the different proteins can be expressed in the same plant, or each can be expressed in a single plant and then combined in the same plant by crossing the single plants with one another. For example, in hybrid seed production, each parent plant can express a single protein. Upon crossing the parent plants to produce hybrids, both proteins are combined in the hybrid plant.

It is well known that Bt Cry proteins are expressed as protoxins, which are converted into the toxic core by proteolysis in the insect gut When combining the ISP3 proteins of the invention with Bt Cry proteins, it is understood that Bt Cry genes either encoding the full protoxin or the toxic core or any intermediate form may be used.

Preferably, for selection purposes but also for increasing the weed control options, the transgenic plants of the invention are also transformed with a DNA encoding a protein conferring resistance to a broad-spectrum herbicide, e.g., herbicides based on glufosinate or glyphosate.

The insecticidally effective *isp3* gene part or its equivalent, preferably the *isp3* chimeric gene, encoding an insecticidally effective portion of the ISP3 protein, can be stably inserted in a conventional manner into the nuclear genome of a single plant cell, and the so-transformed plant cell can be used in a conventional manner to produce a transformed plant that is insect-resistant. In this regard, a T-DNA vector, containing the insecticidally effective *isp3* gene part, in *Agrobacterium tumefaciens* can be used to transform the plant cell, and thereafter, a transformed plant can be regenerated from the transformed plant cell using the procedures described, for example, in EP 0 116 718, EP 0 270 822, PCT publication WO 84/02913 and published European Patent application EPO 242 246 and in Gould et al. (1991). The construction of a T-DNA vector for Agrobacterium mediated plant transformation is well known in the art. The T-DNA vector may be either a binary vector as described in EP 0120 561 and EP 0120 515 or a co-integrate vector which can integrate into the Agrobacterium Ti-plasmid by homologous recombination, as described in EP 0116 718. Preferred T-DNA vectors each contain a promoter operably linked to the insecticidally effective *isp3* gene part between T-DNA border sequences, or at least located to the left of the right border sequence. Border sequences are described in Gielen et al. (1984). Of course, other types of vectors can be used to transform the plant cell, using procedures such as direct gene transfer (as described, for example in EP 0 223 247), pollen mediated transformation (as described, for example in EP 0 270 356 and WO 85/01856), protoplast transformation as, for example, described in US 4,684,611, plant RNA virus-mediated transformation (as described, for example in EP 0 067 553 and US 4,407,956), liposome-mediated transformation (as described, for example in US 4,536,475), and other methods such as the recently described methods for transforming certain lines of corn (e.g., US 6,140,553; Fromm et al., 1990; Gordon-Kamm et al., 1990) and rice (Shimamoto et al., 1989; Datta et al. 1990) and the method for transforming monocots generally (PCT publication WO 92/09696). For cotton transformation, especially preferred is the method described in PCT patent publication WO 00/71733. For rice transformation, reference is made to the methods described in WO92/09696, WO94/00977 and WO95/06722.

The terms 'maize' and "com" are used herein synonymously, referring to *Zea mays.* Cotton as used herein refers to *Gossypium* spp., particularly *G. hirsutum* and *G. barbadense.* The term "rice" refers to *Oryza* spp., particularly *O. sativa. "Soybean"* refers to *Glycine* spp, particularly *G*. *max.*

Besides transformation of the nucleargenome, also transformation of the plastid genome, preferably chloroplast genome, is included in the invention. Kota et al. (1999) have described a method to over-express a Cry2Aa protein in tobacco chloroplasts.

The resulting transformed plant can be used in a conventional plant breeding scheme to produce more transformed plants with the same characteristics or to introduce the insecticidally effective *isp3* gene part into other varieties of the same or related plant species. Seeds, which are obtained from the transformed plants, contain the insecticidally effective *isp3* gene part as a stable genomic insert. Cells of the transformed plant can be cultured in a conventional manner to produce the insecticidally effective portion of the ISP3 toxin or protein, which can be recovered for use in conventional insecticide compositions against Lepidoptera (US 5,254,799).

The insecticidally effective *isp3* gene part is inserted in a plant cell genome so that the inserted gene is downstream (i.e., 3') of, and under the control of, a promoter which can direct the expression of the gene part in the plant cell. This is preferably accomplished by inserting the *isp3* chimeric gene in the plant cell genome, particularly in the nuclear or plastid (e.g., chloroplast) genome.

Preferred promoters include: the strong constitutive 35S promoters (the "35S promoters") of the cauliflower mosaic virus (CaMV) of isolates CM 1841 (Gardner et al., 1981), CabbB-S (Franck et al., 1980) and CabbB-JI (Hull and Howell, 1987); the 35S promoter described by Odell et al. (1985), promoters from the ubiquitin family (e.g., the maize ubiquitin promoter of Christensen et al., 1992, EP 0 342 926, see also Cornejo et al., 1993), the gos2 promoter (de Pater et al., 1992), the emu promoter (Last et al., 1990), Arabidopsis actin promoters such as the promoter described by An et al. (1996), rice actin promoters such as the promoter described by Zhang et al. (1991) and the promoter described in US 5,641,876; promoters of the Cassava vein mosaic virus (WO 97/48819, Verdaguer et al. (1998)), the pPLEX series of promoters from Subterranean Clover Stunt Virus (WO 96/06932, particularly the S7 promoter), a alcohol dehydrogenase promoter, e.g., pAdh1S (GenBank accession numbers X04049, X00581), and the TR1' promoter and the TR2' promoter (the "TR1' promoter" and "TR2' promoter", respectively) which drive the expression of the 1' and 2' genes, respectively, of the T-DNA (Velten et al., .1984). Alternatively, a promoter can be utilized which is not constitutive but rather is specific for one or more tissues or organs of the plant (e.g., leaves and/or roots) whereby the inserted *isp3* gene part is expressed only in cells of the specific tissue(s) or organ(s). For example, the insecticidally effective *isp3* gene part could be selectively expressed in the leaves of a plant (e.g., corn, cotton, rice, soybean) by placing the insecticidally effective gene part under the control of a light-inducible promoter such as the promoter of the ribulose-1,5-bisphosphate carboxylase small subunit gene of the plant itself or of another plant, such as pea, as disclosed in US 5,254,799. The promoter can, for example, be chosen so that the *isp3* gene of the invention is only expressed in those tissues or cells on which the target insect pest feeds so that feeding by the susceptible target insect will result in reduced insect damage to the host plant, compared to plants which do not express the *isp3* gene. A Lepidopteran insect pest mainly damaging the roots can thus effectively be controlled by expressing an *isp3* gene under a root specific promoter. A promoter preferentially active in roots is described in WO00/29566. A preferred promoter for root preferential expression is the ZRP promoter (and modifications thereof) as described in US 5,633,363. Another alternative is to use a promoter whose expression is Inducible, for example a wound-inducible promoter such as e.g. the MPI promoter described by Cordera et al. (1994), which is induced by wounding (such as caused by insect feeding), or a promoter Inducible by a chemical, such as dexamethasone as described by Aoyama and Chua (1997) or a promoter inducible by temperature, such as the heat shock promoter described in US 5,447,858, or a promoter inducible by other external stimuli.

The insecticidally effective *isp3* gene part is inserted into the plant genome so that the inserted gene part is upstream (i.e., 5') of suitable 3' end transcription regulation signals (i.e., transcript formation and polyadenylation signals). This is preferably accomplished by inserting the *isp3* chimeric gene in the plant cell genome. Preferred polyadenylation and transcript formation signals include those of the CaMV 35S gene, the nopaline synthase gene (Depicker et al., 1982), the octopine synthase gene (Gielen et al., 1984) and the T-DNA gene 7 (Veiten and Schell, 1985), which act as 3'-untranslated DNA sequences in transformed plant cells.

Introduction of the T-DNA vector into Agrobacterium can be carried out using known methods, such as electroporation or triparental mating.

The insecticidally effective *isp3* gene part can optionally be inserted in the plant genome as a hybrid gene (US 5,254,799; Vaeck et al., 1987) under the control of the same promoter as a selectable or scorable marker gene, such as the *neo* gene (EP 0 242 236) encoding kanamycin resistance, so that the plant expresses a fusion protein which is easily detectable.

Transformation of plant cells can also be used to produce the proteins of the invention in large amounts in plant cell cultures, e.g., to produce a ISP3 protein that can then be applied onto crops after proper formulation. When reference to a transgenic plant cell is made herein, this refers to a plant cell (or also a plant protoplast) as such in isolation or in tissue culture, or to a plant cell (or protoplast) contained in a plant or In a differentiated organ or tissue, and both possibilities are specifically included herein. Hence, a reference to a plant cell in the description or claims is not meant to refer only to isolated cells in culture, but refers to any plant cell, wherever it may be located or in whatever type of plant tissue or organ it may be present.

All or part of the *isp3* gene, encoding an anti-Lepidopteran protein, can also be used to transform other microorganisms, such as bacteria, such as a *B*. *thuringiensis* which has insecticidal activity against Lepidoptera or Coleoptera.

Thereby, a transformed *Bt* strain can be produced which is useful for combating a wide spectrum of Lepidopteran and/or Coleopteran insect pests or for combating additional Lepidopteran Insect pests. Transformation of bacteria, such as bacteria of the genus *Pseudomonas, Agrobacterium, Bacillus* or *Escherichia,* with all or part of the *isp3* gene of this invention, incorporated in a suitable cloning vehicle, can be carried out in a conventional manner, preferably using conventional electroporation techniques as described in Mahillon et al. (1989) and in PCT Patent publication WO 90/06999.

Transformed *Bacillus* species strains containing the *isp3* gene of this invention can be fermented by conventional methods (Dulmage, 1981; Bernhard and Utz, 1993) to provide high yields of cells. Under appropriate growth conditions which are well understood, these strains secrete ISP3 proteins in high yields.

Alternative suitable host microorganisms in which the *isp3* genes can be expressed are fungi, algae, or viruses, particularly species which are plant colonising (e.g., (endo)symbiontic) species or insect pathogens.

An insecticidal, particularly anti-Lepidopteran, composition of this invention can be formulated in a conventional manner using the microorganisms transformed with the *isp3* gene, or preferably their respective ISP3 proteins or the ISP3 toxin, or an insecticidally effective toxin portion as an active ingredient, together with suitable carriers, diluents, emulsifiers and/or dispersants (e.g., as described by Bemhard and Utz, 1993). This insecticide composition can be formulated as a wettable powder, pellets, granules or dust or as a liquid formulation with aqueous or non-aqueous solvents as a foam, gel, suspension, concentrate, etc.. Examples of compositions comprising insecticidal Bt spores are described in WO96/10083.

A method for controlling insects, particularly Lepidoptera, in accordance with this invention can comprise applying (e.g., spraying), to a locus (area) to be protected, an insecticidal amount of the ISP3 proteins or compositions comprising the ISP3 proteins or comprising host cells transformed with the *isp3* genes of this invention. The locus to be protected can include, for example, the habitat of the insect pests or growing vegetation (e.g. application to the foliage) or an area where vegetation is to be grown (e.g. application to soil or water). A preferred composition comprises an insecticidal amount of at least one of the ISP3 proteins of the invention, preferentially produced by a bacterial host, and the preferred application of the composition are leaf application, soil application or seed coating.

The term "contacting" is used herein to mean "to bring into physical contact with". Contacting a plant with an insecticidal protein means that the insecticidal protein is brought into contact with cells of the plant, either internally (for example by expression in the plant) or externally (for example by applying compositions comprising the insecticidal protein externally to the plant). It is understood that the term does not indicate the length of time of contact, but comprises any time period of contact (e.g. brief contact, long contact). When referring to a method of protecting a plant against insect damage comprising contacting said plant (or cells or tissues thereof) with an insecticidal protein of the invention, the contact is preferentially long enough and extensive enough (with a high enough amount of protein contacting a large enough number of cells) to prevent or reduce insect damage.

This invention further relates to a method for controlling Lepidopteran cotton insect pests, particularly bollworms, budworms, earworms, preferably a Lepidopteran cotton insect pest selected from the group of *Helicoverpa zea* (Corn Earworm), *Helicoverpa armigera* (Cotton Bollworm); *Helicoverpa punctigera* (Native Bollworm), *Heliothis virescens* (Tobacco Budworm), *Spodoptera frugiperda* (Fall Armyworm) and *Pectinophora gossypiella* (Pink Bollworm), which method comprises applying to an area or plant to be protected, a claimed ISP3 protein as defined herein, preferably a ISP3-327D protein as defined herein, (i.e. by contacting a cotton plant with an ISP3 protein of this invention, for example by planting a cotton plant transformed with an *isp3* gene of this invention, or spraying a composition containing a ISP3 protein of this invention). The invention also relates to the use of the ISP3 proteins of this invention, particularly the ISP3-327D protein, against Lepidopteran cotton insect pests to minimize damage to cotton plants.

This invention further relates to a method for controlling Lepidopteran maize insect pests, particularly earworms, armyworms, corn borers, preferably a maize insect pest selected from the group of *Helicoverpa zea* (Corn Earworm), *Agrotis ipsilon* (Black Cutworm), *Ostrinia nubilalis* (European Com Borer) and *Spodoptera frugiperda* (Fall Armyworm), which method comprises applying to an area or plant to be protected, a claimed ISP3 protein as defined herein, preferably a ISP3-327D protein as defined herein, (i.e. by contacting a maize plant with an ISP3 protein of this invention, for example by planting a maize plant transformed with an *isp3* gene of this invention, or spraying a composition containing a ISP3 protein of this invention). The invention also relates to the use of the claimed ISP3 proteins of this invention, particularly the ISP3-327D protein, against Lepidopteran maize insect pests to minimize damage to maize plants.

This invention further relates to a method for controlling Lepidopteran rice insect pests, particularly rice stem-borers, rice skippers, rice cutworms, rice armyworms, rice caseworms, rice leaffolders, preferably a rice insect pest selected from the group of Yellow Stem Borer (*Scirphophaga incertulas*)*,* Leaffolder (*Cnaphalocrocis medinalis*)*,* Pink Stem Borer (*Sesamia inferens*) and Corn Spotted Stem Borer (*Chilo partellus*), which method comprises applying to an area orplant to be protected, a claimed ISP3 protein as defined herein, preferably a ISP3-327D protein as defined herein, (i.e. by contacting a rice plant with an ISP3 protein of this invention, for example by planting a rice plant transformed with an *isp3* gene of this invention, or spraying a composition containing a ISP3 protein of this invention). The invention also relates to the use of the claimed ISP3 proteins of this invention, particularly the ISP3-327D protein, against Lepidopteran rice insect pests to minimize damage to rice plants.

This invention further relates to a method for controlling Lepidopteran soybean insect pests, preferably a soybean insect pest selected from the group of Velvet Bean Caterpillar (*Anticarsia gemmatalis*)*,* Soybean Looper (*Pseudoplusia includens*), Beet Armyworm (*Spodoptera exigua*)*,* Yellowstriped Armyworm (*Spodoptera ornithogalli*), Com Earworm (*Helicoverpa* zea), Pod Borer (*Epinotia aporema*) and *Rachiplusia nu,* which method comprises applying to an area or plant to be protected, a claimed ISP3 protein as defined herein, preferably an ISP3-327D protein as defined herein, (i.e. by contacting a soybean plant with an ISP3 protein of this invention, for example by planting a soybean plant transformed with an *isp3* gene of this invention, or spraying a composition containing a ISP3 protein of this invention). The invention also relates to the use of the claimed ISP3 proteins of this invention, particularly the ISP3-327D protein, against Lepidopteran soybean insect pests to minimize damage to soybean plants.

To obtain the ISP3 toxin or protein, cells of the recombinant hosts expressing the ISP3 protein can be grown in a conventional manner on a suitable culture medium. The secreted toxin can be separated and purified from the growth medium. Alternatively, if the proteins are not secreted, the cells can be lysed using conventional means such as enzyme degradation or detergents or the like. The toxin can then be separated and purified by standard techniques such as chromatography, extraction, electrophoresis, or the like.

The term "gene" means any DNA or RNA fragment comprising a region (the "transcribed region") which is transcribed into an RNA molecule (e.g., an mRNA) in a cell, operably linked to suitable regulatory regions, e.g., a plant-expressible promoter. A gene may thus comprise several operably linked fragments such as a promoter, a 5' leader sequence, a coding region, and a 3' nontranslated sequence, comprising a polyadenylation site. A gene endogenous to a particular organism (such as a plant species or a bacterial strain) is a gene, which is naturally found in that organism in nature. A chimeric gene, when referring to an *isp3* DNA of this invention, refers to an *isp3* DNA sequence having 5' and/or 3' regulatory sequences different from the naturally-occurring bacterial 5' and/or 3' regulatory sequences, which drive the expression of the *isp3* gene in its native host cell.

The term "expression of a gene" refers to the process wherein a DNA or RNA region which is operably linked to appropriate regulatory regions, particularly to a promoter, is transcribed into an RNA which is biologically active i.e., which is either capable of interaction with another nucleic acid or which is capable of being translated into a biologically active polypeptide or protein. A gene is said to encode an RNA when the end product of the expression of the gene is biologically active RNA, such as e.g. an antisense RNA, a ribozyme or a replicative intermediate. A gene is said to encode a protein when the end product of the expression of the gene is a biologically active protein or polypeptide.

For the purpose of this invention the "sequence identity" of two related nucleotide or amino acid sequences, expressed as a percentage, refers to the number of positions in the two optimally aligned sequences which have identical residues (x100) divided by the number of positions compared. A gap, i.e. a position in an alignment where a residue is present in one sequence but not in the other is regarded as a position with non-identical residues. To calculate sequence identity between two sequences for the purpose of this invention, the GAP program, which uses the Needleman and Wunsch algorithm (1970) and which is provided by the Wisconsin Package, Version 10.2, Genetics Computer Group (GCG), 575 Science Drive, Madison, Wisconsin 53711, USA, is used. The GAP parameters used are a gap creation penalty = 50 (nucleotides) / 8 (amino acids), a gap extension penalty = 3 (nucleotides) / 2 (amino acids), and a scoring matrix "nwsgapdna" (nucleotides) or "blosum62" (amino acids).

GAP uses the Needleman and Wunsch global alignment algorithm to align two sequences over their entire length, maximizing the numberof matches and minimizes the number of gaps. The default parameters are a gap creation penalty = 50 (nucleotides) /8 (proteins) and gap extension penalty = 3 (nucleotides) / 2 (proteins). For nucleotides the default scoring matrix used is "nwsgapdna" and for proteins the default scoring matrix is "blosum62" (Henikoff & Henikoff, 1992).

These and/or other embodiments of this invention are reflected in the wordings of the claims, which form part of the description of the invention.

The following Examples illustrate the invention, and are not provided to limit the invention or the protection sought. The sequence listing referred to in the Examples, the Claims and the Description is as follows:
SEQ ID No. 1: DNA sequence of *isp3-1099E* reference DNA
SEQ ID No. 2: amino acid sequence of ISP3-1099E reference protein
SEQ ID No. 3: DNA sequence *isp3-327D*
SEQ ID No. 4: amino acid sequence ISP3-327D
SEQ ID No. 5: DNA sequence of *isp3-2245J* reference DNA
SEQ ID No. 6: amino acid sequence of ISP3-2245J reference protein

Unless stated otherwise in the Examples, all recombinant DNA techniques are carried out according to standard protocols as described in Sambrook and Russell (2001) Molecular Cloning: A Laboratory Manual, Third Edition, Cold Spring Harbor Laboratory Press, NY, in Volumes 1 and 2 of Ausubel et al. (1994) Current Protocols in Molecular Biology, Current Protocols, USA and in Volumes I and II of Brown (1998) Molecular Biology LabFax, Second Edition, Academic Press (UK). Standard materials and methods for plant molecular work are described in Plant Molecular Biology Labfax (1993) by R.D.D. Croy, jointly published by BIOS Scientific Publications Ltd (UK) and Blackwell Scientific Publications, UK. Standard materials and methods for polymerase chain reactions can be found in Dieffenbach and Dveksler (1995) PCR Primer: A Laboratory Manual, Cold Spring Harbor Laboratory Press, and in McPherson at al. (2000) PCR - Basics: From Background to Bench, First Edition, Springer Verlag, Germany.

### Examples

### Reference Example 1: Characterization of bacterial strains

A bacterial strain, named herein BTS01099E, was Isolated from grain dust from Belgium. A further bacterial strain, named herein BTS00327D, was isolated from horse dung from Spain. A further bacterial strain, herein named BTS02245J, was isolated from grain dust from the Philippines.

Each strain was grown overnight on LB agar plates (LB medium with 1.5 % agar added; LB medium:10 g/l trypton, 10 g/l NaCl, 5 g/l yeast extract, pH 7.3) at 28°C. For small scale cultures, 20 ml TB medium (Terrific Broth: 12 g/l tryptone, 24 g/l yeast extract, 3.8 g/l KH₂PO₄, 12.5 g/l K₂HPO₄, 5 ml/l glycerol, pH 7.1) was inoculated and grown for 65 hours at 28°C on a rotating platform having about 70 rotations per minute. After 65 hours, a protease inhibitor mixture was added to the culture. This cocktail has the following ingredients (volumes given are those required to add to one 20 ml culture): 200µl PMSF (100mM), 200µl of a mixture of benzamidine HCl (100mM) and epsilon-amino-n-caproic acid (500 mM), 400µl EGTA (0.5M), 40µl antipain (0.5mg/ml) / leupeptin (0.5mg/ml) and 20µl beta-mercapto ethanol (14M).

The culture medium to which the protease inhibitor mixture had been added, was then centrifuged for 20 minutes at 3000 rpm. In some cases, the supernatant was concentrated about 4 times using Centriprep YM-1 0 Centrifugal Filter Devices (Millipore Cat. No. 4305).

For long term storage, a loop of sporulated cells was added to 0.5ml of 25% glycerol and after vortexing, stored at -70°C. Sporulated cells were obtained by growth of the strain on LB agar plates until sporulation (as visible under the light microscope).

After cultivating on LB agar plates of single cell colonies, microscopical analysis of the strain cultures of BTS01099E, BTS00327D and BTS02245J showed the presence of rod-shaped, motile, single, vegetative cells and sporangia containing an ovale spore. Parasporal crystals were detected in cultures of BTS00327D, BTS01099E and BTS02245J.

Based on the rod-like shape, the aerobic growth, and the presence of parasporal crystals, these 3 strains are believed to be *B. thuringiensis* species strains.

Each strain can be cultivated on conventional standard media, preferably T₃ medium (tryptone 3 g/l, tryptose 2 g/l, yeast extract 1.5 g/l, 5 mg MnCl₂, 0.05 M Na₂HPO₄.2H₂O, 0.05 M NaH₂PO₄.H₂O, pH 6.8 and 1.5% agar), preferably at 28 °C. For long term storage, it is preferred to mix an equal volume of a spore-crystal suspension with an equal volume of 50% glycerol and store this at -70 °C or lyophilize a spore-crystal suspension. For sporulation, growth on T₃ medium is preferred for 72 hours at 28 °C.

### Reference Example 2: Insect bioassays of Bacillus strains (using culture supernatant containing Insecticidal protein)

Bioassays were performed on neonate larvae of *Helicoverpa zea, Heliothis virescens, Ostrinia nubilalis, Spodoptera frugiperda* and *Agrotis ipsilon.*

Cell-free supernatant of bacterial cultures of different Bacillus strains was used in insect bioassays ("surface contamination assay") against various lepidopteran insects.

Strain BTS01099E, BTS00327D or BTS02245J was grown at 28°C in TB medium. Cell-free culture supernatant was harvested 65 hours after culture initiation, dilutions were made and applied onto the surface of solidified artificial diet (agar 20g, water 1000ml, corn flour 96g, yeast 30g, wheat germs 64g, Wesson salt 7.5g, casein 15g, sorbic acid 2g, Aureomycin 0.3g, Nipagin 1g, wheat germ oil 4ml, sucrose 15g, cholesterol 1 g, ascorbic acid 3.5g,

Vanderzand mod. vit. mix 12g) (based on Vanderzand 1962), dispensed in wells of Costar 48-well plates and allowed to solidify. 25µl supernatant solution was applied onto the surface of each well (1cm²). One neonate (L1; first instar) insect larvae was placed on each well and 18-20 larvae were used per sample. Dishes were kept at 25± 1 °C and mortality rates (percentage dead versus living larvae) were recorded after 7 days. As a negative control standard diet and TB was used.

Results (shown In Table 1 below) showed that the cell-free supernatants of strains BTS01099E, BTS00327D and BTS02245J showed toxicity towards *Heliothis virescens, Helicoverpa zea* and *Ostrinia nubilalis* larvae. In addition, the supernatant of strain BTS02245J also showed toxicity towards Spodoptera frugiperda larvae and the supernatant of BTS00327D showed toxicity towards *Agrotis ipsilon* larvae.

**Table 1:**

| Strain | Hv mort (%) | Hz mort (%) | On mort (%) | Sf mort (%) | Ai mort (%) |
|---|---|---|---|---|---|
| BTS02245J | 11 (ir) / 33 (gi) | 94 (gi) / 50 | 50 (gi) | 78 (gi) | nt |
| BTS00327D | 70 | 35-78 (gi) | 100 | nt | 100 |
| IBTS01099E | 25 | 10 | 46 | nt | nt |
| Hv: *Heliothis virescens,* Hz: *Helicoverpa zea;* On: *Ostrinia nubilalis,* Sf: *Spodoptera frugiperda;* Ai: *Agrotis ipsilon;* nt: not tested | | | | | |
| Negative controls (standard diet): | | | | | |
| BTS02245J - Hv 9%, Hz 0%, On 0%, Sf 0% | | | | | |
| BTS00327D - Hv 10%, Hz 6%, On 4%, Ai 0% | | | | | |
| BTS01099E - Hv 10%, Hz 0%, On 0% | | | | | |

### Additional observations:

gi: growth inhibition of larvae (live larvae still in L1/L2 instar stage after 7 days) ir: irregular growth of larvae (a proportion of live larvae in L1/L2 instar stage, a proportion of live larvae in L3/L4 instar stage after 7 days)

Cell-free supernatant of strain BTS02245J caused 11 % and 33% mortality of *H*. *virescens* larvae, 94% and 50% mortality of *H*. *zea* larvae, 50% mortality of *O*. *nubilalis* larvae and 78% mortality of *S*. *frugiperda* larvae, showing that supernatant of this strain has insecticidal activity, particularly against *H. zea* and *S*. *frugiperda.* Toxicity is likely caused by a protein secreted by this strain into the culture medium.

The cell-free supernatant of strain BTS00327D caused 70% mortality of *H*. *virescens* larvae, 35% to 78% mortality of *H*. *zea* larvae, 100% mortality of *O*. *nubilalis* larvae and 100% mortality of *Agrotis ipsilon* larvae. Thus, the supernatant of this strain showed strong toxicity to at least four different species of Lepidopteran insects. Toxicity is likely to be caused by an insecticidally active protein secreted by this strain into the culture medium.

The cell-free supernatant of strain BTS01099E caused 25% mortality of *H. virescens* larvae, 10% mortality of *H. zea* larvae and 46% mortality of *O. nubilalis* larvae, indicating that the supernatant of this strain has toxic activity against different species of Lepidopteran insects. Toxicity is likely to be caused by an insecticidally active protein secreted by this strain into the culture medium.

### Example 3: Cloning of isp3 genes

### Cloning of the reference nucleotide sequence encoding ISP3-1099E from strain BTS01099E

Total DNA of strain BTS01099E was prepared and partially digested with *Sau3A.* The digested DNA was size fractioned on a sucrose gradient. Fragments ranging from 7kb to 10kb were ligated to cloning vector pUC191 (a derivative of pUC19; Yannisch-Perron et al. 1985), after *BamH1* digestion and treatment of the cloning vector with TsAP (thermosensitive alkaline phosphatase). The ligation mixture was then electroporated into *E*. *coli* XL1 -Blue cells. Transformants were plated on LB-triacillin plates containing X-gal and IPTG and white colonies were selected to be used in filter hybridization experiments. Recombinant *E*. *coli* clones containing the vector were then screened with a DIG labeled probe which was prepared as follows. First, a PCR was performed using as template cells from strain BTS01099E. The resulting amplification product was gel-purified and cloned into pGEM-T. The resulting plasmid was used as template in a PCR reaction using DIG- labeled dNTPs and the same primers as in the first PCR reaction. An appropriate amount of this amplification product was used in hybridization reactions.

Following the identification of a positive colony containing a plasmid harboring the full length *isp3* gene, the sequence of this gene was determined using the dye terminator labeling method and a Perkin Elmer ABI Prism-377 DNA sequencer. Both the coding and non-coding strand were sequenced.

The sequence of the open reading frame found in the cloned reference DNA fragment of a positive colony is shown in SEQ ID No. 1 (*isp3-1099E*). This DNA sequence was found to encode the novel reference protein shown in SEQ ID No. 2 (ISP3-1099E).

To show that this DNA sequence is the cause of the insecticidal activity observed, the sequence was expressed in a bacterial strain and the supernatant or cell lysate of the recombinant strain was tested for insecticidal activity in insect bioassays.

### Cloning of the nucleotide sequence encoding ISP3-327D from strain BTS00327D

Total DNA of strain BTS00327D was prepared and partially digested with Sau3A. The digested DNA was size fractioned on a sucrose gradient. Fragments ranging from 7kb to 10kb were ligated to cloning vector pUC19I (a derivative of pUC19; Yannisch-Perron et al. 1985), after *Bam*H1 digestion and treatment of the cloning vector with TsAP (thermosensitive alkaline phosphatase). The ligation mixture was then electroporated into *E. coli* XL1-Blue cells. Transformants were plated on LB-triacillin plates containing X-gal and IPTG and white colonies were selected to be used in filter hybridization experiments. Recombinant *E*. *coli* clones containing the vector were then screened with a DIG labeled probe which was prepared as follows. First, a PCR was performed using as template cells from strain BTS00327D. The resulting amplification product was gel-purified and cloned into pGEM-T. The resulting plasmid was used as template in a PCR reaction using DIG- labeled dNTPs and the same primers as in the first PCR reaction. An appropriate amount of this amplification product was used in hybridization reactions.

Following the identification of a positive colony containing a plasmid harboring the full length *isp3* gene, the sequence of this gene was determined using the dye terminator labeling method and a Perkin Elmer ABI Prism-377 DNA sequencer. Both the coding and non-coding strand were sequenced.

The sequence of the open reading frame found in the cloned DNA fragment of a positive colony is shown in SEQ ID No. 3 *(isp3-327D).* This DNA sequence was found to encode the novel protein shown in SEQ ID No. 4(ISP3-327D).

To show that this DNA sequence is the cause of the insecticidal activity observed, the sequence was expressed in a bacterial strain and the supernatant or cell lysate of the recombinant strain was tested for Insecticidal activity in insect bioassays.

### Cloning of the reference nucleotide sequence encoding ISP3-2245J from strain BTS02245J

Total DNA of strain BTS02245J was prepared and partially digested with *Sau3A.* The digested DNA was size fractioned on a sucrose gradient. Fragments ranging from 7kb to 10kb were ligated to cloning vector pUC191 (a derivative of pUC19; Yannisch-Perron et al. 1985), after *Bam*H1 digestion and treatment of the cloning vector with TsAP (thermosensitive alkaline phosphatase). The ligation mixture was then electroporated into *E. coli* XL1-Blue cells. Transformants were plated on LB-triacillin plates containing X-gal and IPTG and white colonies were selected to be used in filter hybridization experiments. Recombinant *E*. *coli* clones containing the vector were then screened with a DIG labeled probe which was prepared as follows. First, a PCR was performed using as template cells from strain BTS02245J. The resulting amplification product was gel-purified and cloned into pGEM-T. The resulting plasmid was used as template in a PCR reaction using DIG- labeled dNTPs and the same primers as in the first PCR reaction. An appropriate amount of this amplification product was fused in hybridization reactions.

Following the identification of a positive colony containing a plasmid harboring the full length *isp3* gene, the sequence of this gene was determined using the dye terminator labeling method and a Perkin Elmer ABI Prism-377 DNA sequencer. Both the coding and non-coding strand were sequenced.

The sequence of the open reading frame found in the cloned reference DNA fragment of a positive colony is shown in SEQ ID No. 5 (*isp3-2245J*). This DNA sequence was found to encode the novel reference protein shown in SEQ ID No. 6 (ISP3-2245J).

To show that this DNA sequence is the cause of the insecticidal activity observed, the sequence was expressed in a bacterial strain and the supernatant or cell lysate of the recombinant strain was tested for insecticidal activity in insect bioassays.

### Example 4: Recombinant expression of ISP3 proteins in E. coli

Reference DNA of SEQ ID No. 1 (*isp3-9099E*), SEQ ID No. 3 *(isp3-327D)* and reference DNA of SEQ ID No. 5 (*isp3-2245J*) were subcloned into an expression vector, under control of the cry1Ab promoter, and expressed in *E*. *coli* strain WK6. During subcloning, an Ncol restriction site was introduced at the ATG start codon, thereby changing the second amino acid of SEQ ID No 2, SEQ ID No 4 and SEQ ID No. 6 from Asparagine (Asn) into Aspartate (Asp).

SDS-PAG E analysis of transformed *E*. *coli* cell lysates showed that proteins of the expected molecular weight (±88kDa) were produced for each of the three genes. As negative controls cell lysate of non-transformed *E*. *coli* WK6 were used.

Cell lysate of recombinant *E*. *coli* cultures, expressing isp3-327D and isp3-1099E reference protein, was used in insect bioassays (surface contamination assays) as described in Example 5. The results are summarized in Table 2 below. Plus symbols indicate-significant insect mortality over the negative control.

**Table 2:**

| Gene in *E. coli* | Hz | Hv | Sf | Ag | Dw |
|---|---|---|---|---|---|
| *isp3-327D* | + | + | + | + | - |
| *isp3-1099E* | + | + | + | + | - |
| negative control WK6 | - | - | - | - | - |
| Hz: *Helicoverpa zea, Hz: Heliothis virescens,* Sf: *Spodoptera frugiperda,* Dvv: *Diabrotica virgifera virgifera,* Ag: *Anticarsia gemmatalis* | | | | | |
| Bioassays: | | | | | |
| Hz: Surface contamination on heliothis food in 48 multiwell Costar plates, 25µl/well(1cm²), 18x1 L1 perconcentration | | | | | |
| Hv: Surface contamination on heliothis food in 24 multiwell Costar plates, 50ul/well(2cm²), 20x1 L1 per concentration | | | | | |
| Sf: surface contamination on littoralis food in 48 multiwell Costar plates; 25µl/well (1cm²); 18x1 L1 per concentration | | | | | |
| Ag: surface contamination on littoralis food in 24 multiwell Costar plates, 50µl/well(2cm²), 12x2L1 per concentration Incubation at T:25±1 °C; Score after 7 days | | | | | |

For ISP3-327D protein and ISP3-1099F reference protein, significant mortality was found in surface contamination assays with *Helicoverpa zea, Heliothis virescens, Spodoptera frugiperda* and *Anticarsia gemmatalis.* In addition, undiluted cell lysate of recombinant *E. coli* expressing ISP3-327D or ISP3-1099E reference protein showed significant mortality against *Ostrinia nubilalis* (50%(gi) mortality for ISP3-327D, 26% (gi) mortality for ISP3-1099E compared to 0% mortality for the control WK6).

### Example 5: Recombinant expression of ISP3 proteins in Bt

Reference DNA of SEQ ID No. 1 (*isp3*-1099E) and SEQ ID No. 5 were subcloned into a shuttle vector and expressed in a crystal minus strain Bt strain (IPS 78/11 or Berliner 1715cry). In bioassays, supernatant from the non-transformed Bt strain is used as negative control.

The cell-free culture supernatant from the recombinant Bt strain is tested for toxicity against Lepidopteran insect larvae, particularly against *H. virescens, H. zea, H. armigera, O. nubilalis, S. frugiperda, Agrotis ipsilon, Pectinophora gossypiella* and *A. gemmatalis,* using a surface contamination assay as described above (Example 2) and below (to determine LC₅₀ values).

Supernatant from the recombinant Bt strain is obtained as follows.

The Bt strain is grown overnight on LB agar plates containing erythromycin (20 µg/ml) at 28°C. For small scale cultures, 20 ml TB medium containing erythromycin (20 µg/ml) is inoculated and grown for 65 hours at 28°C on a rotating platform having about 70 rotations per minute. After 65 hours, a protease inhibitor mixture is added to the culture. This cocktail has the following ingredients (volumes given are those required to add to one 20 ml culture): 200µl PMSF (100mM), 200µl of a mixture of benzamidine.HCl (100mM) / epsilon-amino-n-caproic acid (500 mM), 400µl EGTA (0.5M), 40µl antipain (0.5 mg/ml) / leupeptin (0.5 mg/ml) and 20µl beta-mercaptoethanol (14M).

The culture medium to which the protease inhibitor mixture had been added, is then centrifuged for 20 minutes at 3000 rpm. In some cases, the supernatant is concentrated about 4 times using centriprep YM-10 Centrifugal Filter Devices (Millipore, Cat. No. 4305).

For *Helicoverpa zea* and *Heliothis virescens* the following artificial diet is used in the surface contamination assay: water 1 liter, agar 20g, soyflour 81 g, wheat germ 36g, Wesson saft mix 10g, sucrose 14.7g, Nipagin 1g, sorbic acid 1.1 g, Vanderzant vit.mix. 9.5g, corn oil 5ml, Nystatin 0.06g, Aureomycin 0.17g.

In the surface contamination assays for other lepidopteran insects, the following artificial diet is used: water 1 liter, agar, 20g, corn flour 112g, wheatgerm 28g, yeast 30g, sorbic acid 1.2g, Nipagin 1g, Aureomycin 0.06g, Nystatin 0.03g, ascorbic acid 4.8g.

The artifical diet is dispensed In wells of Costar 24-multlwell plates and allowed to solidify. 50µl of diluted supernatant is applied onto the surface of each well (2cm²). One or two neonate (L1; first instar) larvae are placed on each well (depending on the species, e.g. for *O. nubilalis* 2 larvae/well) and around 20 to 24 larvae are used per supernatant dilution. Six to eight supernatant dilutions (the dilution factor is around 3), ranging from about 4050 to 0.21 ng/cm² are tested. Dishes are kept at 25±1 °C and mortality rates (percentage dead versus living larvae) are recorded after 7 days. As a negative control standard diet and TB is used. LC₅₀ values and/or LC₉₀ values are calculated with probit analyis using the program POLO PC (from LeOra Software, 1987, Berkely, California). The LC₅₀ value is the total supernatant protein concentration when 50% of the tested insect larvae are killed.

The bioassays show that the proteins encoded by the cloned reference sequences *isp3- 1099E* and *isp3-2245J* each cause significant insecticidal activity against selected Lepidopteran Insects.

SEQ ID No. 3 (*isp3-327D*) was subcloned into a shuttle vector and expressed in the crystal minus Bt strain IPS78/11. In bioassays, supernatant from the non-transformed Bt strain was used as negative control.

SDS-page analysis showed that the culture supernatant contained a protein with a molecular weight close to the calculated molecular weight of the ISP3-327D protein (±88kDa).

Using the surface contamination assay as described in Example 2, the cell-free culture supernatant of Bt strain IPS78/11 expressing SEQ ID No. 3 (*isp3-327D*) showed significant insecticidal activity against *Ostrinia nubilalis* (On), *Pectinophora gossypiella* (Pg) and *Helicoverpa zea (Hz),* as shown in Table 3.

**Table 3:**

| | Hz mort (%) | On mort (%) | Pg mort (%) | Dvv mort(%) |
|---|---|---|---|---|
| *isp3-327D* in IPS 78/11 | 94 (gi) / 58 (gi) | 19 (gi) | 29 (gi) | 0 |
| Control IPS 78/11 | 0 | 6 | 0 | 5 |
| gi: growth inhibition of larvae / Dvv: *Diabrotica virgifera virgifera* | | | | |

### Bioassays:

Surface contamination assays, as described above, using concentrated supernatant after 26hrs culture in TB and following addition of protease inhibitor coctail. Hellothis artificial diet (as above) was used for *H. zea* and littoralis artificial diet for *O. nubilalis* and *P. gossypiella.* For *H. zea* and *O. nubilalis* 48. well Costar plates, 25µl/well (1cm²), 18 wells with one L1 larva per well, were used. For *P. gossypiella* 24 well Costar plates, 50µl/well (2cm²) and 12 wells with two L1 larvae per well were used. Dw artificial diet (as above) was used for Dw in 24 well plates, 50µl/well (2cm²), 6 wells with 4 L1/well.

The bioassay showed that the protein encoded by the cloned sequence *isp3-327D* has significant insecticidal activity against selected Lepidopteran insects.

### Example 6: further characterisation of ISP3-327D

Supernatant from the crystal minus Bt strain IPS78/11 expressing SEQ ID No. 3 (*isp3-327D*) was used to test trypsin digestability of ISP3-327D protein and toxicity of the resulting fragments. Trypsin treatment of supernatant of the transformed IPS78/11 culture resulted in two major bands of about 65kDa and about 23kDa, as determined by SDS-PAGE analysis.

Both trypsin treated supernatant (4 hours treatment; reaction was stopped with PMSF of a final concentration of 0.1 mM) and non-trypsin treated supernatant were used in surface contamination assays against *Helicoverpa zea.* The surface contamination assay was performed on heliothis food in 48 multiwell plates (25µl/well;1 cm²). Six supernatant dilutions were tested. Per dilution 18 wells and one L1 larva per well were used. Mortality was scored after incubation at 25°±I. °C after 7 days.

LC50 values for the untreated and trypsin treated ISP3-327D protein showed overlapping 90% confidence intervals, showing that the trypsin treated protein retains toxic activity against *H. zea.*

### Example 7: Rice Insect bloossays of recombinant ISP3 proteins

The sequences for reference DNA *isp3- 1099E* (SEQ ID No.1), *isp3-327D* (SEQ ID NO.3) and reference DNA *isp3-2245J* (SEQ ID No. 5) were expressed in *E. coli* as described above.

Cell lysates of recombinant *E. coli* were tested in insect bioassays for activity against four Lepidopteran rice pests. Five to six doses per protein were tested.

### (a) Yellow Stem Borer (Scirphophaga incertulas) bioassay:

Yellow Stem Borer adults were collected from rice fields. Eggs laid by the adults were collected and kept in Petri dishes for hatching at 30°C. The newly hatched larvae were used in the bioassays.

Rice stalk sheath of 6 cm were used. Six centimeter long segments of the leaf sheaths were cut from freshly excised rice stalks of the susceptible variety TN1. The inner most part of the segment, along with one sheath cover, were separately dipped into the different protein doses for 30 seconds. The treated stalk sheath was immobilized vertically on 2 cm agar gel in specimen tubes (7cm long; 2.5 cm diameter). 10-15 neonate larvae of Yellow Stem Borer were added to each treated stalk and tubes were sealed and incubated for five days. After 5 days, the numbers of surviving and of dead larvae were counted.

### (b) Leaffolder (Cnaphalocrocis medinalis) bioassay:

Insects were collected from rice fields in northern India. Insect larvae were reared on rice plants in the green house. Emerging adults were confined in oviposition chamber and oviposited plants were kept in water trays for larval hatching. One day old larvae were used in bioassays.

Bioassays were conducted in cylindrical chambers, using freshly excised leaves from rice plants at tillering stage. An 8 cm long leaf lamina was excised from the central whorl of the rice tiller. The leaf lamina was placed in the chamber and the different protein dosages applied. After 30 minutes, five to ten larvae were added per leaf. At least three leaves were used per protein dose. Five days after incubation, the numberof dead and surviving larvae was counted.

### (c) Pink Stem Borer (Sesamia inferens) bioassay:

Insects were collected from rice fields in northern India and reared on rice plants.

Bioassays were conducted in glass vials (7.5cm x 2.5cm diameter) using an artificial diet made of dry bean powder, brewers yeast, sorbic acid, ascorbic acid, methyl paraben, agar and water. The diet was dispensed Into the vials up to 2cm depth. At least three vials were prepared per protein dose. 40µl of test dose was spread uniformly onto the surface of the diet in each vial and left to dry for one hour. Ten to fifteen first instar larvae of Pink Stem Borer were added per vial. After seven days the number of dead and surviving larvae was counted.

### (d) Corn Spotted Stem Boter (Chilo pariellus) bioassays:

Insects were maintained on artificial diet made up of red bean powder, brewers yeast, sorbic acid, sorghum leaf powder, ascorbic acid, methyl para-hydroxy benzoic acid, vitamins, wheat germ oil, Wesson saft mixture, agar, formaldehyde and water. Neonate larvae from these cultures were used in the bioassays. The bioassays were performed as described for Pink Stem Borer (*Sesamia inferens*).

### (e) Results of rice insect bioassays

The results of the Insect bioassays, summarized in table 4 below, showed that ISP3-327D and reference protein ISP3-1099E were highly toxic to four Leptidopteran rice pests, namely Yellow Stem Borer (*Scirphophaga incertulas*), Leaffolder (*Cnaphalocrocis medinalis*), Pink Stem Borer (*Sesamia inferens*) and Com

Spotted Stem Borer (*Chilo partellus*). Further, ISP3-2245J reference protein showed significant toxicity towards three lepidopteran rice pests, namely Yellow Stem Borer (*Scirphophaga incertulas*), Leaffolder (*Cnaphalocrocis medinalis*) and Pink Stem Borer (*Sesamia inferens*), while no toxicity of ISP3-2245J reference protein against Corn Spotted Stem Borer (*Chilo partellus*) was detected.

**Table 4:**

| Gene in *E. coli* | YSB | LF | PSB | SSB |
|---|---|---|---|---|
| *isp3-327D* | + | + | + | + |
| *isp3-1099E* | + | + | + | + |
| *Isp3-2245J* | + | + | + | - |
| negative control | - | - | - | - |
| YSB: Yellow Stem Borer, LF: Leaffolder, PSB: Pink Stem Borer, SSB: Spotted Stem Borer; plus symbols (+) indicate significant mortality over the negative control. | | | | |

### Example 8: Production of ISP3 proteins In transformed plants

Plant expression vectors are constructed comprising a plant-expressible promoter, operably linked to a DNA sequence encoding either reference protein ISP3-1099E, ISP3-327D or reference protein ISP3-2245J (or a toxic fragment or variant thereof), and a 3' polyadenylation signal. A leader sequence, such as that from the chlorophyl a/b binding protein gene from Petunia (Harpster et al. 1988), is inserted 5' of the *isp3* DNA. Preferably, codon-usage of reference DNA *isp3-1099E, isp3-327D* and reference DNA *isp3-2245J* is adapted to that of the host plant (e.g. corn, cotton or rice), for example as described in WO94/24264.

The promoters used to drive the *isp3* genes are selected from the constitutive promoters CaMV 35S (Hull and Howell, 1987), maize ubiquitin promoter, rice actin promoter and Cassava Vein Mosaic Virus promoter. As 3' transcript termination and polyadenylation signal the 3'35S, 3'nos (Depicker et al. 1982), 3'ocs or 3'gene7 are used. For *Agrobacterium* mediated transformation, the expression cassette is inserted into a T-DNA vector, between the right and left border sequence.

Transformation of corn (*Zea mays*), cotton (*Gossypium hirsutum or Gossyplum barbadense*) and rice (*Oryza sativa*) plants.

Corn cells are stably transformed by *Agrobacterium* mediated transformation as described in US 6,140,553. Cotton cells are stably transformed by *Agrobacterium* mediated transformation as described in WO 00/71733. Rice cells are stably transformed as described in WO92/09696.

Transformed cells and plantlets are regenerated as described in the above references. For constructs additionally comprising a selectable marker gene, such as a herbicide resistance gene, for example 2mEPSPS (EPO 508 909 and EP 0 507 698) conferring resistance to glyphosate or the bar or PAT gene conferring resistance to glufosinate ammonium (EP 0 242 236 and EP 0 242 246) transformed cells are grown on selection media containing the selective agent, so that most of the selected regenerants are transformed.

From the regenerants transformants expressing the *isp3* gene are selected by ELISA, Southern blot, Northern blot and/or PCR analysis. Transformation events, particularly single copy events, are then selected for insecticidal activity towards Lepidopteran insect pests (using bioassays). Chimeric *isp3-1099E reference gene isp3-327D* or *isp3-2245J* reference gene-containing progeny plants show improved resistance to lepidopteran insects compared to non-transformed control plants. Particularly plants with high levels of insect tolerance express a high level of ISP3 protein and *isp3* mRNA.

Transformants are further selected for agronomic characteristics (normal phenotype, yield, plant architecture, etc.). Following seed increases, field trials of transformed corn, cotton or rice plants are carried out in different regions where susceptible insect pests are present, demonstrating that plants expressing ISP3 proteins have an increased insect tolerance under field conditions in different environments. Particularly, following infestation (artificial or natural) of the field by insect pests, yield losses of the transformed plants are reduced compared to non-transformed control plants.

To generate plants with broad insecticidal activity, the ISP3 expressing events are crossed to each other and to other transgenic plants, expressing insecticidal proteins with a different, preferably complementary, insecticidal spectrum. Progeny of the crosses are assayed for Insecticidal activity using bioassays for a range of different insect pests. Plants with insecticidal activity against the desired insect range are generated in this way.

This invention is not limited to the above corn, cotton, soybean or rice plants, transformation methods, vector constructs (promoters, 3'ends, etc.) or the particular ISP3 proteins or DNA sequences used. The invention includes variants or equivalents of the ISP3 proteins retaining insecticidal activity.

### REFERENCES

An et al. (1996) Plant J. 10, 107
Aoyama and Chua (1997) Plant Journal 11:605-612
Ausubel et al. (1994) Current Protocols in Molecular Biology, Current Protocols, Vol. 1 and 2, USA.
Bennetzen & Hall (1982) J. Biol. Chem. 257, 3026-3031.
Bernhard, K. and Utz, R. (1993) "Production of Bacillus thuringiensis insecticides for experimental and commercial uses", In Bacillus thuringiensis, An Environmental Biopesticide: Theory and Practice, pp.255-267, eds. Entwistle, P.F., Cory, J.S., Bailey, M.J. and Higgs, S., John Wiley and Sons, New York.
Bih et al. (1999), J. Biol. Chem. 274, 22884-22894.
Brown (1998) Molecular Biology LabFax, Volumes I and II, Second Edition,Academic Press (UK) Callis et. al. (1987) Genes Developm. 1:1183-1200
Christensen et al. (1992) Plant Mol. Biol. 18, 675-689.
Cordera et al. (1994) The Plant Journal 6, 141
Comejo et al. (1993) Plant Mol. Biol. 23, 567-581.
Cornelissen et al. (1986) EMBO J. 5, 37-40.
Datta et al. (1990) Bio/Technology 8, 736-740
De Pater et al. (1992) Plant J. 2, 834-844.
Depicker et al. (1982) J. Molec. Appl. Genetics 1, 561-573.
Dieffenbach and Dveksler (1995) PCR Primer. A Laboratory Manual, Cold Spring Harbor Laboratory Press Doss et al (2002) Protein Expression and Purification 26, 82-88
Dulmage, H.T. (1981) "Production of Bacteria for Biological Control of Insects" in Biological Control in Crop Production, Ed. Paparizas, D.C., Osmun Publishers, Totowa, N.J., USA, pp. 129-141.
Estruch et al. (1996) Proc Natl Acad Sci USA 93, 5389-94.
Franck et al. (1980) Cell 21, 285-294
French et al. (1986) Anal.Biochem. 156, 417-423
French-Constant and Bowen (2000) Cell Mol Life Sci 57, 828-33.
Fromm et al. (1990) Bio/Technology 8, 833-839
Gardner et al. (1981) Nucleic Acids Research 9, 2871-2887
Ge et al. (1991) J. Biol. Chem. 266, 17954-17958
Gielen et al. (1984) EMBO J 3, 835-845
Gill et al. (1992) Ann. Rev. Entomol. 37: 615-636
Gordon-Kamm et al. (1990) The Plant Cell 2, 603-618
Gould et al. (1991) Plant Physiol. 95, 426-434
Haider et al. (1986) Europ J Biochem 156:531 -540
Harlow and Lane (1988) Antibodies: A Manual Laboratory, Cold Spring Harbor Lab Press NY
Harpster et al.(1988), Molecular and General Genetics 212, 182-190
Henikoff and Henikoff (1992) Proc. Natl. Academy Science 89 (10):915-919
Hesse et al. (1989), EMBO J. 8 2453-2461.
Ho et al.(1989). Gene 77, 51-59.
Hofmann et al. (1988) PNAS 85:7844-7848
Höfte et al. (1988) Appl. and Environm. Microbiol. 54, 2010-2017
Hull and Howell (1987) Virology 86, 482-493
Ikemura, 1993, In "Plant Molecular Biology Labfax", Croy, ed., Bios Scientific Publishers Ltd.
Itakura et al.(1977). Science 198, 1056-1063.
Keil et al. (1986), Nucl. Acids Res. 14, 5641-5650.
Klösgen et al. (1989), Mol. Gen. Genet. 217, 155-161.
Klösgen and Weil (1991), Mol. Gen. Genet. 225, 297-304.
Kota et al. (1999) Proc. Natl. Acad. Sci. USA 96, 1840-1845.
Last et al. (1990) Theor. Appl. Genet. 81, 581-588.
Mahillon et al. (1989), FEMS Microbiol. Letters 60, 205-210.
Maxam and Gilbert (1980) Methods in Enzymol. 65, 499-560.
McBride et al.(1995) BIo/Technology 13, 362
McPherson et al. (2000) PCR - Basics: From Background to Bench, First Edition, Springer Verlag, Germany
Morris et al. (1999), Biochem. Biophys. Res. Commun. 255, 328-333.
Murray et al. (1989) Nucleic Acids Research 17(2), 477-498.
Nakamura et al. (2000) Nucl. Acids Res. 28, 292.
Needleman and Wunsch algorithm (1970) J. Mol. Biol. 48: 443-453
Neuhaus & Rogers (1998) Plant Mol. Biol. 38, 127-144.
Nielsen, H. J. Engelbrecht, S. Brunak, and G. von Heijne (1998) A neural network method for identification of prokaryotic and eukaryotic signal peptides and prediction of their cleavage sites
Odell et al. (1985) Nature 313, 810-812.
Oelmuller et al.(1993) Mol. Gen. Genet. 237, 261-272.
Park et al. (1997) J. Biol. Chem. 272, 6876-6881.
R.D.D. Croy (1993) Plant Molecular Biology Labfax jointly published by BIOS Scientific Publications Ltd (UK) and Blackwell Scientific Publications, UK.
Sambrook et al. (1989) Molecular Cloning: A Laboratory Manual, Second Edition, Cold Spring Harbor Laboratory Press, NY
Sambrook and Russell (2001) MolecularCloning: A Laboratory Manual, Third Edition, Cold Spring Harbor Laboratory Press, NY
Sanger et al.(1977) Proc. Natl. Acad. Sci. USA. 74(12), 5463-5467.
Schnepf and Whiteley (1981) Proc. Natl. Acad. Sci. USA 87: 2893-2897
Selvapandiyan et al. (2001) Appl. and Environm. Microbiol 67 (12). 5855-5858
Shcherban et al. (1995), Proc. Natl. Acad. Sci USA 92, 9245-9249.
Shimamoto et al (1989) Nature 338, 274-276
Smith and Waterman (1981)-Advances in Applied Mathematics 2: 482-489
Stanssens et al.(1989) Nucleic Acids Research 12, 4441-4454.
Sutliff et al. (1991) Plant Molec. Biol. 16, 579-591.
Tavladoraki et al. (1998), FEBS Lett. 426, 62-66.
Terashima et al. (1999), Appl. Microbiol. Biotechnol. 52, 516-523.
Vaeck et al. (1987) Nature 328, 33-37.
Van Den Broeck et al. (1985) Nature 313, 358.
Van Rie et al. (1990) Science 247, 72.
Vanderzand (1962) J. Econ. Entomol. 55, 140
Velten et al. (1984) EMBO J 3, 2723-2730
Velten and Schell (1985), Nucleic Acids Research 13, 6981-6998
Verdaguer et al. (1998), Plant Mol. Biol. 37, 1055-1067
Visser et al. (1993) "Domain-Structure Studies of Bacillus thuringiensis Crystal Proteins: A Genetic Approach", In Bacillus thuringlensis, An Environmental Blopesticide: Theory and Practice, pp.71-88, eds. Entwistle, P.F., Cory, J.S., Bailey, M.J. and Higgs, S., John Wiley and Sons, New York.
Von Heijne, Gunnar (1986) Secretory signal peptide identification algorithm described by Gunnarvon Heijne Nucleic Acids Research. 14:11, 4683-4690
Wada et al. (1990). Nucl. Acids Res. 18, 2367-1411.
Warren (1997) "Advances in Insect Control: The role of transgenic plants", 1997, editors Carozzi and Koziel, p109-121, Taylor and Francis London, UK
Waterfield et al.(2001) Trends Microbiol 9, 185-91.
White et al.(1989). Trends in Genet. 5, 185-189
Wilbur and Lipmann (1983) Proc. Nat. Acad. Sci. USA 80: 726
Wong et al.(1992), Plant Molec. Biol.20, 81-93.
Yannisch-Perron et al. (1985) Gene 33, 103-119
Yu et al. (1997) Applied and Environmental Microbioloby 532-536
Zhang et al. (1991) The Plant Cell 3, 1155-1165.

### SEQUENCE LISTING

<110> Bayer BioScience N.V.
<120> Novel Bacillus thuringiensis insecticidal proteins
<130> isp3
<150> US 60/366276
   <151> 2002-03-22
<150> US 60/423999
   <151> 2002-11-06
<160> 6
<170> Patent In version 3.0
<210> 1
   <211> 2364
   <212> DNA
   <213> artificial
<220>
   <223> nucleotide sequence of Isp3-1099E of Bacillus thuringiensis
<400> 1
   atgaacatga ataatactaa attaaacgca agggccttac caagttttat tgattatttt      60
   aatggcattt atggatttgc cactggtatc aaagacatta tgaatatgat tttaaaaacg     120
   gatacaggtg gtaatctaac cctagacgaa atcttaaaga atcagcagtt actaaatgag     180
   atttctggta aattggatgg ggtaaatggg agcttaaatg atcttatcgc acagggaaac 240
   ttaaatacag aattatctaa ggaaatctta aaaattgcaa atgaacagaa tcaagtctta 300
   aatgatgtta ataacaaact cgatgcgata aatacgatgc ttcatatata tctacctaaa 360
   attacatcta tgttaagtga tgtaatgaag caaaattatg cgctaagcct gcaaatagaa 420
   tacttaagta aacaattgca agaaatttcc gataagttag atattattaa cgtaaatgtt 480
   cttattaact ctacacttac tgaaattaca cctgcatatc aacggattaa atatgtgaat 540
   gaaaaatttg aagaattaac ttttgctaca gaaaccactc taaaagtaaa agaggatgga 600
   tcccctgcag atattcttga tgagttaact gagttaactg aattagcgaa aagtgtaaca 660
   aaaaatgaag tggatggttt tgaattttac cttaatacat tccacgatgt aatggtagga 720
   aataatttat tcgggcgttc agctttaaaa actgcttcgg aattaattgc taaagaaaat 780
   gtgaaaacaa gtggcagtga ggtaggaaat gtttataatt tcttaattgt attaacagct 840
   ctacaagcaa aagcttttct tactttaaca acatgccgga aattattagg attagcagat 900
   attgattata cttctattat gaatgaacat ttaaataagg aaaaagagga atttagagta 960
   aacatccttc ctacactttc taatactttt tctaatccta attatgtaaa agctaaagga 1020
   agcgatagag atgcaaagat gattgtggaa gctaaaccag gatatgcttt ggttggattt 1080
   gaaatgagta atgattcaat gacagtatta aaagcatatc aggccaagct aaagcaagat 1140
   tatcaagttg ataaggattc attatcagaa attgtctatg gtgatatgaa taagttatta 1200
   tgtccggatc aatctgaaca aatatattat acaaataata tagcatttcc ccgtgaatat 1260
   gttattacta aacttacttt tacaaaaaaa atgaacagtt taaggtatga ggcgacagct 1320
   aatttttatg attcttctac aggagatatg gatctaaata agacaaaagt agaatcaagt 1380
   gaagcggagt atagtaggct aagtgctagt aatgatggag tctatatgcc gttaggtctt 1440
   atcagtgaaa catttttgac tccaattaat ggttttggac tcgtagtcga tgaaaattca 1500
   agattagtaa ctttaacatg taaatcatat ttaagagaga tattattagc aacagactta 1560
   agtaataaag aaactaaatt gattgtccca cctaatggtt ttattagcaa tattgtagaa 1620
   aatgggaact tagagggaga aaacttagag ccgtggaaag caaataacaa aaatgcgtat 1680
   atagatcata caggcggcgt aaaaggaact aaagtattat atgttcataa ggatggagag 1740
   ttctcacaat ttattgggta taaattgaaa tcgaaaacag aatatgtaat tcaatatatt 1800
   gtaaaaggaa aagctgttat ttatttaaaa gatgaaaaaa atggggatta catttatgaa 1860
   gaaataaata atgaattaga agattttcaa acggttacta aacgttttat tacaggaaca 1920
   gattcttcag gagttcattt aatttttacc agtcaaaatg gcgaggaagc attcgggggg 1980
   aactttatta tttcagaaat taggccatcc gaagagttat taagtccaga attgattaag 2040
   tcggatgctt gggttggaac tcagggagct tggaattcag ggaattctct cacaatttat 2100
   actaatacaa atggaacctt tcgacaaaac cttccgttag aaagttattc aacttatagt 2160
   atgaacttta atataactgg atttggcaag gtgacaataa ggaattctcg tgaagtatta 2220
   tttgaaaaga acttttccca gctttcgcct aaagattatt ctgaaaaatt tacaactgca 2280
   gccaataata ccggattcta tgtagagctt tctcgcggaa cgcagggtgg taatataact 2340
   ttccgagatt tttcaattaa gtaa 2364
<210> 2
   <211> 787
   <212> PRT
   <213> artificial
<220>
   <223> amino acid sequence of ISP3-1099E of Bacillus thuringiensis
<400> 2
   Met Asn Met Asn Asn Thr Lys Leu Asn Ala Arg Ala Leu Pro Ser Phe
   1 5 10 15
   Ile Asp Tyr Phe Asn Gly Ile Tyr Gly Phe Ala Thr Gly Ile Lys Asp
   20 25 30
   Ile Met Asn Met Ile Phe Lys Thr Asp Thr Gly Gly Asn Leu Thr Leu
   35 40 45
   Asp Glu Ile Leu Lys Asn Gln Gln Leu Leu Asn Glu Ile Ser Gly Lys
   50 55 60
   Leu Asp Gly Val Asn Gly Ser Leu Asn Asp Leu Ile Ala Gln Gly Asn
   65 70 75 80
   Leu Asn Thr Glu Leu Ser Lys Glu Ile Leu Lys Ile Ala Asn Glu Gln
   85 90 95
   Asn Gln Val Leu Asn Asp Val Asn Asn Lys Leu Asp Ala Ile Asn Thr
   100 105 110
   Met Leu His Ile Tyr Leu Pro Lys Ile Thr Ser Met Leu Ser Asp Val
   115 120 125
   Met Lys Gln Asn Tyr Ala Leu Ser Leu Gln Ile Glu Tyr Leu Ser Lys
       130                 135                 140
   Gln Leu Gln Glu Ile Ser Asp Lys Leu Asp Ile Ile Asn Val Asn Val
   145                 150                 155                 160
   Leu Ile Asn Ser Thr Leu Thr Glu Ile Thr Pro Ala Tyr Gln Arg Ile
                   165                 170                 175
   Lys Tyr Val Asn Glu Lys Phe Glu Glu Leu Thr Phe Ala Thr Glu Thr
               180                 185                 190
   Thr Leu Lys Val Lys Glu Asp Gly Ser Pro Ala Asp Ile Leu Asp Glu
           195                 200                 205
   Leu Thr Glu Leu Thr Glu Leu Ala Lys Ser Val Thr Lys Asn Glu Val
       210                 215                 220
   Asp Gly Phe Glu Phe Tyr Leu Asn Thr Phe His Asp Val Met Val Gly
   225                 230                 235                 240
   Asn Asn Leu Phe Gly Arg Ser Ala Leu Lys Thr Ala Ser Glu Leu Ile
                   245                 250                 255
   Ala Lys Glu Asn Val Lys Thr Ser Gly Ser Glu Val Gly Asn Val Tyr
               260                 265                 270
   Asn Phe Leu Ile Val Leu Thr Ala Leu Gln Ala Lys Ala Phe Leu Thr
           275                 280                 285
   Leu Thr Thr Cys Arg Lys Leu Leu Gly Leu Ala Asp Ile Asp Tyr Thr
       290                 295                 300
   Ser Ile Met Asn Glu His Leu Asn Lys Glu Lys Glu Glu Phe Arg Val
   305                 310                 315                 320
   Asn Ile Leu Pro Thr Leu Ser Asn Thr Phe Ser Asn Pro Asn Tyr Val
                   325                 330                 335
   Lys Ala Lys Gly Ser Asp Arg Asp Ala Lys Met Ile Val Glu Ala Lys
               340                 345                 350
   Pro Gly Tyr Ala Leu Val Gly Phe Glu Met Ser Asn Asp Ser Met Thr
           355                 360                 365
   Val Leu Lys Ala Tyr Gln Ala Lys Leu Lys Gln Asp Tyr Gln Val Asp
       370                 375                 380
   Lys Asp Ser Leu Ser Glu Ile Val Tyr Gly Asp Met Asn Lys Leu Leu
   385                 390                 395                 400
   Cys Pro Asp Gln Ser Glu Gln Ile Tyr Tyr Thr Asn Asn Ile Ala Phe
                   405                 410                 415
   Pro Arg Glu Tyr Val Ile Thr Lys Leu Thr Phe Thr Lys Lys Met Asn
               420                 425                 430
   Ser Leu Arg Tyr Glu Ala Thr Ala Asn Phe Tyr Asp Ser Ser Thr Gly
   435 440 445
   Asp Met Asp Leu Asn Lys Thr Lys Val Glu Ser Ser Glu Ala Glu Tyr
   450 455 460
   Ser Arg Leu Ser Ala Ser Asn Asp Gly Val Tyr Met Pro Leu Gly Leu
   465 470 475 480
   Ile Ser Glu Thr Phe Leu Thr Pro Ile Asn Gly Phe Gly Leu Val Val
   485 490 495
   Asp Glu Asn Ser Arg Leu Val Thr Leu Thr Cys Lys Ser Tyr Leu Arg
   500 505 510
   Glu Ile Leu Leu Ala Thr Asp Leu Ser Asn Lys Glu Thr Lys Leu Ile
   515 520 525
   Val Pro Pro Asn Gly Phe Ile Ser Asn Ile Val Glu Asn Gly Asn Leu
   530 535 540
   Glu Gly Glu Asn Leu Glu Pro Trp Lys Ala Asn Asn Lys Asn Ala Tyr
   545 550 555 560
   Ile Asp His Thr Gly Gly Val Lys Gly Thr Lys Val Leu Tyr Val His
   565 570 575
   Lys Asp Gly Glu Phe Ser Gln Phe Ile Gly Tyr Lys Leu Lys Ser Lys
   580 585 590
   Thr Glu Tyr Val Ile Gln Tyr Ile Val Lys Gly Lys Ala Val Ile Tyr
   595 600 605
   Leu Lys Asp Glu Lys Asn Gly Asp Tyr Ile Tyr Glu Glu Ile Asn Asn
   610 615 620
   Glu Leu Glu Asp Phe Gln Thr Val Thr Lys Arg Phe Ile Thr Gly Thr
   625 630 635 640
   Asp Ser Ser Gly Val His Leu Ile Phe Thr Ser Gln Asn Gly Glu Glu
   645 650 655
   Ala Phe Gly Gly Asn Phe Ile Ile Ser Glu Ile Arg Pro Ser Glu Glu
   660 665 670
   Leu Leu Ser Pro Glu Leu Ile Lys Ser Asp Ala Trp Val Gly Thr Gln
   675 680 685
   Gly Ala Trp Asn Ser Gly Asn Ser Leu Thr Ile Tyr Thr Asn Thr Asn
   690 695 700
   Gly Thr Phe Arg Gln Asn Leu Pro Leu Glu Ser Tyr Ser Thr Tyr Ser
   705 710 715 720
   Met Asn Phe Asn Ile Thr Gly Phe Gly Lys Val Thr Ile Arg Asn Ser
   725 730 735
   Arg Glu Val Leu Phe Glu Lys Asn Phe Ser Gln Leu Ser Pro Lys Asp
   740 745 750
   Tyr Ser Glu Lys Phe Thr Thr Ala Ala Asn Asn Thr Gly Phe Tyr Val
   755 760 765
   Glu Leu Ser Arg Gly Thr Gln Gly Gly Asn Ile Thr Phe Arg Asp Phe
   770 775 780
   Ser Ile Lys
   785
<210> 3
   <211> 2367
   <212> DNA
   <213> artificial
<220>
   <223> nucleotide sequence of isp3-327D of Bacillus thuringiensis
<400> 3
   atgaatatga ataatactaa attaaacgca agggccctac cgagttttat tgattatttt     60
   aatggcattt atggatttgc cactggtatc aaagacatta tgaatatgat ttttaaaacg    120
   gatacaggtg gtaatctaac cttagacgaa atcctaaaga atcagcagtt actaaatgag    180
   atttctggta aattggatgg ggtaaatggg agcttaaatg atcttatcgc acagggaaac    240
   ttaaatacag aattatctaa ggaaatctta aaaattgcaa atgaacagaa tcaagtctta    300
   aatgatgtta ataacaaact cgatgcgata aatacgatgc ttcatatata tctacctaaa    360
   attacatcta tgttaagtga tgtaatgaag caaaattatg cgctaagtct gcaaatagaa    420
   tacttaagta agcaattgca agaaatttct gataaattag atattattaa cgtaaatgtt    480
   cttattaact ctacacttac tgaaattaca cctgcatatc aacggattaa atatgtgaat    540
   gaaaaatttg aagaattaac ttttgctaca gaaaccactt taaaagtaaa aaaggatagc    600
   tcgcctgctg atattcttga tgagttaact gaattaactg aactagcgaa aagtgttaca    660
   aaaaatgacg ttgatggttt tgaattttac cttaatacat tccacgatgt aatggtagga    720
   aataatttat tcgggcgttc agctttaaaa actgcttcag aattaattgc taaagaaaat    780
   gtgaaaacaa gtggcagtga agtaggaaat gtttataatt tcttaattgt attaacagct    840
   ctacaagcaa aagcttttct tactttaaca acatgccgaa aattattagg cttagcagat    900
   attgattata cttctattat gaatgaacat ttaaataagg aaaaagagga atttagagta    960
   aacatccttc ctacactttc taatactttt tctaatccta attatgcaaa agttaaagga   1020
   agtgatgaag atgcaaagat gattgtggaa gctaaaccag gacatgcatt ggttgggttt   1080
   gaaatgagca atgattcaat cacagtatta aaagtatatg aggctaagct aaaacaaaat   1140
   tatcaagttg ataaggattc cttatcggag gttatttatg gtgatacgga taaattattt   1200
   tgtccagatc aatctgaaca aatatattat acaaataaca tagtattccc aaatgaatat   1260
   gtaattacta aaattgattt cactaaaaaa atgaaaactt taagatatga ggtaacagcg   1320
   aatttttatg attcttctac aggagaaatt gacttaaata agaaaaaagt agaatcaagt   1380
   gaagcggagt atagaacgtt aagtgctaat gatgatggag tgtatatgcc attaggtgtc   1440
   atcagtgaaa catttttgac tccgataaat gggtttggcc tccaagctga tgaaaattca   1500
   agattaatta ctttaacatg taaatcatat ttaagagaac tactgctagc aacagactta   1560
   agcaataaag aaactaaatt gatcgtccca ccaagtggtt ttattagcaa tattgtagag   1620
   aacgggtcca tagaagagga caatttagag ccgtggaaag caaataataa gaatgcgtat   1680
   gtagatcata caggcggagt gaatggaact aaagctttat atgttcataa ggacggagga   1740
   ttttcacaat ttattggaga taagttaaaa ccgaaaactg agtatgtaat ccaatatact   1800
   gttaaaggaa aaccttctat tcatttaaaa gatgaaaata ctggatatat tcattatgaa   1860
   gatacaaata ataatttaaa agattatcaa actattacta aacgttttac tacaggaact   1920
   gatttaaagg gagtgtattt aattttaaaa agtcaaaatg gagatgaagc ttggggagat   1980
   aaatttacaa ttttagaaat taagcctgcg gaggatttat taagcccaga attaattaat   2040
   ccgaattctt ggattacgac tccaggggct agcatttcag gaaataaact tttcattaac   2100
   ttggggacaa atgggacctt tagacaaagt ctttcattaa acagttattc aacttatagt   2160
   ataagcttta ctgcatcagg accatttaat gtgacggtaa gaaattctag ggaagtatta   2220
   tttgaacgaa gcaaccttat gtcttcaact agtcatattt ctgggacatt caaaactgaa   2280
   tccaataata ccggattata tgtagaactt tcccgtcgct ctggtggtgg tggtcatata   2340
   tcatttgaaa acgtttctat taaataa                                        2367
<210> 4
   <211> 788
   <212> PRT
   <213> artificial
<220>
   <223> amino acid sequence of ISP3-327D of Bacillus thuringiensis
<400> 4
   Met Asn Met Asn Asn Thr Lys Leu Asn Ala Arg Ala Leu Pro Ser Phe
   1 5 10 15
   Ile Asp Tyr Phe Asn Gly Ile Tyr Gly Phe Ala Thr Gly Ile Lys Asp
   20 25 30
   Ile Met Asn Met Ile Phe Lys Thr Asp Thr Gly Gly Asn Leu Thr Leu
   35 40 45
   Asp Glu Ile Leu Lys Asn Gln Gln Leu Leu Asn Glu Ile Ser Gly Lys
   50 55 60
   Leu Asp Gly Val Asn Gly Ser Leu Asn Asp Leu Ile Ala Gln Gly Asn
   65 70 75 80
   Leu Asn Thr Glu Leu Ser Lys Glu Ile Leu Lys Ile Ala Asn Glu Gln
   85 90 95
   Asn Gln Val Leu Asn Asp Val Asn Asn Lys Leu Asp Ala Ile Asn Thr
   100 105 110
   Met Leu His Ile Tyr Leu Pro Lys Ile Thr Ser Met Leu Ser Asp Val
   115 120 125
   Met Lys Gln Asn Tyr Ala Leu Ser Leu Gln Ile Glu Tyr Leu Ser Lys
   130 135 140
   Gln Leu Gln Glu Ile Ser Asp Lys Leu Asp Ile Ile Asn Val Asn Val
   145 150 155 160
   Leu Ile Asn Ser Thr Leu Thr Glu Ile Thr Pro Ala Tyr Gln Arg Ile
   165 170 175
   Lys Tyr Val Asn Glu Lys Phe Glu Glu Leu Thr Phe Ala Thr Glu Thr
   180 185 190
   Thr Leu Lys Val Lys Lys Asp Ser Ser Pro Ala Asp Ile Leu Asp Glu
   195 200 205
   Leu Thr Glu Leu Thr Glu Leu Ala Lys Ser Val Thr Lys Asn Asp Val
   210 215 220
   Asp Gly Phe Glu Phe Tyr Leu Asn Thr Phe His Asp Val Met Val Gly
   225 230 235 240
   Asn Asn Leu Phe Gly Arg Ser Ala Leu Lys Thr Ala Ser Glu Leu Ile
   245 250 255
   Ala Lys Glu Asn Val Lys Thr Ser Gly Ser Glu Val Gly Asn Val Tyr
   260 265 270
   Asn Phe Leu Ile Val Leu Thr Ala Leu Gln Ala Lys Ala Phe Leu Thr
   275 280 285
   Leu Thr Thr Cys Arg Lys Leu Leu Gly Leu Ala Asp Ile Asp Tyr Thr
   290 295 300
   Ser Ile Met Asn Glu His Leu Asn Lys Glu Lys Glu Glu Phe Arg Val
   305 310 315 320
   Asn Ile Leu Pro Thr Leu Ser Asn Thr Phe Ser Asn Pro Asn Tyr Ala
   325 330 335
   Lys Val Lys Gly Ser Asp Glu Asp Ala Lys Met Ile Val Glu Ala Lys
   340 345 350
   Pro Gly His Ala Leu Val Gly Phe Glu Met Ser Asn Asp Ser Ile Thr
   355 360 365
   Val Leu Lys Val Tyr Glu Ala Lys Leu Lys Gln Asn Tyr Gln Val Asp
   370 375 380
   Lys Asp Ser Leu Ser Glu Val Ile Tyr Gly Asp Thr Asp Lys Leu Phe
   385 390 395 400
   Cys Pro Asp Gln Ser Glu Gln Ile Tyr Tyr Thr Asn Asn Ile Val Phe
   405 410 415
   Pro Asn Glu Tyr Val Ile Thr Lys Ile Asp Phe Thr Lys Lys Met Lys
   420 425 430
   Thr Leu Arg Tyr Glu Val Thr Ala Asn Phe Tyr Asp Ser Ser Thr Gly
   435 440 445
   Glu Ile Asp Leu Asn Lys Lys Lys Val Glu Ser Ser Glu Ala Glu Tyr
   450 455 460
   Arg Thr Leu Ser Ala Asn Asp Asp Gly Val Tyr Met Pro Leu Gly Val
   465 470 475 480
   Ile Ser Glu Thr Phe Leu Thr Pro Ile Asn Gly Phe Gly Leu Gln Ala
   485 490 495
   Asp Glu Asn Ser Arg Leu Ile Thr Leu Thr Cys Lys Ser Tyr Leu Arg
   500 505 510
   Glu Leu Leu Leu Ala Thr Asp Leu Ser Asn Lys Glu Thr Lys Leu Ile
   515 520 525
   Val Pro Pro Ser Gly Phe Ile Ser Asn Ile Val Glu Asn Gly Ser Ile
   530 535 540
   Glu Glu Asp Asn Leu Glu Pro Trp Lys Ala Asn Asn Lys Asn Ala Tyr
   545 550 555 560
   Val Asp His Thr Gly Gly Val Asn Gly Thr Lys Ala Leu Tyr Val His
   565 570 575
   Lys Asp Gly Gly Phe Ser Gln Phe Ile Gly Asp Lys Leu Lys Pro Lys
   580 585 590
   Thr Glu Tyr Val Ile Gln Tyr Thr Val Lys Gly Lys Pro Ser Ile His
   595 600 605
   Leu Lys Asp Glu Asn Thr Gly Tyr Ile His Tyr Glu Asp Thr Asn Asn
   610 615 620
   Asn Leu Lys Asp Tyr Gln Thr Ile Thr Lys Arg Phe Thr Thr Gly Thr
   625 630 635 640
   Asp Leu Lys Gly Val Tyr Leu Ile Leu Lys Ser Gln Asn Gly Asp Glu
   645 650 655
   Ala Trp Gly Asp Lys Phe Thr Ile Leu Glu Ile Lys Pro Ala Glu Asp
   660 665 670
   Leu Leu Ser Pro Glu Leu Ile Asn Pro Asn Ser Trp Ile Thr Thr Pro
   675 680 685
   Gly Ala Ser Ile Ser Gly Asn Lys Leu Phe Ile Asn Leu Gly Thr Asn
   690 695 700
   Gly Thr Phe Arg Gln Ser Leu Ser Leu Asn Ser Tyr Ser Thr Tyr Ser
   705 710 715 720
   Ile Ser Phe Thr Ala Ser Gly Pro Phe Asn Val Thr Val Arg Asn Ser
   725 730 735
   Arg Glu Val Leu Phe Glu Arg Ser Asn Leu Met Ser Ser Thr Ser His
   740 745 750
   Ile Ser Gly Thr Phe Lys Thr Glu Ser Asn Asn Thr Gly Leu Tyr Val
   755 760 765
   Glu Leu Ser Arg Arg Ser Gly Gly Gly Gly His Ile Ser Phe Glu Asn
   770 775 780
   Val Ser Ile Lys
   785
<210> 5
   <211> 2361
   <212> DNA
   <213> artificial
<220>
   <223> nucleotide sequence isp3-2245J of Bacillus thuringiensis
<400> 5
   atgaacatga ataatgctaa attaaatgca agggccttac caagttttat tgattatttt 60
   aatggtattt atggatttgc cactggtatt aaagacatta tgaatatgat ttttaaaacg 120
   gatacaggtt ctaatctaac cctagacgaa attttaaaga atcagcagtt actaaatgaa 180
   atttctggta aattggatgg ggtaaatggg agcttaaatg atcttatcgc acagggaaac 240
   ttaaatacag aattagctaa gcaaatctta aaagttgcaa atgaacaaaa tcaagtttta 300
   aatgatgtta ataacaaact agatgcgata aattcgatgc ttaaaatata tctacctaaa 360
   attacatcta tgttaagtga tgtaatgaag caaaattatg tgctaagctt gcaaatagaa 420
   tacttaagta aacaattgca agaaatctcc gacaagctag atattattaa cgtaaatgtg 480
   cttattaact ctacgcttac tgaaattaca cctgcgtatc aacgaatgaa atatgtgaat 540
   gaaaaatttg aagaattaac ttttgctaca gaaaccactt taaaagtaaa aaaggatagc 600
   cctcctgctg atattcttga cgaattaact gaattaactg aactagcgaa aagtgttaca 660
   aaaaatgacg tggatggttt tgaattttac cttaatacat tccacgatgt aatggtagga 720
   aataatttat tcggtcgttc agctttaaaa actgcttcgg aattaattgc taaagaaaat 780
   gtgaaaacaa gtggcagtga agtaggaaac gtttataatt tcttaattgt attaacagct 840
   ctacaagcaa aagcttttct tactttaaca acatgccgaa aattattagg cttagcagat 900
   attgattata cttctatcat gaatgagcat ttaaataagg aaaaagaaga atttagagta 960
   aacatccttc ccacactttc taataccttt tctaatccta attatgcaaa agctaaggga 1020
   agtaatgaag atacaaagat gattgtggaa gctaaaccag gatatgtttt ggttggattt 1080
   gaaatgagca atgattcaat tacagtatta aaagcatatc aagctaagct aaaaaaagat 1140
   tatcaaattg ataaggattc gttatcagaa ataatatata gtgatacgga taaattatta 1200
   tgtccggatc aatctgaaca aatatattat acaaagaaca tagcatttcc aaatgaatat 1260
   gttattacta aaattgcttt tacaaaaaaa atgaacagtt taaggtatga ggcgacagcg 1320
   aatttttatg attcttctac aggggatatt gatctaaata agacaaaagt agaatcaagt 1380
   gaagcggagt atagtatgct aaaagctagt gatgatgaag tttacatgcc gctaggtctt 1440
   atcagtgaaa catttttgaa tccaattaat ggatttaggc ttgcagtcga tgaaaattcc 1500
   agactagtaa ctttaacatg tagatcatat ttaagagaga cattgttagc gacagattta 1560
   aataataaag aaactaaatt gattgtccca cctaatgttt ttattagcaa tattgtagag 1620
   aatggaaata tagaaatgga caccttagaa ccatggaagg caaataatga gaatgcgaat 1680
   gtagattatt caggcggagt gaatggaact agagccttat atgttcataa ggatggtgaa 1740
   ttctcacatt ttattggaga caagttgaaa tctaaaacag aatacttgat tcgatatatt 1800
   gtaaaaggaa aagcttctat ttttttaaaa gatgaaaaaa atgaaaatta catttatgag 1860
   gatacaaata ataatttaga agattatcaa actattacta aacgttttac tacaggaact 1920
   gattcgacag gagtttattt aatttttaat agtcaaaatg gagatgaagc ttggggagat 1980
   aactttatta ttttggaaat tagtccgtgt gaaaagttat taagtccaga attaattaaa 2040
   acagataaat ggaatagtac gggatcaact tatattagcg atgatagact cactctttat 2100
   cggggaggac gaggaatttt aaagcaaaac cttcaattag acggtttttc aacttataga 2160
   gtcaattttt ctgtggacgg agatgctaat gtaaggattc gtaattctag ggaagtgtta 2220
   cttgaaaaaa gatatttgaa ccgtaaaggt gtttctgaaa tgtttactac aaaatttgat 2280
   aaagataact tttatgtaga gctttctcaa ggggataatc ttggtactat tgtacatttt 2340
   tatgatttct ctattaaata a 2361
<210> 6
   <211> 786
   <212> PRT
   <213> artificial
<220>
   <223> amino acid sequence of ISP3-2245J of Bacillus thuringiensis
<400> 6
   Met Asn Met Asn Asn Ala Lys Leu Asn Ala Arg Ala Leu Pro Ser Phe
   1 5 10 15
   Ile Asp Tyr Phe Asn Gly Ile Tyr Gly Phe Ala Thr Gly Ile Lys Asp
   20 25 30
   Ile Met Asn Met Ile Phe Lys Thr Asp Thr Gly Ser Asn Leu Thr Leu
   35 40 45
   Asp Glu Ile Leu Lys Asn Gln Gln Leu Leu Asn Glu Ile Ser Gly Lys
   50 55 60
   Leu Asp Gly Val Asn Gly Ser Leu Asn Asp Leu Ile Ala Gln Gly Asn
   65 70 75 80
   Leu Asn Thr Glu Leu Ala Lys Gln Ile Leu Lys Val Ala Asn Glu Gln
   85 90 95
   Asn Gln Val Leu Asn Asp Val Asn Asn Lys Leu Asp Ala Ile Asn Ser
   100 105 110
   Met Leu Lys Ile Tyr Leu Pro Lys Ile Thr Ser Met Leu Ser Asp Val
   115 120 125
   Met Lys Gln Asn Tyr Val Leu Ser Leu Gln Ile Glu Tyr Leu Ser Lys
   130 135 140
   Gln Leu Gln Glu Ile Ser Asp Lys Leu Asp Ile Ile Asn Val Asn Val
   145 150 155 160
   Leu Ile Asn Ser Thr Leu Thr Glu Ile Thr Pro Ala Tyr Gln Arg Met
   165 170 175
   Lys Tyr Val Asn Glu Lys Phe Glu Glu Leu Thr Phe Ala Thr Glu Thr
   180 185 190
   Thr Leu Lys Val Lys Lys Asp Ser Pro Pro Ala Asp Ile Leu Asp Glu
   195 200 205
   Leu Thr Glu Leu Thr Glu Leu Ala Lys Ser Val Thr Lys Asn Asp Val
   210 215 220
   Asp Gly Phe Glu Phe Tyr Leu Asn Thr Phe His Asp Val Met Val Gly
   225 230 235 240
   Asn Asn Leu Phe Gly Arg Ser Ala Leu Lys Thr Ala Ser Glu Leu Ile
   245 250 255
   Ala Lys Glu Asn Val Lys Thr Ser Gly Ser Glu Val Gly Asn Val Tyr
   260 265 270
   Asn Phe Leu Ile Val Leu Thr Ala Leu Gln Ala Lys Ala Phe Leu Thr
   275 280 285
   Leu Thr Thr Cys Arg Lys Leu Leu Gly Leu Ala Asp Ile Asp Tyr Thr
   290 295 300
   Ser Ile Met Asn Glu His Leu Asn Lys Glu Lys Glu Glu Phe Arg Val
   305 310 315 320
   Asn Ile Leu Pro Thr Leu Ser Asn Thr Phe Ser Asn Pro Asn Tyr Ala
   325 330 335
   Lys Ala Lys Gly Ser Asn Glu Asp Thr Lys Met Ile Val Glu Ala Lys
   340 345 350
   Pro Gly Tyr Val Leu Val Gly Phe Glu Met Ser Asn Asp Ser Ile Thr
   355 360 365
   Val Leu Lys Ala Tyr Gln Ala Lys Leu Lys Lys Asp Tyr Gln Ile Asp
   370 375 380
   Lys Asp Ser Leu Ser Glu Ile Ile Tyr Ser Asp Thr Asp Lys Leu Leu
   385 390 395 400
   Cys Pro Asp Gln Ser Glu Gln Ile Tyr Tyr Thr Lys Asn Ile Ala Phe
   405 410 415
   Pro Asn Glu Tyr Val Ile Thr Lys Ile Ala Phe Thr Lys Lys Met Asn
   420 425 430
   Ser Leu Arg Tyr Glu Ala Thr Ala Asn Phe Tyr Asp Ser Ser Thr Gly
   435 440 445
   Asp Ile Asp Leu Asn Lys Thr Lys Val Glu Ser Ser Glu Ala Glu Tyr
   450 455 460
   Ser Met Leu Lys Ala Ser Asp Asp Glu Val Tyr Met Pro Leu Gly Leu
   465 470 475 480
   Ile Ser Glu Thr Phe Leu Asn Pro Ile Asn Gly Phe Arg Leu Ala Val
   485 490 495
   Asp Glu Asn Ser Arg Leu Val Thr Leu Thr Cys Arg Ser Tyr Leu Arg
   500 505 510
   Glu Thr Leu Leu Ala Thr Asp Leu Asn Asn Lys Glu Thr Lys Leu Ile
   515 520 525
   Val Pro Pro Asn Val Phe Ile Ser Asn Ile Val Glu Asn Gly Asn Ile
   530 535 540
   Glu Met Asp Thr Leu Glu Pro Trp Lys Ala Asn Asn Glu Asn Ala Asn
   545 550 555 560
   Val Asp Tyr Ser Gly Gly Val Asn Gly Thr Arg Ala Leu Tyr Val His
   565 570 575
   Lys Asp Gly Glu Phe Ser His Phe Ile Gly Asp Lys Leu Lys Ser Lys
   580 585 590
   Thr Glu Tyr Leu Ile Arg Tyr Ile Val Lys Gly Lys Ala Ser Ile Phe
   595 600 605
   Leu Lys Asp Glu Lys Asn Glu Asn Tyr Ile Tyr Glu Asp Thr Asn Asn
   610 615 620
   Asn Leu Glu Asp Tyr Gln Thr Ile Thr Lys Arg Phe Thr Thr Gly Thr
   625 630 635 640
   Asp Ser Thr Gly Val Tyr Leu Ile Phe Asn Ser Gln Asn Gly Asp Glu
   645 650 655
   Ala Trp Gly Asp Asn Phe Ile Ile Leu Glu Ile Ser Pro Cys Glu Lys
   660 665 670
   Leu Leu Ser Pro Glu Leu Ile Lys Thr Asp Lys Trp Asn Ser Thr Gly
           675                 680                 685
   Ser Thr Tyr Ile Ser Asp Asp Arg Leu Thr Leu Tyr Arg Gly Gly Arg
       690                 695                 700
   Gly Ile Leu Lys Gln Asn Leu Gln Leu Asp Gly Phe Ser Thr Tyr Arg
   705                 710                 715                 720
   Val Asn Phe Ser Val Asp Gly Asp Ala Asn Val Arg Ile Arg Asn Ser
                   725                 730                 735
   Arg Glu Val Leu Leu Glu Lys Arg Tyr Leu Asn Arg Lys Gly Val Ser
               740                 745                 750
   Glu Met Phe Thr Thr Lys Phe Asp Lys Asp Asn Phe Tyr Val Glu Leu
           755                 760                 765
   Ser Gln Gly Asp Asn Leu Gly Thr Ile Val His Phe Tyr Asp Phe Ser
       770                 775                 780
   Ile Lys
   785

## Claims (Claims for the following Contracting State(s): AT, BE, BG, CH, CY, CZ, DE, DK, EE, ES, FI, FR, GB, GR, HU, IE, IT, LI, LU, MC, NL, PT, SE, SI, SK, TR)

1. An isolated protein insecticidal to *Ostrinia nubilalis,* comprising the amino acid sequence of a variant of the protein of SEQ ID No. 4, wherein said variant has 5 to 10 amino acids or less than 5 amino acids added, replaced or deleted as compared to the protein of SEQ ID No. 4 without significantly changing the insecticidal activity of the protein, wherein said protein starts with a Met-Asp or Met-Ala dipeptide, by insertion of a codon encoding an Asp or Ala amino acid downstream of the start codon in the DNA encoding the protein.

2. An isolated protein insecticidal to *Ostrinia nubilalis,* comprising the amino acid sequence of SEQ ID No. 4.

3. The protein of claim 1 or 2, which is insecticidal against *Pectinophora gossypiella, Scirphophaga incertulas, Cnaphalocrocis medinalis, Sesamia inferens, Chilo partellus* and *Anticarsia gemmatalis.*

4. An isolated nucleic acid sequence encoding the protein of any one of claims 1 to 3.

5. The isolated nucleic acid sequence of claim 4, comprising nucleotides 1 to 2367 of SEQ ID No. 3.

6. The isolated nucleic acid sequence of claim 4, which is a synthetic sequence that has been optimized for expression in monocotyledonous plants or dicotyledonous plants.

7. A chimeric gene comprising a promoter sequence operably linked to a nucleic acid sequence of any one of claims 4 to 6.

8. The chimeric gene of claim 7, which also comprises a coding region encoding a transit peptide for chloroplast or other plastid targeting, linked to the nucleic acid sequence of any one of claims 4 to 6.

9. A vector comprising the chimeric gene of claim 7 or 8.

10. A transgenic host cell comprising the chimeric gene of claim 7 or 8.

11. The host cell of claim 10, which is a plant cell.

12. The host cell of claim 10, which is a microorganism.

13. A transgenic plant comprising the chimeric gene of claim 7 or 8.

14. The plant of claim 13, which is a maize, cotton, rice or soybean plant.

15. A method of protecting a plant against insect damage comprising contacting said plant with the insecticidal protein of any one of claims 1 to 3.

16. The method of claim 15, wherein said insecticidal protein is encoded by a chimeric gene integrated in the genome of said plant.

17. The method of claim 15, wherein said protein is applied externally to said plant.

18. The method of any one of claims 15-17, wherein said plant is a maize, cotton, soybean or rice plant.

19. A *Bacillus thuringiensis* strain transformed with a nucleic acid sequence according to any one of claims 4 to 6.

20. An insecticidal composition comprising the protein of any one of claims 1 to 3 which, when applied externally to a plant, increases resistance to insect damage compared to control plants to which no such composition is applied.

21. The use of the protein of any one of claims 1 to 3 against a Lepidopteran cotton, maize, rice or soybean insect pest.

22. The use of claim 21, wherein said insect pest is *Helicoverpa zea, Heliothis virescens, Ostrinia nubilalis, Spodoptera frugiperola, Pectinophora gossypiella, Scirphophaga incertulas, Cnaphalocrocis medinalis, Sesamia inferens, Chilo partellus* and *Anticarsia gemmatalis.*

23. The use of claim 22, wherein said insect pest is *Scirphophaga incertulas, Cnaphalocrocis medinalis, Sesamia inferens, Chilo partellus* and *Anticarsia gemmatalis.*

24. The plant of claim 13, which simultaneously expresses with the protein of SEQ ID No. 4 or its fragment or variant: a Cry protein, a Cry1F protein, a hybrid derived from a Cry1F protein, a Cry1A-type protein or a toxic fragment thereof, a Cry1Ac protein or a hybrid derived therefrom, a Cry1Ab protein or an insecticidal fragment thereof, a Cry2Ae protein, a VIP3Aa protein or a toxic fragment thereof, or an insecticidal protein from *Xenothabdus, Serratia* or *Photorhabdus* species strains.

25. The plant of claim 24 which is maize, rice, cotton or soybean.

26. The plant of claim 24 or 25 which is a hybrid plant.

27. Use of the protein of any one of claims 1 to 3, co-expressed in maize, rice, cotton or soybean plants, in combination with another insect control protein, wherein said other insect control protein is: a Cry1Ac protein, a Cry1Ab protein, a Cry2Ae protein, or a VIP3Aa protein or derivatives thereof.

28. The plant cell of claim 11 or the plant of claim 13, also comprising a PAT gene conferring resistance to glufosinate ammonium, or a 2mEPSPS gene conferring resistance to glyphosate.

29. Use of a protein insecticidal to *Ostrinia nubilalis,* comprising the amino acid sequence of a variant of the protein of SEQ ID No. 4, co-expressed in maize, rice, cotton or soybean plants, in combination with another insect control protein, wherein said other insect control protein is: a Cry2Ae protein, or a VIP3Aa protein or derivatives thereof, wherein said variant has 5 to 10 amino acids or less than 5 amino acids added, replaced or deleted as compared to the protein of SEQ ID No. 4 without significantly changing the insecticidal activity of the protein.

## Claims (Claims for the following Contracting State(s): RO)

1. An isolated protein insecticidal to *Ostrinia nubilalis,* comprising the amino acid sequence of a variant of the protein of SEQ ID No. 4, wherein said variant has 5 to 10 amino acids or less than 5 amino acids added, replaced or deleted as compared to the protein of SEQ ID No. 4 without significantly changing the insecticidal activity of the protein.

2. The protein of claim 1, comprising the amino acid sequence of a variant of SEQ ID No. 4 wherein said variant has less than 5 amino acids added, replaced or deleted as compared to the protein of SEQ ID No. 4 without significantly changing the insecticidal activity of the protein.

3. The protein of any one of claims 1 or 2, wherein said protein starts with a Met-Asp or Met-Ala dipeptide, by insertion of a codon encoding an Asp or Ala amino acid downstream of the start codon in the DNA encoding the protein of any one of claims 1 to 2.

4. The protein of claim 1, comprising the amino acid sequence of SEQ ID No. 4.

5. An isolated protein insecticidal to *Ostrinia nubilalis,* comprising the amino acid sequence of the smallest fragment of the protein of SEQ ID No. 4 retaining insecticidal activity, which is obtained by Enzymatic digestion of the protein of SEQ ID No. 4 with gut-juice fluid from *Ostrinia nubilalis.*

6. The protein of any one of claims 1 to 5, which is insecticidal against *Pectinophora gossypiella, Scirphophaga incertulas, Cnaphalocrocis medinalis, Sesamia inferens, Chilo partellus* and *Anticarsia gemmatalis.*

7. An isolated nucleic acid sequence encoding the protein of any one of claims 1 to 6.

8. The isolated nucleic acid sequence of claim 7, comprising nucleotides 1 to 2367 of SEQ ID No. 3.

9. The isolated nucleic acid sequence of claim 7, which is a synthetic sequence that has been optimized for expression in monocotyledonous plants or dicotyledonous plants.

10. A chimeric gene comprising a promoter sequence operably linked to a nucleic acid sequence of any one of claims 7 to 9.

11. The chimeric gene of claim 10, which also comprises a coding region encoding a transit peptide for chloroplast or other plastid targeting, linked to the nucleic acid sequence of any one of claims 7 to 9.

12. A vector comprising the chimeric gene of claim 10 or 11.

13. A transgenic host cell comprising the chimeric gene of claim 10 or 11.

14. The host cell of claim 13, which is a plant cell.

15. The host cell of claim 13, which is a microorganism.

16. A transgenic plant comprising the chimeric gene of claim 10 or 11.

17. The plant of claim 18, which is a maize, cotton, rice or soybean plant.

18. A method of protecting a plant against insect damage comprising contacting said plant with the insecticidal protein of any one of claims 1 to 6.

19. The method of claim 18, wherein said insecticidal protein is encoded by a chimeric gene integrated in the genome of said plant.

20. The method of claim 18, wherein said protein is applied externally to said plant.

21. The method of any one of claims 18 to 20, wherein said plant is a maize, cotton, soybean or rice plant.

22. A *Bacillus thuringiensis* strain transformed with a nucleic acid sequence according to any one of claims 7 to 9.

23. An insecticidal composition comprising the protein of any one of claims 1 to 6 which, when applied externally to a plant, increases resistance to insect damage compared to control plants to which no such composition is applied.

24. The use of the protein of any one of claims 1 to 6 against a Lepidopteran cotton, maize, rice or soybean insect pest.

25. The use of claim 24, wherein said insect pest is *Helicoverpa zea, Heliothis virescens, Ostrinia nubilalis, Spodoptera frugiperda, Pectinophora gossypiella, Scirphophaga incertulas, Cnaphalocrocis medinalis, Sesamia inferens, Chilo partellus* and *Anticarsia gemmatalis.*

26. The use of claim 25, wherein said insect pest is *Scirphophaga incertulas, Cnaphalocrocis medinalis, Sesamia inferens, Chilo partellus* and *Anticarsia gemmatalis.*

27. The plant of claim 16, which simultaneously expresses with the protein of SEQ ID No. 4 or its fragment or variant : a Cry protein, a Cry1F protein, a hybrid derived from a Cry1F protein, a Cry1A-type protein or a toxic fragment thereof, a Cry1Ac protein or a hybrid derived therefrom, a Cry1Ab protein or an insecticidal fragment thereof, a Cry2Ae protein, a VIP3Aa protein or a toxic fragment thereof, or an insecticidal protein from *Xenorhabdus, Serratia* or *Photorhabdus* species strains.

28. The plant of claim 27 which is maize, rice, cotton or soybean.

29. The plant of claim 27 or 28 which is a hybrid plant.

30. Use of the protein of any one of claims 1 to 6 co-expressed in maize, rice, cotton or soybean plants, in combination with another insect control protein, wherein said other insect control protein is: a Cry1Ac protein, a Cry1Ab protein, a Cry2Ae protein, or a VIP3Aa proteinor derivatives thereof.

31. The plant cell of claim 14 or the plant of claim 16, also comprising a PAT gene conferring resistance to glufosinate ammonium, or a 2mEPSPS gene conferring resistance to glyphosate.

## Patentansprüche (Patentansprüche für folgende(n) Vertragsstaat(en): AT, BE, BG, CH, CY, CZ, DE, DK, EE, ES, FI, FR, GB, GR, HU, IE, IT, LI, LU, MC, NL, PT, SE, SI, SK, TR)

1. Isoliertes für *Ostrinia nubilalis* insektizides Protein, umfassend die Aminosäuresequenz einer Variante des Proteins gemäß SEQ ID Nr.:4, wobei diese Variante im Vergleich zu dem Protein gemäß SEQ ID Nr.:4 5 bis 10 oder weniger als 5 hinzugefügte, ersetzte oder deletierte Aminosäuren aufweist, ohne dass dabei die insektizide Wirksamkeit des Proteins wesentlich verändert wird, wobei das Protein mit einem Met-Asp- oder Met-Ala-Dipeptid beginnt, durch Insertieren eines für eine Aminosäure Asp oder Ala codierenden Codons stromabwärts des Start-Codons in der DNA, die für das Protein codiert.

2. Isoliertes für *Ostrinia nubilalis* insektizides Protein, umfassend die Aminosäuresequenz SEQ ID Nr.:4.

3. Protein nach Anspruch 1 oder 2, das für *Pectinophora gossypiella, Scirphophaga incertulas, Cnaphalocrocis medinalis, Sesamia inferens, Chilo partellus* und *Anticarsia gemmatalis* insektizid wirkt.

4. Isolierte Nukleinsäuresequenz, die für das Protein nach einem der Ansprüche 1 bis 3 codiert.

5. Isolierte Nukleinsäuresequenz nach Anspruch 4, umfassend die Nukleotide 1 bis 2367 von SEQ ID Nr.:3.

6. Isolierte Nukleinsäuresequenz nach Anspruch 4, bei der es sich um eine synthetische Sequenz, die für Expression in monokotylen Pflanzen oder dikotylen Pflanzen optimiert worden ist, handelt.

7. Chimäres Gen, umfassend eine Promotersequenz, die operativ mit einer Nukleinsäuresequenz nach einem der Ansprüche 4 bis 6 verknüpft ist.

8. Chimäres Gen nach Anspruch 7, das auch eine Codierregion, die für ein Transitpeptid für das Targeting an die Chloroplasten oder sonstige Plastide codiert, in Verknüpfung mit der Nukleinsäuresequenz nach einem der Ansprüche 4 bis 6 umfasst.

9. Vektor, umfassend das chimäre Gen nach Anspruch 7 oder 8.

10. Transgene Wirtszelle, umfassend das chimäre Gen nach Anspruch 7 oder 8.

11. Wirtszelle nach Anspruch 10, bei der es sich um eine Pflanzenzelle handelt.

12. Wirtszelle nach Anspruch 10, bei der es sich um einen Mikroorganismus handelt.

13. Transgene Pflanze, umfassend das chimäre Gen nach Anspruch 7 oder 8.

14. Pflanze nach Anspruch 13, bei der es sich um eine Mais-, Baumwoll-, Reis- oder Sojabohnenpflanze handelt.

15. Verfahren zum Schützen einer Pflanze gegen Schädigung durch Insekten, umfassend das Inkontaktbringen dieser Pflanze mit dem insektiziden Protein nach einem der Ansprüche 1 bis 3.

16. Verfahren nach Anspruch 15, wobei das insektizide Protein von einem chimären Gen, das in das Genom dieser Pflanze integriert ist, codiert wird.

17. Verfahren nach Anspruch 15, wobei das Protein von außen auf diese Pflanze aufgebracht wird.

18. Verfahren nach einem der Ansprüche 15 bis 17, wobei es sich bei der Pflanze um eine Mais-, Baumwoll-, Sojabohnen- oder Reispflanze handelt.

19. *Bacillus-thuringiensis-Stamm,* der mit einer Nukleinsäuresequenz nach einem der Ansprüche 4 bis 6 transformiert ist.

20. Insektizide Zusammensetzung, umfassend das Protein nach einem der Ansprüche 1 bis 3, die, wenn sie von außen auf eine Pflanze aufgebracht wird, die Resistenz gegenüber Schädigung durch Insekten im Vergleich zu Kontrollpflanzen, auf die keine solche Zusammensetzung aufgebracht wird, erhöht.

21. Verwendung des Proteins nach einem der Ansprüche 1 bis 3 gegen ein Lepidopteren-Schadinsekt an Baumwolle, Mais, Reis oder Sojabohne.

22. Verwendung nach Anspruch 21, wobei es sich bei dem Schadinsekt um *Helicoverpa zea, Heliothis virescens, Ostrinia nubilalis, Spodoptera frugiperda, Pectinophora gossypiella, Scirphophaga incertulas, Cnaphalocrocis medinalis, Sesamia inferens, Chilo partellus* und *Anticarsia gemmatalis* handelt.

23. Verwendung nach Anspruch 22, wobei es sich bei dem Schadinsekt um *Scirphophaga incertulas, Cnaphalocrocis medinalis, Sesamia inferens, Chilo partellus* und *Anticarsia gemmatalis* handelt.

24. Pflanze nach Anspruch 13, die gleichzeitig mit dem Protein gemäß SEQ ID Nr.:4 oder seinem Fragment bzw. seiner Variante Folgendes exprimiert: ein Cry-Protein, ein Cry1F-Protein, einen von einem Cry1F-Protein abgeleiteten Hybriden, ein Protein des Cry1A-Typs oder ein toxisches Fragment davon, ein Cry1Ac-Protein oder einen davon abgeleiteten Hybriden, ein Cry1Ab-Protein oder ein insektizides Fragment davon, ein Cry2Ae-Protein, ein VIP3Aa-Protein oder ein toxisches Fragment davon, oder ein insektizides Protein aus Stämmen der Arten *Xenorhabdus, Serratia* oder *Photorhabdus.*

25. Pflanze nach Anspruch 24, bei der es sich um Mais, Reis, Baumwolle oder Sojabohne handelt.

26. Pflanze nach Anspruch 24 oder 25, bei der es sich um eine Hybridpflanze handelt.

27. Verwendung des Proteins nach einem der Ansprüche 1 bis 3, das in Mais-, Reis-, Baumwoll- oder Sojabohnenpflanzen in Kombination mit einem weiteren Insektenkontrollprotein coexprimiert wird, wobei es sich bei dem weiteren Insektenkontrollprotein um ein Cry1Ac-Protein, ein Cry1Ab-Protein, ein Cry2Ae-Protein oder ein VIP3Aa-Protein oder Derivate davon handelt.

28. Pflanzenzelle nach Anspruch 11 oder Pflanze nach Anspruch 13, die auch ein PAT-Gen, das Resistenz gegenüber Glufosinate-Ammonium verleiht, oder ein 2mEPSPS-Gen, das Resistenz gegen Glyphosate verleiht, umfasst.

29. Verwendung eines für *Ostrinia nubilalis* insektiziden Proteins, umfassend die Aminosäuresequenz einer Variante des Proteins gemäß SEQ ID Nr.:4, das in Mais-, Reis-, Baumwoll- oder Sojabohnenpflanzen in Kombination mit einem weiteren Insektenkontrollprotein coexprimiert wird, wobei es sich bei dem weiteren Insektenkontrollprotein um ein Cry2Ae-Protein oder ein VIP3Aa-Protein oder Derivate davon handelt, wobei diese Variante im Vergleich zu dem Protein gemäß SEQ ID Nr.:4 5 bis 10 oder weniger als 5 hinzugefügte, ersetzte oder deletierte Aminosäuren aufweist, ohne dass dabei die insektizide Wirksamkeit des Proteins wesentlich verändert wird.

## Patentansprüche (Patentansprüche für folgende(n) Vertragsstaat(en): RO)

1. Isoliertes für *Ostrinia nubilalis* insektizides Protein, umfassend die Aminosäuresequenz einer Variante des Proteins gemäß SEQ ID Nr.:4, wobei diese Variante im Vergleich zu dem Protein gemäß SEQ ID Nr.:4 5 bis 10 oder weniger als 5 hinzugefügte, ersetzte oder deletierte Aminosäuren aufweist, ohne dass dabei die insektizide Wirksamkeit des Proteins wesentlich verändert wird.

2. Protein nach Anspruch 1, umfassend die Aminosäuresequenz einer Variante der SEQ ID Nr.:4, wobei diese Variante im Vergleich zu dem Protein gemäß SEQ ID Nr.:4 weniger als 5 hinzugefügte, ersetzte oder deletierte Aminosäuren aufweist, ohne dass dabei die insektizide Wirksamkeit des Proteins wesentlich verändert wird.

3. Protein nach einem der Ansprüche 1 oder 2, wobei das Protein mit einem Met-Asp- oder Met-Ala-Dipeptid beginnt, durch Insertieren eines für eine Aminosäure Asp oder Ala codierenden Codons stromabwärts des Start-Codons in der DNA, die für das Protein nach einem der Ansprüche 1 bis 2 codiert.

4. Protein nach Anspruch 1, umfassend die Aminosäuresequenz gemäß SEQ ID Nr.:4.

5. Isoliertes für *Ostrinia nubilalis* insektizides Protein, umfassend die Aminosäuresequenz des kleinsten Fragments des Proteins gemäß SEQ ID Nr.:4, das eine insektizide Wirksamkeit beibehält, wobei das Protein durch enzymatische Verdauung des Proteins gemäß SEQ ID Nr.:4 mit Darmflüssigkeit aus *Ostrinia nubilalis* erhalten wird.

6. Protein nach einem der Ansprüche 1 bis 5, das für *Pectinophora gossypiella, Scirphophaga incertulas, Cnaphalocrocis medinalls, Sesamia inferens, Chilo partellus* und *Anticarsia gemmatalis* insektizid wirkt.

7. Isolierte Nukleinsäuresequenz, die für das Protein nach einem der Ansprüche 1 bis 6 codiert.

8. Isolierte Nukleinsäuresequenz nach Anspruch 7, umfassend die Nukleotide 1 bis 2367 von SEQ ID Nr.:3.

9. Isolierte Nukleinsäuresequenz nach Anspruch 7, bei der es sich um eine synthetische Sequenz, die für Expression in monokotylen Pflanzen oder dikotylen Pflanzen optimiert worden ist, handelt.

10. Chimäres Gen, umfassend eine Promotersequenz, die operativ mit einer Nukleinsäuresequenz nach einem der Ansprüche 7 bis 9 verknüpft ist.

11. Chimäres Gen nach Anspruch 10, das auch eine Codierregion, die für ein Transitpeptid für das Targeting an die Chloroplasten oder sonstige Plastide codiert, in Verknüpfung mit der Nukleinsäuresequenz nach einem der Ansprüche 7 bis 9 umfasst.

12. Vektor, umfassend das chimäre Gen nach Anspruch 10 oder 11.

13. Transgene Wirtszelle, umfassend das chimäre Gen nach Anspruch 10 oder 11.

14. Wirtszelle nach Anspruch 13, bei der es sich um eine Pflanzenzelle handelt.

15. Wirtszelle nach Anspruch 13, bei der es sich um einen Mikroorganismus handelt.

16. Transgene Pflanze, umfassend das chimäre Gen nach Anspruch 10 oder 11.

17. Pflanze nach Anspruch 16, bei der es sich um eine Mais-, Baumwoll-, Reis- oder Sojabohnenpflanze handelt.

18. Verfahren zum Schützen einer Pflanze gegen Schädigung durch Insekten, umfassend das Inkontaktbringen dieser Pflanze mit dem insektiziden Protein nach einem der Ansprüche 1 bis 6.

19. Verfahren nach Anspruch 18, wobei das insektizide Protein von einem chimären Gen, das in das Genom dieser Pflanze integriert ist, codiert wird.

20. Verfahren nach Anspruch 18, wobei das Protein von außen auf diese Pflanze aufgebracht wird.

21. Verfahren nach einem der Ansprüche 18 bis 20, wobei es sich bei der Pflanze um eine Mais-, Baumwoll-, Sojabohnen- oder Reispflanze handelt.

22. *Bacillus-thuringiensis-Stamm,* der mit einer Nukleinsäuresequenz nach einem der Ansprüche 7 bis 9 transformiert ist.

23. Insektizide Zusammensetzung, umfassend das Protein nach einem der Ansprüche 1 bis 6, die, wenn sie von außen auf eine Pflanze aufgebracht wird, die Resistenz gegenüber Schädigung durch Insekten im Vergleich zu Kontrollpflanzen, auf die keine solche Zusammensetzung aufgebracht wird, erhöht.

24. Verwendung des Proteins nach einem der Ansprüche 1 bis 6 gegen ein Lepidopteren-Schadinsekt an Baumwolle, Mais, Reis oder Sojabohne.

25. Verwendung nach Anspruch 24, wobei es sich bei dem Schadinsekt um *Helicoverpa zea, Heliothis virescens, Ostrinia nubilalis, Spodoptera frugiperda, Pectinophora gossypiella, Scirphophaga incertulas, Cnaphalocrocis medinalis, Sesamia inferens, Chilo partellus* und *Anticarsia gemmatalis* handelt.

26. Verwendung nach Anspruch 25, wobei es sich bei dem Schadinsekt um *Scirphophaga incertulas, Cnaphalocrocis medinalis, Sesamia inferens, Chilo partellus* und *Anticarsia gemmatalis* handelt.

27. Pflanze nach Anspruch 16, die gleichzeitig mit dem Protein gemäß SEQ ID Nr.:4 oder seinem Fragment bzw. seiner Variante Folgendes exprimiert: ein Cry-Protein, ein Cry1F-Protein, einen von einem Cry1F-Protein abgeleiteten Hybriden, ein Protein des Cry1A-Typs oder ein toxisches Fragment davon, ein Cry1Ac-Protein oder einen davon abgeleiteten Hybriden, ein Cry1Ab-Protein oder ein insektizides Fragment davon, ein Cry2Ae-Protein, ein VIP3Aa-Protein oder ein toxisches Fragment davon, oder ein insektizides Protein aus Stämmen der Arten *Xenorhabdus, Serratia* oder *Photorhabdus.*

28. Pflanze nach Anspruch 27, bei der es sich um Mais, Reis, Baumwolle oder Sojabohne handelt.

29. Pflanze nach Anspruch 27 oder 28, bei der es sich um eine Hybridpflanze handelt.

30. Verwendung des Proteins nach einem der Ansprüche 1 bis 6, das in Mais-, Reis-, Baumwoll- oder Sojabohnenpflanzen in Kombination mit einem weiteren Insektenkontrollprotein coexprimiert wird, wobei es sich bei dem weiteren Insektenkontrollprotein um ein Cry1Ac-Protein, ein Cry1Ab-Protein, ein Cry2Ae-Protein oder ein VIP3Aa-Protein oder Derivate davon handelt.

31. Pflanzenzelle nach Anspruch 14 oder Pflanze nach Anspruch 16, die auch ein PAT-Gen, das Resistenz gegenüber Glufosinate-Ammonium verleiht, oder ein 2mEPSPS-Gen, das Resistenz gegen Glyphosate verleiht, umfasst.

## Revendications (Revendications pour l'(les) Etat(s) contractant(s) suivant(s): AT, BE, BG, CH, CY, CZ, DE, DK, EE, ES, FI, FR, GB, GR, HU, IE, IT, LI, LU, MC, NL, PT, SE, SI, SK, TR)

1. Protéine isolée insecticide vis-à-vis *d'Ostrinia nubilalis*, comprenant la séquence d'acides aminés d'un variant de la protéine ayant la séquence SEQ ID N° 4, ledit variant ayant 5 à 10 acides aminés ou moins de 5 acides aminés qui ont été ajoutés, remplacés ou délétés par comparaison avec la protéine ayant la séquence SEQ ID N° 4, sans changement significatif de l'activité insecticide de la protéine ladite protéine commençant par un dipeptide Met-Asp ou Met-Ala, par insertion d'un codon codant pour un acide aminé Asp ou Ala en aval du codon d'initiation de l'ADN codant pour la protéine.

2. Protéine isolée insecticide vis-à-vis *d'Ostrinia nubilalis*, comprenant la séquence d'acides aminés SEQ ID N° 4.

3. Protéine selon la revendication 1 ou 2, qui est insecticide vis-à-vis de *Pectinophora gossypiella, Scirphophaga incertulas, Cnaphalocrocis medinalis, Sesamia inferens, Chilo partellus* et *Anticarsia gemmatalis.*

4. Séquence d'acide nucléique isolé codant pour la protéine selon l'une quelconque des revendications 1 à 3.

5. Séquence d'acide nucléique isolé selon la revendication 4, comprenant les nucléotides 1 à 2367 de la séquence SEQ ID N° 3.

6. Séquence d'acide nucléique isolé selon la revendication 4, qui est une séquence synthétique ayant été optimisée pour expression dans des plantes monocotylédones ou dicotylédones.

7. Gène chimère comprenant une séquence promoteur liée d'une manière opérationnelle à une séquence d'acide nucléique selon l'une quelconque des revendications 4 à 6.

8. Gène chimère selon la revendication 7, qui comprend aussi une région codante, qui code pour un peptide de transit pour adressage au chloroplaste ou à un autre plastide, lié à la séquence d'acide nucléique selon l'une quelconque des revendications 4 à 6.

9. Vecteur comprenant le gène chimère selon la revendication 7 ou 8.

10. Cellule hôte transgénique comprenant le gène chimère selon la revendication 7 ou 8.

11. Cellule hôte selon la revendication 10, qui est une cellule végétale.

12. Cellule hôte selon la revendication 10, qui est un microorganisme.

13. Plante transgénique comprenant le gène chimère selon la revendication 7 ou 8.

14. Plante selon la revendication 13, qui est un plant de maïs, de coton, de riz ou de soja.

15. Procédé de protection d'une plante à l'encontre des dommages dus à des insectes, comprenant la mise en contact de ladite plante avec la protéine insecticide selon l'une quelconque des revendications 1 à 3.

16. Procédé selon la revendication 15, dans lequel ladite protéine insecticide est codée par un gène chimère intégré dans le génome de ladite plante.

17. Procédé selon la revendication 15, dans lequel ladite protéine est appliquée à l'extérieur de ladite plante.

18. Procédé selon l'une quelconque des revendications 15 à 17, dans lequel ladite plante est un plant de maïs, de coton, de soja ou de riz.

19. Souche de *Bacillus thuringiensis,* transformée avec une séquence d'acide nucléique selon l'une quelconque des revendications 4 à 6.

20. Composition insecticide comprenant la protéine selon l'une quelconque des revendications 1 à 3, qui, quand elle est appliquée à l'extérieur d'une plante, augmente la résistance à des dommages dus à des insectes, par comparaison avec des plantes témoins sur lesquelles aucune composition de ce type n'est appliquée.

21. Utilisation de la protéine selon l'une quelconque des revendications 1 à 3 contre un insecte ravageur lépidoptère du coton, du maïs, du riz ou du soja.

22. Utilisation selon la revendication 21, pour laquelle ledit insecte ravageur est *Helicoverpa zea, Heliothis virescens, Ostrinia nubilalis, Spodoptera frugiperda, Pectinophora gossypiella, Scirphophaga incertulas, Cnaphalocrocis medinalis, Sesamia inferens, Chilo partellus* et *Anticarsia gemmatalis.*

23. Utilisation selon la revendication 22, pour laquelle ledit insecte ravageur est *Scirphophaga incertulas, Cnaphalocrocis medinalis, Sesamia inferens, Chilo partellus* et *Anticarsia gemmatalis.*

24. Plante selon la revendication 13, qui, simultanément à la protéine ayant la SEQ ID N° 4 ou un fragment ou un variant de cette dernière, exprime : une protéine Cry, une protéine Cry1F, un hybride dérivant d'une protéine Cry1F, une protéine de type Cry1A ou un fragment toxique de cette dernière, un fragment Cry1Ac ou un hybride qui dérive de cette dernière, une protéine Cry1Ab ou un fragment insecticide de cette dernière, une protéine Cry2Ae, une protéine VIP3Aa ou un fragment toxique de cette dernière, ou une protéine insecticide provenant de souches de l'espèce *Senorhabdus, Serratia* ou *Photorhabdus.*

25. Plante selon la revendication 24, qui est le maïs, le riz, le coton ou le soja.

26. Plante selon la revendication 24 ou 25, qui est une plante hybride.

27. Utilisation de la protéine selon l'une quelconque des revendications 1 à 3, co-exprimée dans des plants de maïs, de riz, de coton ou de soja, en combinaison avec une autre protéine de maîtrise d'insectes, ladite autre protéine de maîtrise d'insectes étant une protéine Cry1Ac, une protéine Cry1Ab, une protéine Cry2Ae ou une protéine VIP3Aa, ou des dérivés de ces dernières.

28. Cellule végétale selon la revendication 11, ou plante selon la revendication 13, comprenant aussi un gène PAT conférant une résistance au glufosinate-ammonium, ou un gène 2mEPSPS conférant une résistance au glyphosate.

29. Utilisation d'une protéine insecticide vis-à-vis d'*Ostrinia nubilalis*, comprenant la séquence d'acides aminés d'un variant de la protéine ayant la séquence SEQ ID N° 4, co-exprimée dans des plants de maïs, de riz, de coton ou de soja, en combinaison avec une autre protéine de maîtrise d'insectes, ladite autre protéine de maîtrise d'insectes étant une protéine Cry2Ae ou une protéine VIP3Aa, ou des dérivés de ces dernières, ledit variant ayant 5 à 10 acides aminés ou moins de 5 acides aminés qui ont été ajoutés, remplacés ou délétés par comparaison avec la protéine ayant la séquence SEQ ID N° 4, sans changement significatif de l'activité insecticide de la protéine.

## Revendications (Revendications pour l'(les) Etat(s) contractant(s) suivant(s): RO)

1. Protéine isolée insecticide vis-à-vis *d'Ostrinia nubilalis*, comprenant la séquence d'acides aminés d'un variant de la protéine ayant la séquence SEQ ID N° 4, ledit variant ayant 5 à 10 acides aminés ou moins de 5 acides aminés qui ont été ajoutés, remplacés ou délétés par comparaison avec la protéine ayant la séquence SEQ ID N° 4, sans changement significatif de l'activité insecticide de la protéine.

2. Protéine selon la revendication 1, comprenant la séquence d'acides aminés d'un variant ayant la séquence SEQ ID N° 4, ledit variant ayant moins de 5 acides aminés qui ont été ajoutés, remplacés ou délétés par comparaison avec la protéine ayant la séquence SEQ ID N° 4, sans changement significatif de l'activité insecticide de la protéine.

3. Protéine selon l'une quelconque des revendications 1 ou 2, ladite protéine commençant par un dipeptide Met-Asp ou Met-Ala, par insertion d'un codon codant pour un acide aminé Asp ou Ala en aval du codon d'initiation de l'ADN codant pour la protéine selon l'une quelconque des revendications 1 à 2.

4. Protéine selon la revendication 1, comprenant la séquence d'acides aminés SEQ ID N° 4.

5. Protéine isolée insecticide vis-à-vis *d'Ostrinia nubilalis*, comprenant la séquence d'acides aminés du fragment de la protéine ayant la séquence SEQ ID N° 4 le plus petit qui conserve une activité insecticide, que l'on obtient par digestion enzymatique de la protéine ayant la séquence SEQ ID N° 4 avec le suc intestinal d'*Ostrinia nubilalis.*

6. Protéine selon l'une quelconque des revendications 1 à 5, qui est insecticide vis-à-vis de *Pectinophora gossypiella, Scirphophaga incertulas, Cnaphalocrocis medinalis, Sesamia inferens, Chilo partellus* et *Anticarsia gemmatalis.*

7. Séquence d'acide nucléique isolé codant pour la protéine selon l'une quelconque des revendications 1 à 6.

8. Séquence d'acide nucléique isolé selon la revendication 7, comprenant les nucléotides 1 à 2367 de la séquence SEQ ID N° 3.

9. Séquence d'acide nucléique isolé selon la revendication 7, qui est une séquence synthétique ayant été optimisée pour expression dans des plantes monocotylédones ou dicotylédones.

10. Gène chimère comprenant une séquence promoteur liée d'une manière opérationnelle à une séquence d'acide nucléique selon l'une quelconque des revendications 7 à 9.

11. Gène chimère selon la revendication 10, qui comprend aussi une région codante, qui code pour un peptide de transit pour adressage au chloroplaste ou à un autre plastide, lié à la séquence d'acide nucléique selon l'une quelconque des revendications 7 à 9.

12. Vecteur comprenant le gène chimère selon la revendication 10 ou 11.

13. Cellule hôte transgénique comprenant le gène chimère selon la revendication 10 ou 11.

14. Cellule hôte selon la revendication 13, qui est une cellule végétale.

15. Cellule hôte selon la revendication 13, qui est un microorganisme.

16. Plante transgénique comprenant le gène chimère selon la revendication 10 ou 11.

17. Plante selon la revendication 16, qui est un plant de maïs, de coton, de riz ou de soja.

18. Procédé de protection d'une plante à l'encontre des dommages dus à des insectes, comprenant la mise en contact de ladite plante avec la protéine insecticide selon l'une quelconque des revendications 1 à 6.

19. Procédé selon la revendication 18, dans lequel ladite protéine insecticide est codée par un gène chimère intégré dans le génome de ladite plante.

20. Procédé selon la revendication 18, dans lequel ladite protéine est appliquée à l'extérieur de ladite plante.

21. Procédé selon l'une quelconque des revendications 18 à 20, dans lequel ladite plante est un plant de maïs, de coton, de soja ou de riz.

22. Souche de *Bacillus thuringiensis*, transformée avec une séquence d'acide nucléique selon l'une quelconque des revendications 7 à 9.

23. Composition insecticide comprenant la protéine selon l'une quelconque des revendications 1 à 6, qui, quand elle est appliquée à l'extérieur d'une plante, augmente la résistance à des dommages dus à des insectes, par comparaison avec des plantes témoins sur lesquelles aucune composition de ce type n'est appliquée.

24. Utilisation de la protéine selon l'une quelconque des revendications 1 à 6 contre un insecte ravageur lépidoptère du coton, du maïs, du riz ou du soja.

25. Utilisation selon la revendication 24, pour laquelle ledit insecte ravageur est *Helicoverpa zea, Heliothis virescens, Ostrinia nubilalis, Spodoptera frugiperda, Pectinophora gossypiella, Scirphophaga incertulas, Cnaphalocrocis medinalis, Sesamia inferens, Chilo partellus* et *Anticarsia gemmatalis.*

26. Utilisation selon la revendication 25, pour laquelle ledit insecte ravageur est *Scirphophaga incertulas, Cnaphalocrocis medinalis, Sesamia inferens, Chilo partellus* et *Anticarsia gemmatalis.*

27. Plante selon la revendication 16, qui, simultanément à la protéine ayant la SEQ ID N° 4 ou un fragment ou un variant de cette dernière, exprime : une protéine Cry, une protéine Cry1F, un hybride dérivant d'une protéine Cry1F, une protéine de type Cry1A ou un fragment toxique de cette dernière, un fragment Cry1Ac ou un hybride qui dérive de cette dernière, une protéine Cry1Ab ou un fragment insecticide de cette dernière, une protéine Cry2Ae, une protéine VIP3Aa ou un fragment toxique de cette dernière, ou une protéine insecticide provenant de souches de l'espèce *Senorhabdus, Serratia* ou *Photorhabdus.*

28. Plante selon la revendication 27, qui est le mais, le riz, le coton ou le soja.

29. Plante selon la revendication 27 ou 28, qui est une plante hybride.

30. Utilisation de la protéine selon l'une quelconque des revendications 1 à 6, co-exprimée dans des plants de maïs, de riz, de coton ou de soja, en combinaison avec une autre protéine de maîtrise d'insectes, ladite autre protéine de maîtrise d'insectes étant une protéine Cry1Ac, une protéine Cry1Ab, une protéine Cry2Ae ou une protéine VIP3Aa, ou des dérivés de ces dernières.

31. Cellule végétale selon la revendication 14, ou plante selon la revendication 16, comprenant aussi un gène PAT conférant une résistance au glufosinate-ammonium, ou un gène 2mEPSPS conférant une résistance au glyphosate.
